# EUROPEAN PATENT SPECIFICATION

(11) **EP 3 555 616 B1**
(45) Date of publication and mention of the grant of the patent: **14.08.2024**
(21) Application number: 17880088.4
(22) Date of filing: 15.12.2017
(51) Int. Cl.: G01N 33/02, G01N 33/04, G01N 33/08, G01N 33/12, G01N 33/50, G01N 33/53, G01N 33/543

(54) **COMPOSITIONS, DEVICES, AND METHODS OF ATTENTION DEFICIT DISORDER/ ATTENTION DEFICIT HYPERACTIVITY DISORDER (ADD/ADHD) SENSITIVITY TESTING**
ZUSAMMENSETZUNGEN, VORRICHTUNGEN UND VERFAHREN ZUR EMPFINDLICHKEITSPRÜFUNG FÜR AUFMERKSAMKEITSDEFIZITSTÖRUNG/ AUFMERKSAMKEITSDEFIZIT-/HYPERAKTIVITÄTSSTÖRUNG (ADHD)
COMPOSITIONS, DISPOSITIFS ET MÉTHODES PERMETTANT DE TESTER UNE SENSIBILITÉ LIÉE AU TROUBLE DÉFICITAIRE DE L'ATTENTION AVEC OU SANS HYPERACTIVITÉ (TDA/TDAH)

(30) Priority: 15.12.2016 US 201662434957 P
(43) Date of publication of application: 23.10.2019
(73) Proprietor: Biomerica Inc., Irvine, California 92614 (US)
(72) Inventor: LADERMAN, Elisabeth, Irvine, California 92614 (US); IRANI-COHEN, Zackary, Irvine, California 92614 (US)
(74) Representative: Schlich, George
(86) International application number: PCT/IB2017/058023
(87) International publication number: WO 2018/109746

(56) References cited:
- WO-A1-2004/099785
- WO-A1-2016/077808
- WO-A1-2016/077808
- US-B2- 7 601 509
- L.M.J PELSSER: "ADHD, a food-induced hypersensitivity syndrome: in quest of a cause", 1 January 2011 (2011-01-01), XP055690757, Retrieved from the Internet <URL:https://www.adhdenvoeding.nl/wp-content/uploads/2016/10/1.Proefschrift-ADHD-en-Voeding.pdf>
- NIGG JT ET AL.: "Restriction and Elimination Diets in ADHD Treatment", CHILD ADOLESC PSYCHIATR CLIN N AM, vol. 23, no. 4, 1 October 2014 (2014-10-01), pages 937 - 953, XP055495183

## Description

### Field

The field of the disclosure is related to the identification of food items that trigger and/or exacerbate ADD/ADHD, particularly as it relates to the testing, identification and possible elimination of selected food items as trigger foods for patients diagnosed with or suspected to have ADD/ADHD.

### Background

The background description includes information that may be useful in understanding the present disclosure. It is not an admission that any of the information provided herein is prior art or relevant to the presently claimed invention, or that any publication specifically or implicitly referenced is prior art.

Food sensitivity, especially as it relates to ADD/ADHD (a type of neurodevelopmental psychiatric disorder), often presents with significant problems of attention and/or hyperactivity, and underlying causes of ADD/ADHD are not well understood in the medical community. Most typically, ADD/ADHD is diagnosed by an assessment of a person's childhood behavioral and mental development. Unfortunately, treatment of ADD/ADHD is often less than effective and may present new difficulties due to neurochemical modulatory effects. Elimination of one or more food items has also shown promise in at least reducing incidence and/or severity of the symptoms. However, ADD/ADHD is often quite diverse with respect to dietary items triggering symptoms, and no standardized test to help identify trigger food items with a reasonable degree of certainty is known, leaving such patients often to trial-and-error.

In a textbook by Pelsser, titled "ADHD, a food-induced hypersensitivity syndrome: in quest of cause", there is disclosed, according to its subtitle, "the effects of a restricted elimination diet (RED) on ADHD, ODD and comorbid somatic complaints, and a preliminary survey of the mechanisms of an RED". WO 2016/077808 discloses compositions, devices, and methods of irritable bowel syndrome sensitivity testing.

Accordingly, there is still a need for compositions, devices, and methods of food sensitivity testing, especially for identification and possible elimination of trigger foods for patients identified with or suspected of having ADD/ADHD.

### Summary

**The** present invention provides a test panel for testing food sensitivity in a patient diagnosed with or suspected of having attention deficit disorder / attention deficit hyperactivity disorder (ADD/ADHD), comprising:
a plurality of distinct food preparations, wherein each food preparation is independently coupled to an individually addressable solid carrier;
wherein the plurality of distinct food preparations consists of cantaloupe, wheat, tomato, cucumber, squashes, almond, egg, cauliflower, pinto bean, broccoli, orange, butter, corn, lettuce, rye, peach, green pea, carrot, tea, mustard, strawberry, celery, green pepper, garlic, oat, onion, banana, eggplant, cabbage, safflower, olive, cottage cheese, grapefruit, black walnut, cow's milk, soybean, chili pepper, tobacco, malt, cinnamon, cane sugar, and potato.

The plurality of distinct food preparations have an average discriminatory p-value of ≤ 0.07 as determined by raw p-value or an average discriminatory p-value of ≤ 0.10 as determined by FDR multiplicity adjusted p-value.

In certain embodiments of the disclosure, the distinct food preparations are crude filtered aqueous extracts or processed aqueous extracts.

The present invention also provides an *in vitro* method of testing food sensitivity in a patient diagnosed with or suspected of having attention deficit disorder / attention deficit hyperactivity disorder (ADD/ADHD), comprising:
contacting the test panel of the invention with a bodily fluid of a patient that is diagnosed with or suspected of having ADD/ADHD, wherein the step of contacting is performed under conditions that allow IgG from the bodily fluid to bind to at least one component of each food preparation;
measuring IgG bound to the at least one component of each food preparation to obtain a signal; and
updating or generating a report using the signal.

In certain embodiments, the bodily fluid of the patient is selected from the group consisting of whole blood, plasma, serum, saliva, urine and a fecal suspension.

In another embodiment, the step of measuring IgG bound to the at least one component of each food preparation is performed via immunoassay test. In yet another embodiment, the method comprises comparing the signal to a gender-stratified reference value for each food preparation using gender identification to obtain a result, wherein the gender-stratified reference value for each food preparation is the 90^{th} percentile rank, or higher, of signals obtained by contacting bodily fluid from a control group of subjects that is not diagnosed with or suspected of having ADD/ADHD with the food preparation.

In another embodiment, the solid carrier is a well of a multi well plate, a bead, an electrical sensor, a chemical sensor, a microchip, or an adsorptive film.

In other embodiments, the average discriminatory p-value is determined by a process comprising comparing assay values of a first patient test cohort that is diagnosed with or suspected of having ADD/ADHD headaches with assay values of a second patient test cohort that is not diagnosed with or not suspected of having ADD/ADHD headaches.

Various objects, features, aspects and advantages of the embodiments described herein will become more apparent from the following detailed description of the embodiments, along with the accompanying drawing figures in which like numerals represent like components.

### Brief Description of The Drawings

**Table 1** shows a list of food items from which food preparations can be prepared.
**Table 2** shows statistical data of foods ranked according to 2-tailed FDR multiplicity-adjusted p-values.
**Table 3** shows statistical data of ELISA score by food and gender.
**Table 4** shows cutoff values of foods for a predetermined percentile rank.
**Figure 1A** illustrates ELISA signal score of male ADD/ADHD patients and control tested with cantaloupe.
**Figure 1B** illustrates a distribution of percentage of male ADD/ADHD subjects exceeding the 90^{th} and 95^{th} percentile tested with cantaloupe.
**Figure 1C** illustrates a signal distribution in women along with the 95^{th} percentile cutoff as determined from the female control population tested with cantaloupe.
**Figure 1D** illustrates a distribution of percentage of female ADD/ADHD subjects exceeding the 90^{th} and 95^{th} percentile tested with cantaloupe.
**Figure 2A** illustrates ELISA signal score of male ADD/ADHD patients and control tested with wheat.
**Figure 2B** illustrates a distribution of percentage of male ADD/ADHD subjects exceeding the 90^{th} and 95^{th} percentile tested with wheat.
**Figure 2C** illustrates a signal distribution in women along with the 95^{th} percentile cutoff as determined from the female control population tested with wheat.
**Figure 2D** illustrates a distribution of percentage of female ADD/ADHD subjects exceeding the 90^{th} and 95^{th} percentile tested with wheat.
**Figure 3A** illustrates ELISA signal score of male ADD/ADHD patients and control tested with tomato.
**Figure 3B** illustrates a distribution of percentage of male ADD/ADHD subjects exceeding the 90^{th} and 95^{th} percentile tested with tomato.
**Figure 3C** illustrates a signal distribution in women along with the 95^{th} percentile cutoff as determined from the female control population tested with tomato.
**Figure 3D** illustrates a distribution of percentage of female ADD/ADHD subjects exceeding the 90^{th} and 95^{th} percentile tested with tomato.
**Figure 4A** illustrates ELISA signal score of male ADD/ADHD patients and control tested with cucumber.
**Figure 4B** illustrates a distribution of percentage of male ADD/ADHD subjects exceeding the 90^{th} and 95^{th} percentile tested with cucumber.
**Figure 4C** illustrates a signal distribution in women along with the 95^{th} percentile cutoff as determined from the female control population tested with cucumber.
**Figure 4D** illustrates a distribution of percentage of female ADD/ADHD subjects exceeding the 90^{th} and 95^{th} percentile tested with cucumber.
**Figure 5A** illustrates distributions of ADD/ADHD subjects by number of foods that were identified as trigger foods at the 90^{th} percentile.
**Figure 5B** illustrates distributions of ADD/ADHD subjects by number of foods that were identified as trigger foods at the 95^{th} percentile.
**Table 5A** shows raw data of ADD/ADHD patients and control with number of positive results based on the 90^{th} percentile.
**Table 5B** shows raw data of ADD/ADHD patients and control with number of positive results based on the 95^{th} percentile.
**Table 6A** shows statistical data summarizing the raw data of ADD/ADHD patient populations shown in Table SA.
**Table 6B** shows statistical data summarizing the raw data of ADD/ADHD patient populations shown in Table 5B.
**Table 7A** shows statistical data summarizing the raw data of control populations shown in Table SA.
**Table 7B** shows statistical data summarizing the raw data of control populations shown in Table 5B.
**Table 8A** shows statistical data summarizing the raw data of ADD/ADHD patient populations shown in Table 5A transformed by logarithmic transformation.
**Table 8B** shows statistical data summarizing the raw data of ADD/ADHD patient populations shown in Table 5B transformed by logarithmic transformation.
**Table 9A** shows statistical data summarizing the raw data of control populations shown in Table 5A transformed by logarithmic transformation.
**Table 9B** shows statistical data summarizing the raw data of control populations shown in Table 5B transformed by logarithmic transformation.
**Table 10A** shows statistical data of an independent T-test to compare the geometric mean number of positive foods between the ADD/ADHD and non-ADD/ADHD samples based on the 90^{th} percentile.
**Table 10B** shows statistical data of an independent T-test to compare the geometric mean number of positive foods between the ADD/ADHD and non-ADD/ADHD samples based on the 95^{th} percentile.
**Table 11A** shows statistical data of a Mann-Whitney test to compare the geometric mean number of positive foods between the ADD/ADHD and non-ADD/ADHD samples based on the 90^{th} percentile.
**Table 11B** shows statistical data of a Mann-Whitney test to compare the geometric mean number of positive foods between the ADD/ADHD and non-ADD/ADHD samples based on the 95^{th} percentile.

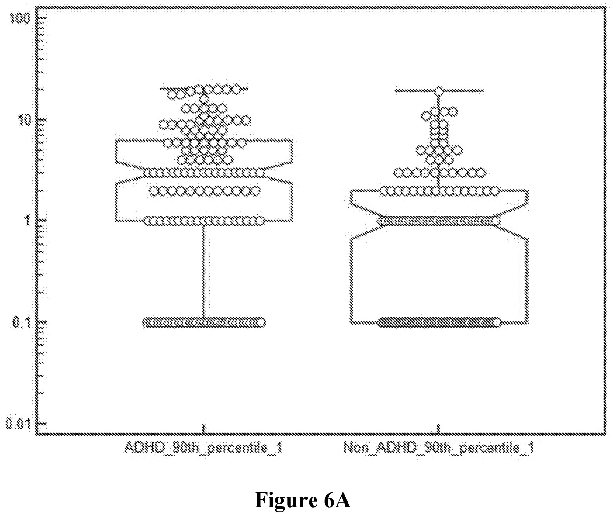

**Figure 6A** illustrates a box and whisker plot of data shown in Table 5A.

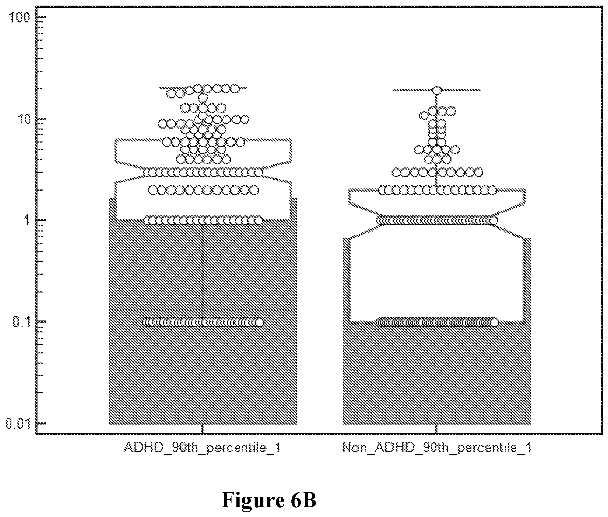

**Figure 6B** illustrates a notched box and whisker plot of data shown in Table 5A.

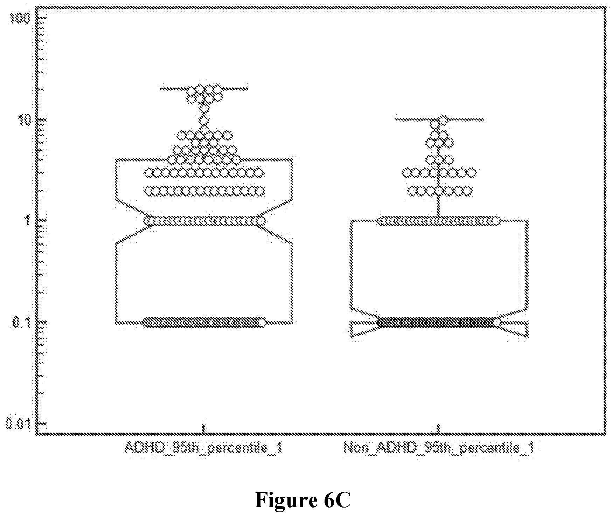

**Figure 6C** illustrates a box and whisker plot of data shown in Table 5B.

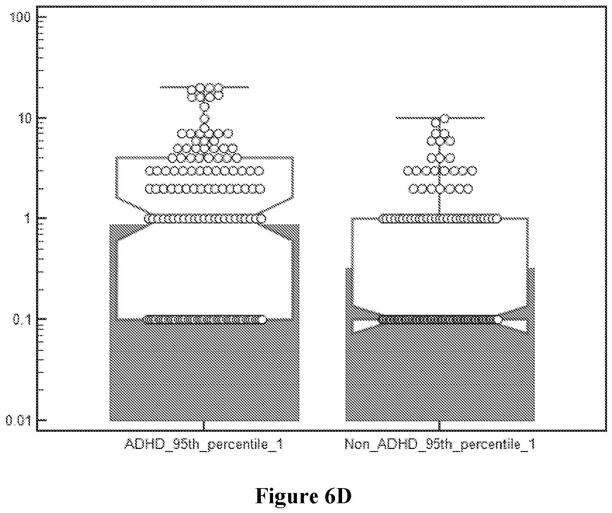

**Figure 6D** illustrates a notched box and whisker plot of data shown in Table 5B.
**Table 12A** shows statistical data of a Receiver Operating Characteristic (ROC) curve analysis of data shown in Tables 5A-11A.
**Table 12B** shows statistical data of a Receiver Operating Characteristic (ROC) curve analysis of data shown in Tables 5B-11B.

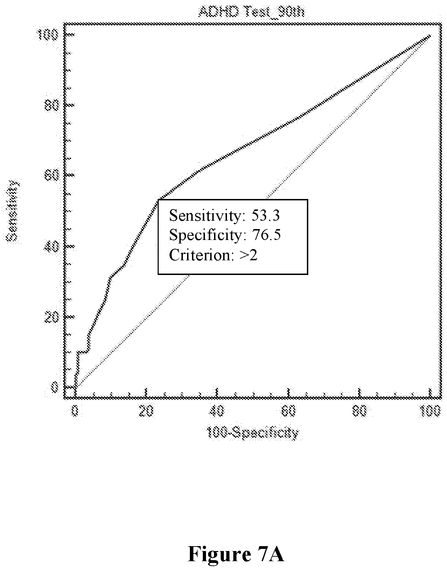

**Figure 7A** illustrates the ROC curve corresponding to the statistical data shown in Table 12A.

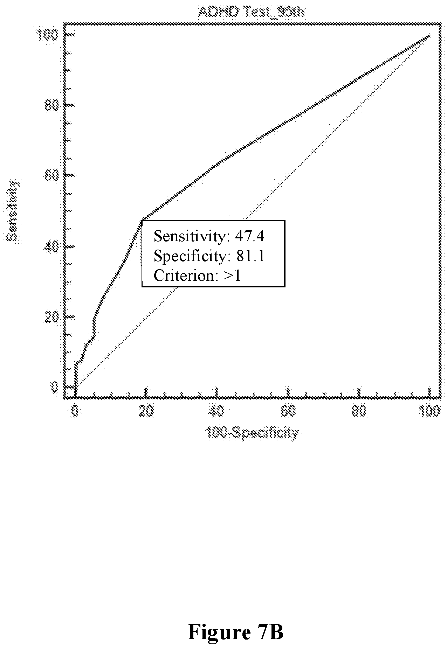

**Figure 7B** illustrates the ROC curve corresponding to the statistical data shown in Table 12B.
**Table 13A** shows a statistical data of performance metrics in predicting ADD/ADHD status among female patients from number of positive foods based on the 90^{th} percentile.
**Table 13B** shows a statistical data of performance metrics in predicting ADD/ADHD status among male patients from number of positive foods based on the 90^{th} percentile.
**Table 14A** shows a statistical data of performance metrics in predicting ADD/ADHD status among female patients from number of positive foods based on the 95^{th} percentile.
**Table 14B** shows a statistical data of performance metrics in predicting ADD/ADHD status among male patients from number of positive foods based on the 95^{th} percentile.

### Detailed Description

The inventors have discovered that food preparations used in food tests to identify trigger foods in patients diagnosed with or suspected of having ADD/ADHD are not equally well predictive and/or associated with ADD/ADHD symptoms. Indeed, various experiments have revealed that among a wide variety of food items, certain food items are highly predictive/associated with ADD/ADHD whereas others have no statistically significant association with ADD/ADHD.

Even more unexpectedly, the inventors discovered that in addition to the high variability of food items, gender variability with respect to response in a test plays a substantial role in the determination of association of a food item with ADD/ADHD. Consequently, based on the inventors' findings and further contemplations, test kits, detection apparatus or devices, array, chip or test panel and methods of using same are now presented with substantially higher predictive power in the choice of food items (i.e., trigger foods) that could be eliminated for reduction of ADD/ADHD signs and symptoms.

The following discussion provides many example embodiments of the inventive subject matter. Although each embodiment represents a single combination of inventive elements, the inventive subject matter is considered to include all possible combinations of the disclosed elements. Thus if one embodiment comprises elements A, B, and C, and a second embodiment comprises elements B and D, then the inventive subject matter is also considered to include other remaining combinations of A, B, C, or D, even if not explicitly disclosed.

In some embodiments, the numbers expressing quantities or ranges, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors necessarily resulting from the standard deviation found in their respective testing measurements. Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

The term "average discriminatory p-value", as used herein, generally refers to an average of all the p-values with a particular probability (e.g., ≤0.05) as determined by raw p-value or with a particular probability (e.g., ≤0.1) as determined by FDR multiplicity adjusted p-value that were identified by analytical methods described herein, e.g., the analytical methods that produced the results summarized in Table 2 (for example, and in particular, foods 1-37 in Table 2). In one embodiment, average discriminatory p-value refers to an average of all the p-values that are ≤0.07 as determined by raw p-value that were identified by analytical methods described herein.

The term "food preparation", as used herein, refers to a solubilized aqueous extraction of a specific food item (e.g., cantaloupe, wheat, milk, etc.). In certain embodiments, the specific food item is solubilized using a blender or similar apparatus, in the presence of a buffer and the food item is processed until the structure of the food item is broken down into a homogenous liquid suspension or solution.

The term "individually addressable", as used herein, refers to a portion of a solid carrier (e.g. an ELISA well, etc.), wherein a food preparation is immobilized (or coupled, etc.) to said portion of the solid carrier in a manner that separates said food preparation from other food preparations immobilized to the solid carrier, and that allows for the detection of an immunoglobulin (e.g., an IgG or other binding molecule) capable of binding to said food preparation (or a component thereof).

The term "one component of the food preparation" or "a component of the food preparation", as used herein, refers to any portion of a food preparation (e.g., a protein(s), a lipid(s), a sugar(s), etc.) that is antigenic (i.e., capable of inducing and/or eliciting an immune response in a subject or patient).

The term "trigger food", as used herein, broadly refers to a food preparation, or a component thereof, which will result in a significantly elevated immune response in a subject (e.g., a patient) exposed to the food preparation, or a component thereof, wherein the elevated immune response is highly correlated to the presence of a disease symptom(s), and which potentially may trigger and/or exacerbate a disease symptom(s), and/or which may potentially result in an alleviation or reduction of symptoms by removing the food preparation, or component thereof.

All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the disclosure and does not pose a limitation on the scope of any embodiments of the disclosure otherwise claimed. No language in the specification should be construed as indicating any non-claimed element essential to the practice of any embodiments of the disclosure.

In some embodiments, the numbers expressing quantities of ingredients, properties such as concentration, reaction conditions, and so forth, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors necessarily resulting from the standard deviation found in their respective testing measurements. Moreover, and unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

While not limiting to the inventive subject matter, food preparations will typically be drawn from foods generally known or suspected to trigger and/or exacerbate signs or symptoms of ADD/ADHD, or which alleviate ADD/ADHD symptomology when removed. Particularly suitable food preparations may be identified by the experimental procedures outlined below.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate certain embodiments of the disclosure and does not pose a limitation on the scope of the any embodiments of the disclosure otherwise claimed. No language in the specification should be construed as indicating any non-claimed element essential to the practice of the disclosure.

Of course, it should be noted that the particular format of the test kit, detection device or apparatus, array, chip, or panel may vary considerably and contemplated formats include micro well plates, dip sticks, membrane-bound arrays, etc. Consequently, the solid carrier to which the food preparations are coupled may include wells of a multiwell plate, a (e.g., color-coded or magnetic) bead, or an adsorptive film (e.g., nitrocellulose or micro/nanoporous polymeric film), or an electrical sensor, (*e.g*., a printed copper sensor or microchip).

While in certain embodiments food preparations are prepared from single food items as crude extracts, or crude filtered extracts, it is contemplated that food preparations can be prepared from mixtures of a plurality of food items (*e.g*., a mixture of citrus comprising lemon, orange, and a grapefruit, a mixture of yeast comprising baker's yeast and brewer's yeast, a mixture of rice comprising a brown rice and white rice, a mixture of sugars comprising honey, malt, and cane sugar. In some embodiments, it is also contemplated that food preparations can be prepared from purified food antigens or recombinant food antigens.

As provided in certain embodiments, the food preparation is immobilized on a solid surface (typically in an addressable manner), accordingly, it is contemplated that the step of measuring the IgG bound to the component of the food preparation is performed via an ELISA test. Exemplary solid surfaces include, but are not limited to, wells in a multiwell plate, such that each food preparation may be isolated to a separate microwell. In certain embodiments, the food preparation will be coupled to, or immobilized on, the solid surface. In other embodiments, the food preparations) will be coupled to a molecular tag that allows for binding to human immunoglobulins (*e.g*., IgG) in solution. See, e.g., Example 3.

Thus, it should be appreciated that by having a high-confidence test system as described herein, the rate of false-positive and false negatives can be significantly reduced, and especially where the test systems and methods are gender stratified or adjusted for gender differences as shown below. Such advantages have heretofore not been realized and it is expected that the systems and methods presented herein will substantially increase the predictive power of food sensitivity tests for patients diagnosed with or suspected of having ADD/ADHD.

### Examples

Example 1: General Protocol for food preparation generation: Commercially available food extracts (available from Biomerica Inc., 17571 Von Karman Ave, Irvine, CA 92614) prepared from the edible portion of the respective raw foods were used to prepare ELISA plates following the manufacturer's instructions.

For some food extracts, the inventors expect that food extracts prepared with certain specific procedures to generate the food extracts, provides more superior results in detecting elevated immunoglobulin (e.g., IgG) reactivity in ADD/ADHD patients compared to commercially available food extracts. For example, in certain embodiments related to grains and nuts, a three-step procedure of generating food extracts may be used. The first step is a defatting step. In this step, lipids from grains and nuts are extracted by contacting the flour of grains and nuts with a non-polar solvent and collecting residue. Then, the defatted grain or nut flour are extracted by contacting the flour with elevated pH to obtain a mixture and removing the solid from the mixture to obtain the liquid extract. Once the liquid extract is generated, the liquid extract is stabilized by adding an aqueous formulation. In certain embodiments, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For another example, in certain embodiments related to meats and fish, a two-step procedure of generating food extracts may be used. The first step is an extraction step. In this step, extracts from raw, uncooked meats or fish are generated by emulsifying the raw, uncooked meats or fish in an aqueous buffer formulation in a high impact pressure processor. Then, solid materials are removed to obtain liquid extract. Once the liquid extract is generated, the liquid extract is stabilized by adding an aqueous formulation. In certain embodiments, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For still another example, in certain embodiments related to fruits and vegetables, a two-step procedure of generating food extracts may be used. The first step is an extraction step. In this step, liquid extracts from fruits or vegetables are generated using an extractor (e.g., masticating juicer, etc.) to pulverize foods and extract juice. Then, solid materials are removed to obtain liquid extract. Once the liquid extract is generated, the liquid extract is stabilized by adding an aqueous formulation. In certain embodiments, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

Example 2: Blocking of ELISA plates: To optimize signal to noise, plates will be blocked with a blocking buffer. In one embodiment, the blocking buffer includes 20-50 mM of a phosphate buffer (pH 4-9), bovine serum albumin (BSA) and a polyvinyl alcohol (PVA).

Example 3: ELISA preparation and sample testing: Food antigen preparations (i.e., food preparations) were immobilized onto respective microtiter wells following the manufacturer's instructions. For the assays, the food antigens (i.e., food preparations) were allowed to react with antibodies present in the patients' serum (e.g., IgG), and excess serum proteins were removed by a wash step. For detection of IgG antibody binding, enzyme labeled anti-IgG antibody conjugate was allowed to react with antigen-antibody complex. A color was developed by the addition of a substrate that reacts with the coupled enzyme. The color intensity was measured and is directly proportional to the concentration of IgG antibody specific to a particular food antigen.

Example 4: Methodology to determine ranked food list in order of ability of ELISA signals to distinguish ADD/ADHD from control subjects: Out of an initial selection (e.g*.*, 100 food items, or 150 food items, or even more), samples can be eliminated prior to analysis due to low consumption in an intended, or target population. In addition, specific food items can be used as being representative of the larger more generic food group, especially where prior testing has established a correlation among different species within a generic group (e.g. in both genders, but also suitable for correlation for a single gender). For example, in one embodiment, green pepper could be dropped in favor of chili pepper as representative of the "pepper" food group, or sweet potato could be dropped in favor of potato as representative of the "potato" food group. In other embodiments, the final list foods will be less than 50 food items. In another embodiment, the final list of foods will be equal to or less than of 40 food items. In other embodiments, the final list of foods is selected from the group consisting of less than 50 food items, less than 49 food items, less than 48 food items, less than 47 food items, less than 46 food items, less than 45 food items, less than 44 food items, less than 43 food items, less than 42 food items, less than 41 food items, less than 40 food items, less than 39 food items, less than 38 food items, less than 37 food items, less than 36 food items, less than 35 food items, less than 34 food items, less than 33 food items, less than 32 food items, less than 31 food items, less than 30 food items, less than 29 food items, less than 28 food items, less than 27 food items, less than 26 food items, less than 25 food items, less than 24 food items, less than 23 food items, less than 22 food items, less than 21 food items, less than 20 food items less than 19 food items, less than 18 food items, less than 17 food items, less than 16 food items, less than 15 food items, less than 14 food items, less than 13 food items, less than 12 food items, less than 11 food items, less than 10 food items, less than 9 food items, less than 8 food items, less than 7 food items, less than 6 food items, less than 5 food items, less than 4 food items, less than 3 food items, and less than 2 food items.

Since the foods ultimately selected for the food panel will not be specific for a particular gender, a gender-neutral food list is necessary. Since the observed sample will be at least initially imbalanced by gender (*e.g.*, Controls: 38.6% female, ADD/ADHD: 67.1% female), differences in ELISA signal magnitude strictly due to gender will be removed by modeling signal scores against gender using a two-sample t-test and storing the residuals for further analysis. For each of the tested foods (i.e., food preparations), residual signal scores will be compared between ADD/ADHD and controls using a permutation test on a two-sample t-test with a relative high number of resamplings (*e.g*., in certain embodiments >1,000 resamplings; in other embodiments >10,000 resamplings; in yet other embodiments >50,000 resamplings). The Satterthwaite approximation can then be used for the denominator degrees of freedom to account for lack of homogeneity of variances, and the 2-tailed permuted p-value will represent the raw p-value for each food. False Discovery Rates (FDR) among the comparisons, will be adjusted by any acceptable statistical procedures (*e.g*., Benjamini-Hochberg, Family-wise Error Rate (FWER), Per Comparison Error Rate (PCER), etc.).

Foods (i.e., food preparations) were then ranked according to their 2-tailed FDR multiplicity-adjusted p-values. Foods with adjusted p-values equal to or lower than the desired FDR threshold are deemed to have significantly higher signal scores among ADD/ADHD than control subjects and therefore deemed candidates for inclusion into a food panel. A typical result that is representative of the outcome of the statistical procedure is provided in **Table 2.** Here the ranking of foods is according to 2-tailed permutation T-test p-values with FDR adjustment.

Based on earlier experiments, the inventors contemplate that even for the same food preparation tested, the ELISA score for at least several food items (i.e., food preparations) will vary dramatically, and exemplary raw data are provided in **Table 3.** As should be readily appreciated, data unstratified by gender will therefore lose significant explanatory power where the same cutoff value is applied to raw data for male and female data. To overcome such disadvantage, the inventors therefore contemplate stratification of the data by gender as described below.

Example 5: Statistical Method for Cutpoint Selection for each Food: The determination of what ELISA signal scores would constitute a "positive" response can be made by summarizing the distribution of signal scores among the Control subjects. For each food (i.e., food preparations), ADD/ADHD subjects who have observed scores greater than or equal to selected quantiles of the Control subject distribution will be deemed "positive". To attenuate the influence of any one subject on cutpoint determination, each food-specific and gender-specific dataset will be bootstrap resampled 1000 times. Within each bootstrap replicate, the 90th and 95th percentiles of the Control signal scores will be determined. Each ADD/ADHD subject in the bootstrap sample will be compared to the 90th and 95% percentiles to determine whether he/she had a "positive" response. The final 90th and 95th percentile-based cutpoints for each food and gender will be computed as the average 90th and 95th percentiles across the 1000 samples. The number of foods for which each ADD/ADHD subject will be rated as "positive" was computed by pooling data across foods. Using such method, the inventors will be now able to identify cutoff values for a predetermined percentile rank that in most cases was substantially different as can be taken from **Table 4.**

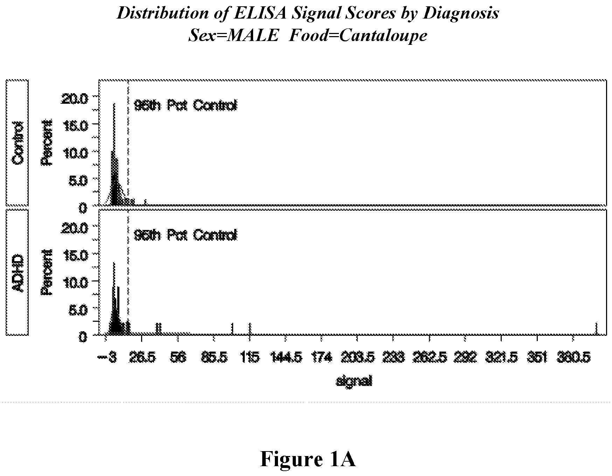

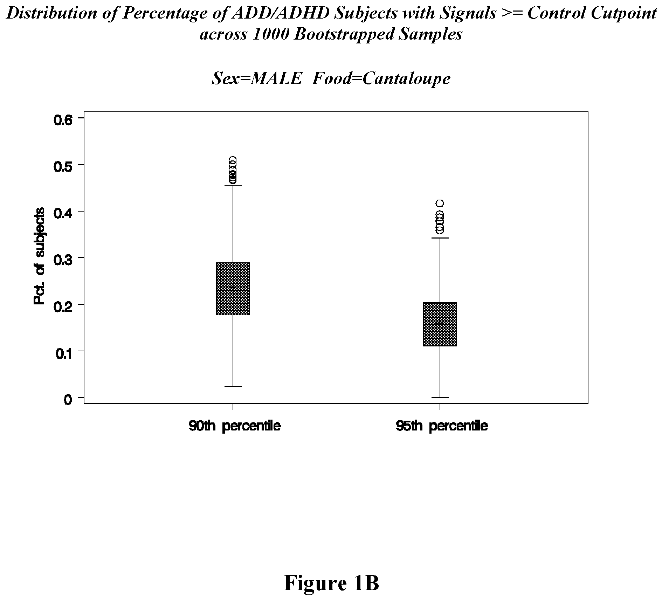

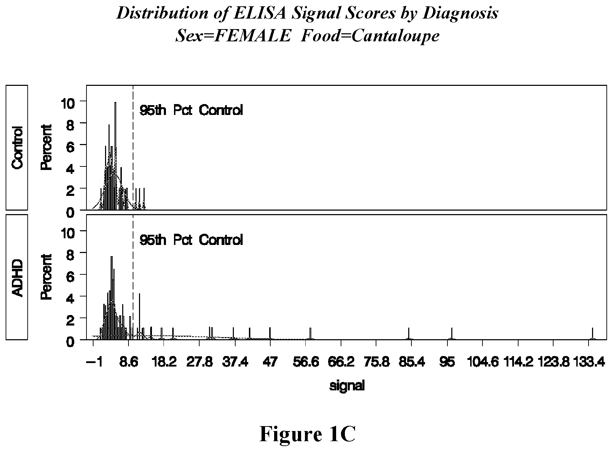

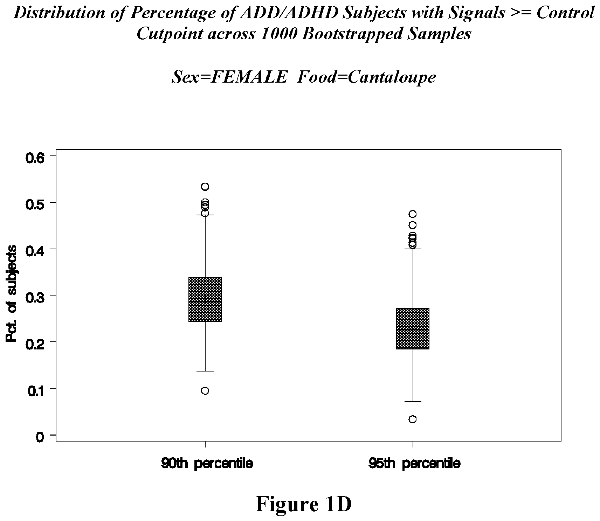

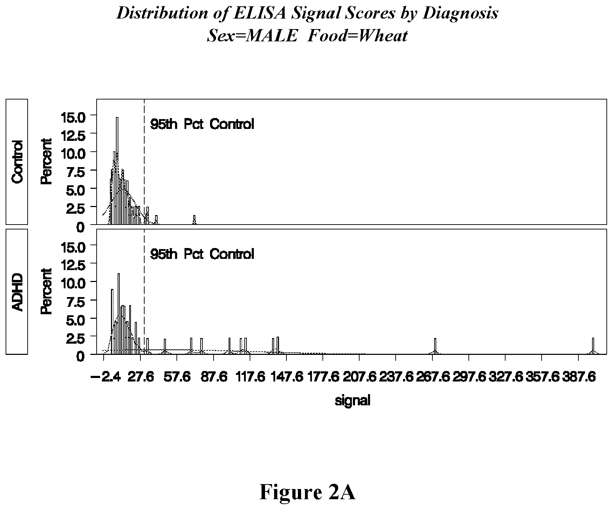

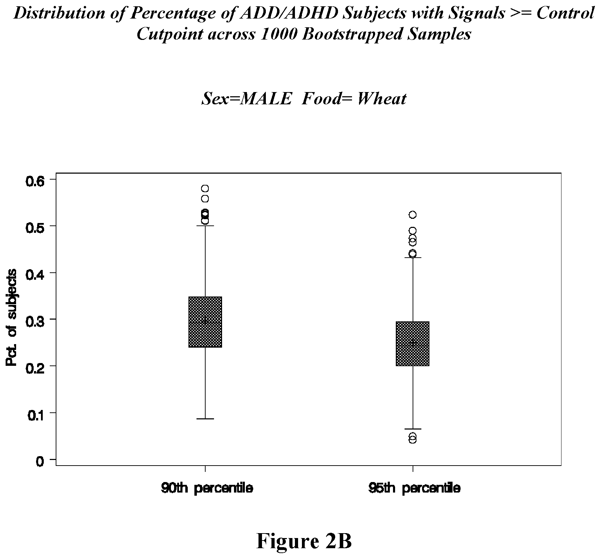

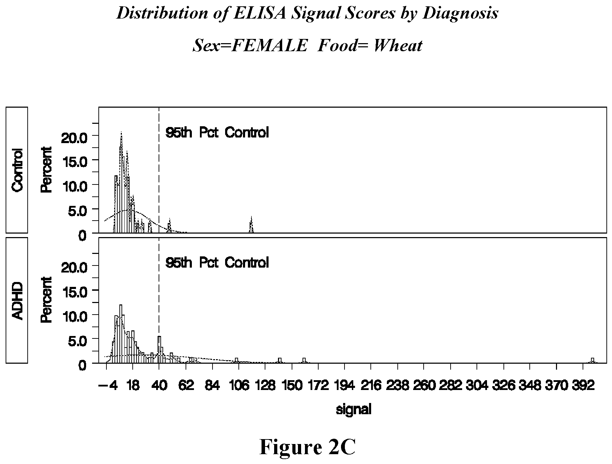

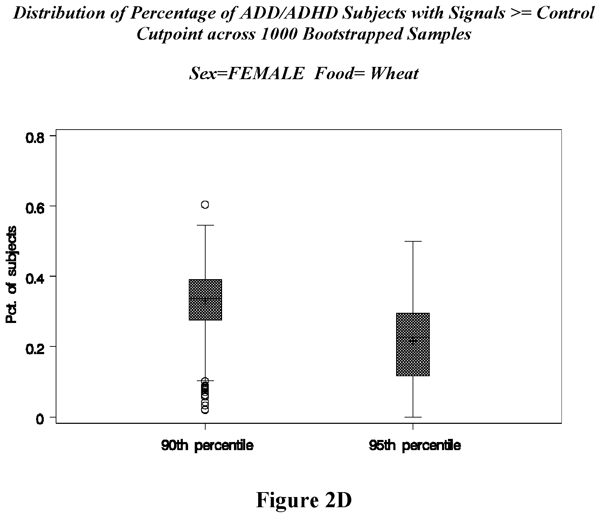

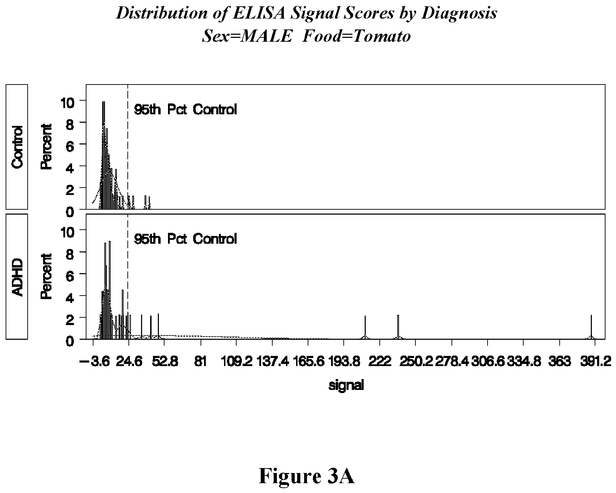

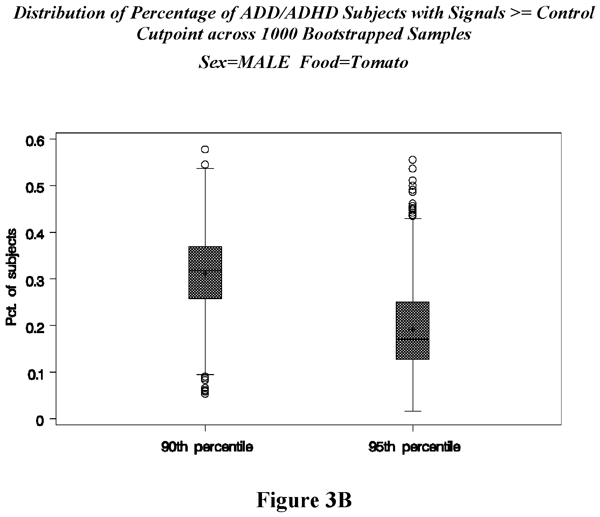

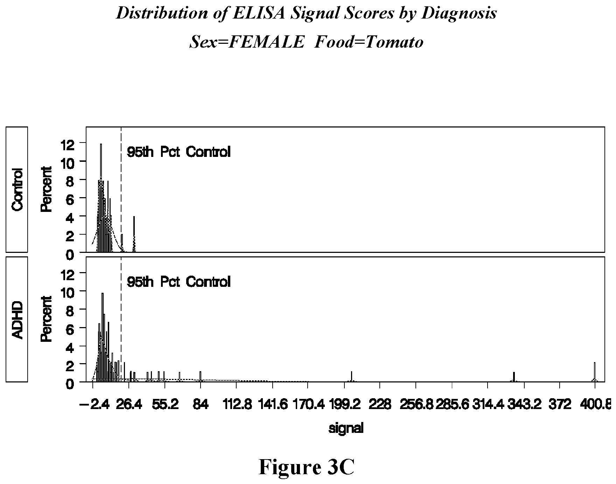

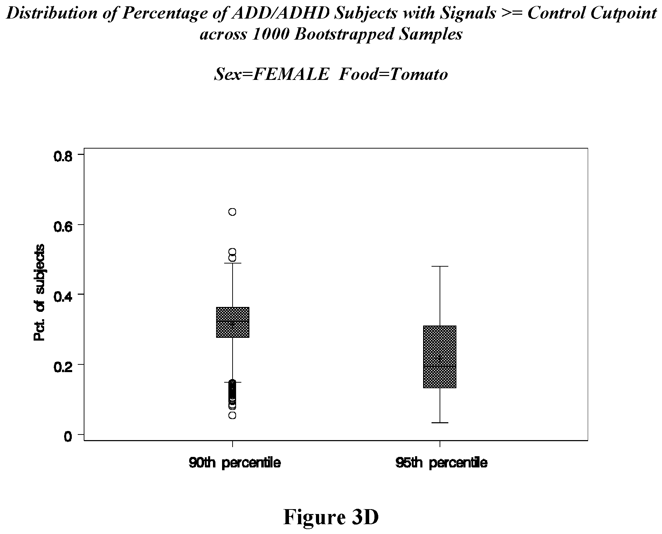

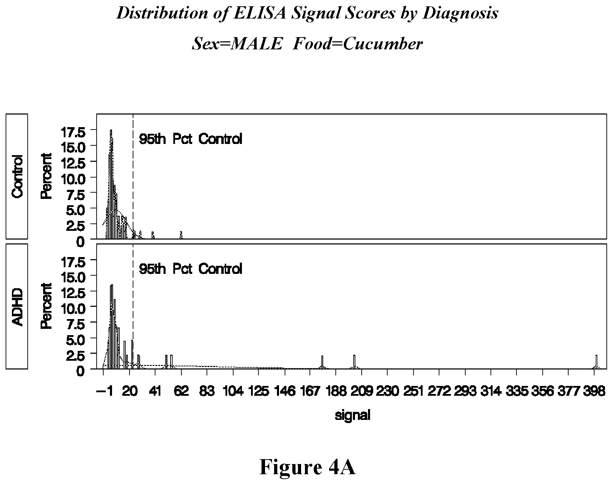

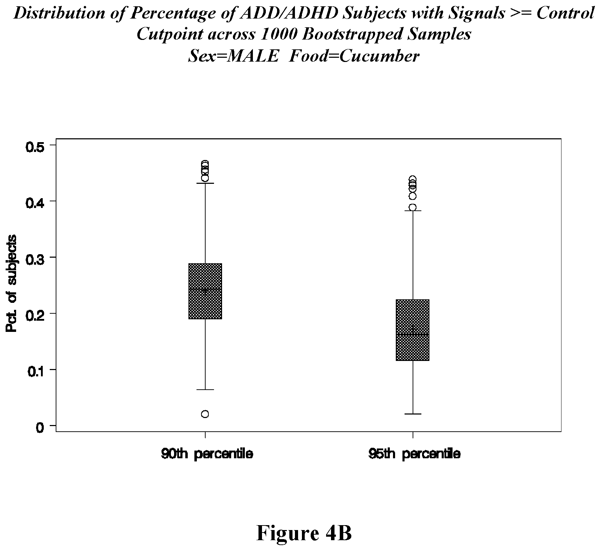

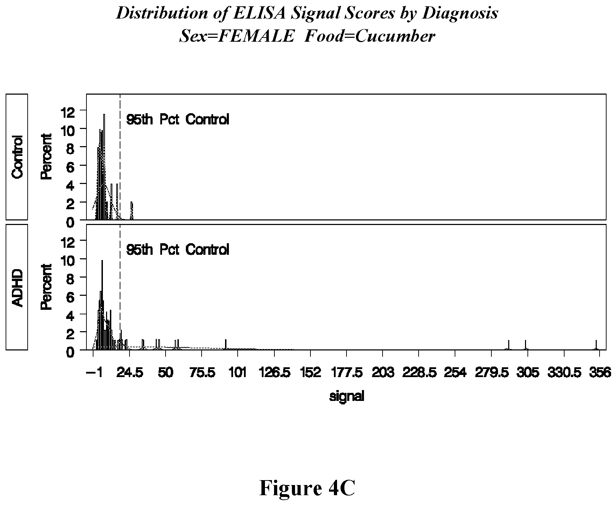

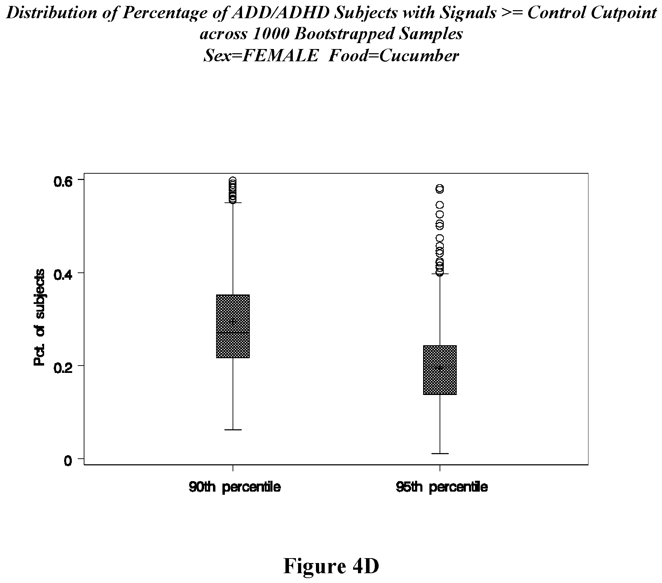

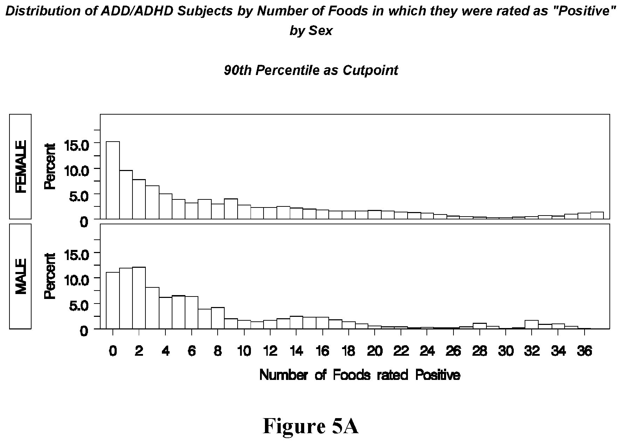

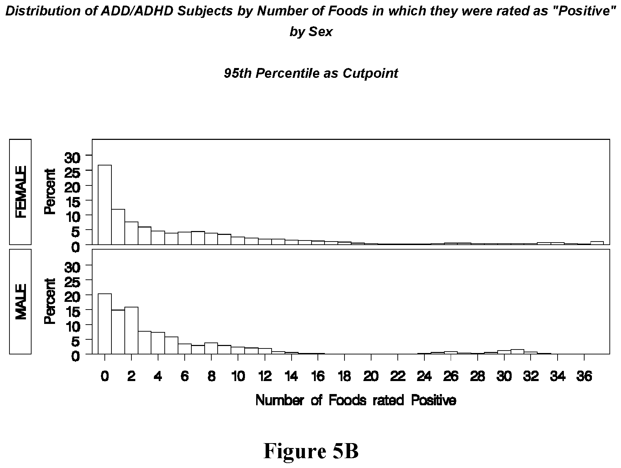

Typical examples for the gender difference in IgG response in blood with respect to cantaloupe is shown in **Figures 1A-1D****,** where Figure 1A shows the signal distribution in men along with the 95^{th} percentile cutoff as determined from the male control population. Figure 1B shows the distribution of percentage of male ADD/ADHD subjects exceeding the 90^{th} and 95^{th} percentile, while Figure 1C shows the signal distribution in women along with the 95^{th} percentile cutoff as determined from the female control population. Figure 1D shows the distribution of percentage of female ADD/ADHD subjects exceeding the 90^{th} and 95^{th} percentile. In the same fashion, **Figures 2A-2D** exemplarily depict the differential response to wheat, **Figures 3A-3D** exemplarily depict the differential response to tomato, and **Figures 4A-4D** exemplarily depict the differential response to cucumber. **Figures 5A-5B** show the distribution of ADD/ADHD subjects by number of foods that were identified as trigger foods at the 90^{th} percentile (SA) and 95^{th} percentile (5B). Inventors contemplate that regardless of the particular food items (i.e., food preparations), male and female responses will be notably distinct.

It should be noted that nothing in the art have provided any predictable food groups related to ADD/ADHD that is gender-stratified. Thus, a discovery of food items (i.e., food preparations) that show distinct responses by gender is a surprising result, which could not be obviously expected in view of all previously available arts. In other words, selection of food items based on gender stratification provides an unexpected technical effect such that statistical significances for particular food items as triggering food among male or female ADD/ADHD patients have been significantly improved.

Example 6: Normalization of IgG Response Data: While the raw data of the patient's IgG response results can be used to compare strength of response among given foods, it is also contemplated that the IgG response results of a patient are normalized and indexed to generate unit-less numbers for comparison of relative strength of response to a given food. For example, one or more of a patient's food specific IgG results (*e.g*., IgG specific to orange and IgG specific to malt) can be normalized to the patient's total IgG. The normalized value of the patient's IgG specific to orange can be 0.1 and the normalized value of the patient's IgG specific to malt can be 0.3. In this scenario, the relative strength of the patient's response to malt is three times higher compared to orange. Then, the patient's sensitivity to malt and orange can be indexed as such.

In other examples, one or more of a patient's food specific IgG results (*e.g*., IgG specific to shrimp and IgG specific to pork) can be normalized to the global mean of that patient's food specific IgG results. The global means of the patient's food specific IgG can be measured by total amount of the patient's food specific IgG. In this scenario, the patient's specific IgG to shrimp can be normalized to the mean of patient's total food specific IgG (*e.g.,* mean of IgG levels to shrimp, pork, Dungeness crab, chicken, peas, etc.) . However, it is also contemplated that the global means of the patient's food specific IgG can be measured by the patient's IgG levels to a specific type of food via multiple tests. If the patient has been tested for his sensitivity to shrimp five times and to pork seven times previously, the patient's new IgG values to shrimp or to pork are normalized to the mean of five-times test results to shrimp or the mean of seven-times test results to pork. The normalized value of the patient's IgG specific to shrimp can be 6.0 and the normalized value of the patient's IgG specific to pork can be 1.0. In this scenario, the patient has six times higher sensitivity to shrimp at this time compared to his average sensitivity to shrimp, but substantially similar sensitivity to pork. Then, the patient's sensitivity to shrimp and pork can be indexed based on such comparison.

Example 7: Methodology to determine the subset of ADD/ADHD patients with food sensitivities that underlie ADD/ADHD: While it is suspected that food sensitivities plays a substantial role in signs and symptoms of ADD/ADHD, some ADD/ADHD patients may not have food sensitivities that underlie ADD/ADHD. Those patients would not be benefit from dietary intervention to treat signs and symptoms of ADD/ADHD. To determine the subset of such patients, body fluid samples of ADD/ADHD patients and non- ADD/ADHD patients can be tested with ELISA test using test devices with up to 37 food samples.

**Table 5A** and **Table 5B** provide exemplary raw data. As should be readily appreciated, the data indicate number of positive results out of 37 sample foods based on 90^{th} percentile value (**Table 5A**) or 95^{th} percentile value (**Table 5B**). The first column is ADD/ADHD (n=137), second column is non-ADD/ADHD (n=132) by ICD-10 code. Average and median number of positive foods was computed for ADD/ADHD and non-ADD/ADHD patients. From the raw data shown in **Table 5A** and **Table 5B**, average and standard deviation of the number of positive foods (i.e., trigger foods) was computed for ADD/ADHD and non-ADD/ADHD patients. Additionally, the number and percentage of patients with zero positive foods was calculated for both ADD/ADHD and non-ADD/ADHD. The number and percentage of patients with zero positive foods in the ADD/ADHD population is approximately 40% lower than the percentage of patients with zero positive foods in the non- ADD/ADHD population (24.1% vs. 38.6%, respectively) based on 90^{th} percentile value (**Table 5A**), and the percentage of patients in the ADD/ADHD population with zero positive foods is also significantly lower (i.e. approximately 40% lower) than that seen in the non-ADD/ADHD population (35.8% vs. 59.1%, respectively) based on 95^{th} percentile value (**Table 5B**). Thus, it can be easily appreciated that the ADD/ADHD patient having sensitivity to zero positive foods is unlikely to have food sensitivities underlying their signs and symptoms of ADD/ADHD.

**Table 6A** and **Table 7A** show exemplary statistical data summarizing the raw data of two patient populations shown in Table 5A. The statistical data includes normality, arithmetic mean, median, percentiles and 95% confidence interval (CI) for the mean and median representing number of positive foods (i.e., trigger foods) in the ADD/ADHD population and the non-ADD/ADHD population **Table 6B** and **Table 7B** show exemplary statistical data summarizing the raw data of two patient populations shown in Table 5B. The statistical data includes normality, arithmetic mean, median, percentiles and 95% confidence interval (CI) for the mean and median representing number of positive foods in the ADD/ADHD population and the non-ADD/ADHD population.

**Table 8A** and **Table 9A** show exemplary statistical data summarizing the raw data of two patient populations shown in Table 5A. In Tables 8A and 9A, the raw data was transformed by logarithmic transformation to improve the data interpretation. **Table 8B** and **Table 9B** show another exemplary statistical data summarizing the raw data of two patient populations shown in Table 5B. In Tables 8B and 9B, the raw data was transformed by logarithmic transformation to improve the data interpretation.

**Table 10A** and **Table 11A** show exemplary statistical data of an independent T-test (Table 10A, logarithmically transformed data) and a Mann-Whitney test (Table 11A) to compare the geometric mean number of positive foods (i.e., trigger foods) between the ADD/ADHD and non-ADD/ADHD samples. The data shown in Table 10A and Table 11A indicate statistically significant differences in the geometric mean of positive number of foods between the ADD/ADHD population and the non-ADD/ADHD population. In both statistical tests, it is shown that the number of positive responses with 37 food samples is significantly higher in the ADD/ADHD population than in the non-ADD/ADHD population with an average discriminatory p-value of ≤ 0.0001. These statistical data is also illustrated as a box and whisker plot in **Figure 6A****,** and a notched box and whisker plot in **Figure 6B****.**

**Table 10B** and **Table 11B** show exemplary statistical data of an independent T-test (Table 10A, logarithmically transformed data) and a Mann-Whitney test (Table 11B) to compare the geometric mean number of positive foods (i.e., trigger foods) between the ADD/ADHD and non-ADD/ADHD samples. The data shown in Table 10B and Table 11B indicate statistically significant differences in the geometric mean of positive number of foods between the ADD/ADHD population and the non-ADD/ADHD population. In both statistical tests, it is shown that the number of positive responses with 37 food samples is significantly higher in the ADD/ADHD population than in the non-ADD/ADHD population with an average discriminatory p-value of < 0.0001. These statistical data is also illustrated as a box and whisker plot in **Figure 6C****,** and a notched box and whisker plot in **Figure 6D****.**

**Table 12A** shows exemplary statistical data of a Receiver Operating Characteristic (ROC) curve analysis of data shown in Tables 5A-11A to determine the diagnostic power of the test used in Table 5 at discriminating ADD/ADHD from non- ADD/ADHD subjects. When a cutoff criterion of more than 2 positive foods (i.e., trigger foods) is used, the test yields a data with 53.3% sensitivity and 76.5% specificity, with an area under the curve (AUROC) of 0.660. The p-value for the ROC is significant at a p-value of <0.0001. **Figure 7A** illustrates the ROC curve corresponding to the statistical data shown in Table 12A. Because the statistical difference between the ADD/ADHD population and the non-ADD/ADHD population is significant when the test results are cut off to a positive number of 2, the number of foods for which a patient tests positive could be used as a confirmation of the primary clinical diagnosis of ADD/ADHD, and whether it is likely that food sensitivities underlies on the patient's signs and symptoms of ADD/ADHD. Therefore, the above test can be used as another 'rule in' test to add to currently available clinical criteria for diagnosis for ADD/ADHD.

As shown in Tables 5A-12A, and Figure 7A, based on 90^{th} percentile data, the number of positive foods (i.e., trigger foods) seen in ADD/ADHD vs. non-ADD/ADHD subjects is significantly different whether the geometric mean or median of the data is compared. The number of positive foods that a person has is indicative of the presence of ADD/ADHD in subjects. The test has discriminatory power to detect ADD/ADHD with 53.3% sensitivity and 76.5% specificity. Additionally, the absolute number and percentage of subjects with 0 positive foods is also very different in ADD/ADHD vs. non-ADD/ADHD subjects, with a far lower percentage of ADD/ADHD subjects (24.1%) having 0 positive foods than non-ADD/ADHD subjects (38.6%). The data suggests a subset of ADD/ADHD patients may have ADD/ADHD due to other factors than diet, and may not benefit from dietary restriction.

**Table 12B** shows exemplary statistical data of a Receiver Operating Characteristic (ROC) curve analysis of data shown in Tables 5B-11B to determine the diagnostic power of the test used in Table 5 at discriminating ADD/ADHD from non-ADD/ADHD subjects. When a cutoff criterion of more than 1 positive foods (i.e., trigger foods) is used, the test yields a data with 47.4% sensitivity and 81.1% specificity, with an area under the curve (AUROC) of 0.659. The p-value for the ROC is significant at a p-value of <0.0001. **Figure 7B** illustrates the ROC curve corresponding to the statistical data shown in Table 12B. Because the statistical difference between the ADD/ADHD population and the non-ADD/ADHD population is significant when the test results are cut off to positive number of >1, the number of foods that a patient tests positive could be used as a confirmation of the primary clinical diagnosis of ADD/ADHD, and whether it is likely that food sensitivities underlies on the patient's signs and symptoms of ADD/ADHD. Therefore, the above test can be used as another 'rule in' test to add to currently available clinical criteria for diagnosis for ADD/ADHD.

As shown in Tables 5B-12B, and Figure 7B, based on 95^{th} percentile data, the number of positive foods (i.e., trigger foods) seen in ADD/ADHD vs. non-ADD/ADHD subjects is significantly different whether the geometric mean or median of the data is compared. The number of positive foods that a person has is indicative of the presence of ADD/ADHD in subjects. The test has discriminatory power to detect ADD/ADHD with 47.4% sensitivity and 81.1% specificity. Additionally, the absolute number and percentage of subjects with 0 positive foods is also very different in ADD/ADHD vs. non-ADD/ADHD subjects, with a far lower percentage of ADD/ADHD subjects (35.8%) having 0 positive foods than non- ADD/ADHD subjects (59.1%). The data suggests a subset of ADD/ADHD patients may have ADD/ADHD due to other factors than diet, and may not benefit from dietary restriction.

Example 8: Method for determining distribution of per-person number of foods declared "positive": To determine the distribution of number of "positive" foods (i.e., trigger foods) per person and measure the diagnostic performance, the analysis will be performed with 37 food items from Table 2, which shows most positive responses to ADD/ADHD patients. To attenuate the influence of any one subject on this analysis, each food-specific and gender-specific dataset will be bootstrap resampled 1000 times. Then, for each food item in the bootstrap sample, sex-specific cutpoint will be determined using the 90th and 95th percentiles of the control population. Once the sex-specific cutpoints are determined, the sex-specific cutpoints will be compared with the observed ELISA signal scores for both control and ADD/ADHD subjects. In this comparison, if the observed signal is equal or more than the cutpoint value, then it will be determined "positive" food, and if the observed signal is less than the cutpoint value, then it will be determined "negative" food.

Once all food items (i.e., food preparations) were determined either positive or negative, the results of the 74 (37 foods x 2 cutpoints) calls for each subject will be saved within each bootstrap replicate. Then, for each subject, 37 calls will be summed using 90^{th} percentile as cutpoint to get "Number of Positive Foods (90^{th})," and the rest of 37 calls will be summed using 95^{th} percentile to get "Number of Positive Foods (95^{th})." Then, within each replicate, "Number of Positive Foods (90^{th})" and "Number of Positive Foods (95^{th})" will be summarized across subjects to get descriptive statistics for each replicate as follows: 1) overall means equals to the mean of means, 2) overall standard deviation equals to the mean of standard deviations, 3) overall medial equals to the mean of medians, 4) overall minimum equals to the minimum of minimums, and 5) overall maximum equals to maximum of maximum. In this analysis, to avoid non-integer "Number of Positive Foods" when computing frequency distribution and histogram, the authors will pretend that the 1000 repetitions of the same original dataset were actually 999 sets of new subjects of the same size added to the original sample. Once the summarization of data is done, frequency distributions and histograms will be generated for both "Number of Positive Foods (90^{th})" and "Number of Positive Foods (95^{th})" for both genders and for both ADD/ADHD subjects and control subjects using programs "a_pos_foods.sas, a_pos_foods_by_dx.sas".

Example 9: Method for measuring diagnostic performance: To measure diagnostic performance for each food items (i.e., food preparations ) for each subject, we will use data of "Number of Positive Foods (90^{th})" and "Number of Positive Foods (95^{th})" for each subject within each bootstrap replicate described above. In this analysis, the cutpoint was set to 1. Thus, if a subject has one or more "Number of Positive Foods (90^{th})", then the subject will be called "Has ADD/ADHD." If a subject has less than one "Number of Positive Foods (90^{th})", then the subject will be called "Does Not Have ADD/ADHD." When all calls were made, the calls were compared with actual diagnosis to determine whether a call was a True Positive (TP), True Negative (TN), False Positive (FP), or False Negative (FN). The comparisons will be summarized across subjects to get the performance metrics of sensitivity, specificity, positive predictive value, and negative predictive value for both "Number of Positive Foods (90^{th})" and "Number of Positive Foods(95^{th})" when the cutpoint is set to 1 for each method. Each (sensitivity, 1-specificity) pair becomes a point on the ROC curve for this replicate.

To increase the accuracy, the analysis above will be repeated by incrementing cutpoint from 2 up to 37, and repeated for each of the 1000 bootstrap replicates. Then the performance metrics across the 1000 bootstrap replicates will be summarized by calculating averages using a program "tjpos_foods_by_dx.sas". The results of diagnostic performance for female and male are shown in **Tables 13A and 13B** (90th percentile) and **Tables 14 A and 14B** (95th percentile).

**Table 1**

| | | | |
|---|---|---|---|
| Abalone | Cured Cheese | Onion | Walnut, black |
| Adlay | Cuttlefish | Orange | Watermelon |
| Almond | Duck | Oyster | Welch Onion |
| American Cheese | Durian | Papaya | Wheat |
| Apple | Eel | Paprika | Wheat bran |
| Artichoke | Egg White (separate) | Parsley | Yeast (S. cerevisiae) |
| Asparagus | Egg Yolk (separate) | Peach | Yogurt |
| Avocado | Egg, white/yolk (comb.) | Peanut | |
| Baby Bok Choy | Eggplant | Pear | **FOOD ADDITIVES** |
| Bamboo shoots | Garlic | Pepper, Black | Arabic Gum |
| Banana | Ginger | Pineapple | Carboxymethyl Cellulose |
| Barley, whole grain | Gluten - Gliadin | Pinto bean | Carrageneenan |
| Beef | Goat's milk | Plum | FD&C Blue #1 |
| Beets | Grape, white/concord | Pork | FD&C Red #3 |
| Beta-lactoglobulin | Grapefruit | Potato | FD&C Red #40 |
| Blueberry | Grass Carp | Rabbit | FD&C Yellow #5 |
| Broccoli | Green Onion | Rice | FD&C Yellow #6 |
| Buckwheat | Green pea | Roquefort Cheese | Gelatin |
| Butter | Green pepper | Rye | Guar Gum |
| Cabbage | Guava | Saccharine | Maltodextrin |
| Cane sugar | Hair Tail | Safflower seed | Pectin |
| Cantaloupe | Hake | Salmon | Whey |
| Caraway | Halibut | Sardine | Xanthan Gum |
| Carrot | Hazelnut | Scallop | |
| Casein | Honey | Sesame | |
| Cashew | Kelp | Shark fin | |
| Cauliflower | Kidney bean | Sheep's milk | |
| Celery | Kiwi Fruit | Shrimp | |
| Chard | Lamb | Sole | |
| Cheddar Cheese | Leek | Soybean | |
| Chick Peas | Lemon | Spinach | |
| Chicken | Lentils | Squashes | |
| Chili pepper | Lettuce, Iceberg | Squid | |
| Chocolate | Lima bean | Strawberry | |
| Cinnamon | Lobster | String bean | |
| Clam | Longan | Sunflower seed | |
| Cocoa Bean | Mackerel | Sweet potato | |
| Coconut | Malt | Swiss cheese | |
| Codfish | Mango | Taro | |
| Coffee | Marjoram | Tea, black | |
| Cola nut | Millet | Tobacco | |
| Corn | Mung bean | Tomato | |
| Cottage cheese | Mushroom | Trout | |
| Cow's milk | Mustard seed | Tuna | |
| Crab | Oat | Turkey | |
| Cucumber | Olive | Vanilla | |

### Ranking of Foods according to 2-tailed Permutation T-test p-values with FDR adjustment

**Table 2**

| *Rank* | *Food* | *Raw p-value* | *FDR Multiplicity-adj p-value* |
|---|---|---|---|
| 1 | Cantaloupe | 0.0000 | 0.0017 |
| 2 | Wheat | 0.0000 | 0.0017 |
| 3 | Tomato | 0.0001 | 0.0029 |
| 4 | Cucumber | 0.0001 | 0.0030 |
| 5 | Squashes | 0.0003 | 0.0046 |
| 6 | Almond | 0.0003 | 0.0046 |
| 7 | Egg | 0.0004 | 0.0048 |
| 8 | Cauliflower | 0.0005 | 0.0048 |
| 9 | Pinto_Bean | 0.0005 | 0.0048 |
| 10 | Broccoli | 0.0006 | 0.0048 |
| 11 | Orange | 0.0008 | 0.0063 |
| 12 | Butter | 0.0011 | 0.0077 |
| 13 | Corn | 0.0012 | 0.0077 |
| 14 | Lettuce | 0.0013 | 0.0081 |
| 15 | Rye | 0.0016 | 0.0092 |
| 16 | Peach | 0.0026 | 0.0139 |
| 17 | Green_Pea | 0.0031 | 0.0156 |
| 18 | Carrot | 0.0035 | 0.0168 |
| 19 | Tea | 0.0051 | 0.0233 |
| 20 | Mustard | 0.0058 | 0.0249 |
| 21 | Strawberry | 0.0062 | 0.0253 |
| 22 | Celery | 0.0093 | 0.0351 |
| 23 | Green_Pepper | 0.0094 | 0.0351 |
| 24 | Garlic | 0.0101 | 0.0358 |
| 25 | Oat | 0.0104 | 0.0358 |
| 26 | Onion | 0.0120 | 0.0396 |
| 27 | Banana | 0.0139 | 0.0443 |
| 28 | Eggplant | 0.0156 | 0.0479 |
| 29 | Cabbage | 0.0162 | 0.0480 |
| 30 | Safflower | 0.0168 | 0.0480 |
| 31 | Olive | 0.0175 | 0.0487 |
| 32 | Cottage_Ch_ | 0.0190 | 0.0512 |
| 33 | Grapefruit | 0.0208 | 0.0541 |
| 34 | Walnut_Blk | 0.0243 | 0.0615 |
| 35 | Cow_Milk | 0.0256 | 0.0629 |
| 36 | Soybean | 0.0300 | 0.0717 |
| 37 | Chili_Pepper | 0.0376 | 0.0874 |
| 38 | Tobacco | 0.0481 | 0.1088 |
| 39 | Malt | 0.0507 | 0.1118 |
| 40 | Cinnamon | 0.0545 | 0.1171 |
| 41 | Cane_Sugar | 0.0619 | 0.1299 |
| 42 | Potato | 0.0665 | 0.1362 |
| 43 | Millet | 0.0704 | 0.1407 |
| 44 | Sunflower_Sd | 0.0807 | 0.1577 |
| 45 | Crab | 0.0850 | 0.1625 |
| 46 | Lima_Bean | 0.1080 | 0.2020 |
| 47 | Chocolate | 0.1114 | 0.2038 |
| 48 | Spinach | 0.1205 | 0.2159 |
| 49 | Buck_Wheat | 0.1283 | 0.2241 |
| 50 | Honey | 0.1303 | 0.2241 |
| 51 | Pineapple | 0.1376 | 0.2274 |
| 52 | Yeast_Brewer | 0.1382 | 0.2274 |
| 53 | Rice | 0.1401 | 0.2274 |
| 54 | Apple | 0.1962 | 0.3125 |
| 55 | Mushroom | 0.2001 | 0.3128 |
| 56 | Yeast_Baker | 0.2551 | 0.3918 |
| 57 | Avocado | 0.2707 | 0.4075 |
| 58 | Cheddar_Ch_ | 0.2803 | 0.4075 |
| 59 | Sesame | 0.2821 | 0.4075 |
| 60 | Clam | 0.2843 | 0.4075 |
| 61 | Pork | 0.2982 | 0.4204 |
| 62 | Blueberry | 0.3534 | 0.4903 |
| 63 | Swiss_Ch_ | 0.3654 | 0.4987 |
| 64 | Barley | 0.3841 | 0.5162 |
| 65 | String_Bean | 0.4313 | 0.5707 |
| 66 | Peanut | 0.4626 | 0.6028 |
| 67 | Scallop | 0.4997 | 0.6414 |
| 68 | Lemon | 0.5191 | 0.6565 |
| 69 | Goat_Milk | 0.5378 | 0.6624 |
| 70 | Oyster | 0.5392 | 0.6624 |
| 71 | Lobster | 0.5621 | 0.6809 |
| 72 | Chicken | 0.6743 | 0.8055 |
| 73 | Amer_Cheese | 0.7096 | 0.8312 |
| 74 | Coffee | 0.7152 | 0.8312 |
| 75 | Beef | 0.7616 | 0.8733 |
| 76 | Codfish | 0.8330 | 0.9426 |
| 77 | Salmon | 0.8779 | 0.9646 |
| 78 | Cashew | 0.8950 | 0.9646 |
| 79 | Sardine | 0.8972 | 0.9646 |
| 80 | Parsley | 0.9073 | 0.9646 |
| 81 | Turkey | 0.9086 | 0.9646 |
| 82 | Grape | 0.9376 | 0.9780 |
| 83 | Yogurt | 0.9439 | 0.9780 |
| 84 | Cola_Nut | 0.9742 | 0.9931 |
| 85 | Sweet_Pot_ | 0.9840 | 0.9931 |
| 86 | Trout | 0.9931 | 0.9931 |

### Basic Descriptive Statistics of ELISA Score by Food and Gender Comparing ADD/ADHD to Control

**Table 3**

| | | | | *ELISA Score* | | | |
|---|---|---|---|---|---|---|---|
| *Sex* | *Food* | *Diagnosis* | *N* | *Mean* | *SD* | *Min* | *Max* |
| FEMALE | Almond | Control | 51 | 4.057 | 1.630 | 1.270 | 8.090 |
| | | ADHD | 92 | 6.654 | 7.307 | 0.700 | 49.320 |
| | | Diff (1-2) | _ | -2.597 | 5.950 | _ | _ |
| | Amer_Cheese | Control | 51 | 32.961 | 66.654 | 0.100 | 400.00 |
| | | ADHD | 92 | 32.901 | 33.849 | 1.210 | 148.00 |
| | | Diff (1-2) | _ | 0.060 | 48.113 | _ | _ |
| | Apple | Control | 51 | 4.792 | 6.600 | 0.100 | 44.160 |
| | | ADHD | 92 | 5.578 | 8.466 | 0.110 | 57.720 |
| | | Diff (1-2) | _ | -0.785 | 7.855 | _ | _ |
| | Avocado | Control | 51 | 2.144 | 1.129 | 0.100 | 5.560 |
| | | ADHD | 92 | 3.177 | 4.055 | 0.100 | 33.660 |
| | | Diff (1-2) | _ | -1.032 | 3.326 | _ | _ |
| | Banana | Control | 51 | 3.042 | 2.909 | 0.100 | 17.210 |
| | | ADHD | 92 | 6.176 | 11.816 | 0.100 | 105.04 |
| | | Diff (1-2) | _ | -3.134 | 9.649 | _ | _ |
| | Barley | Control | 51 | 3.867 | 3.919 | 0.100 | 25.110 |
| | | ADHD | 92 | 9.954 | 19.858 | 0.100 | 133.34 |
| | | Diff (1-2) | _ | -6.087 | 16.123 | _ | _ |
| | Beef | Control | 51 | 9.007 | 12.047 | 1.030 | 81.660 |
| | | ADHD | 92 | 12.865 | 40.258 | 1.270 | 380.80 |
| | | Diff (1-2) | _ | -3.858 | 33.128 | _ | _ |
| | Blueberry | Control | 51 | 3.533 | 4.712 | 0.100 | 26.460 |
| | | ADHD | 92 | 4.293 | 5.077 | 0.860 | 34.650 |
| | | Diff (1-2) | _ | -0.760 | 4.951 | _ | _ |
| | Broccoli | Control | 51 | 5.211 | 4.424 | 0.110 | 29.600 |
| | | ADHD | 92 | 9.398 | 10.582 | 2.570 | 91.250 |
| | | Diff (1-2) | _ | -4.187 | 8.900 | _ | _ |
| | Buck_Wheat | Control | 51 | 5.151 | 4.281 | 0.100 | 26.450 |
| | | ADHD | 92 | 6.364 | 7.554 | 0.870 | 60.670 |
| | | Diff (1-2) | _ | -1.213 | 6.583 | _ | _ |
| | Butter | Control | 51 | 17.809 | 24.982 | 0.100 | 150.93 |
| | | ADHD | 92 | 23.143 | 21.663 | 0.740 | 104.00 |
| | | Diff (1-2) | _ | -5.334 | 22.895 | | |
| | Cabbage | Control | 51 | 5.038 | 6.005 | 0.350 | 37.840 |
| | | ADHD | 92 | 8.829 | 14.699 | 0.740 | 121.38 |
| | | Diff (1-2) | _ | -3.791 | 12.338 | _ | |
| | Cane_Sugar | Control | 51 | 15.190 | 10.152 | 3.460 | 50.450 |
| | | ADHD | 92 | 25.370 | 25.407 | 4.050 | 116.64 |
| | | Diff (1-2) | _ | -10.180 | 21.287 | _ | |
| | Cantaloupe | Control | 51 | 4.707 | 2.368 | 1.150 | 12.760 |
| | | ADHD | 92 | 11.342 | 20.203 | 1.070 | 134.48 |
| | | Diff (1-2) | _ | -6.635 | 16.291 | | |
| | Carrot | Control | 51 | 2.702 | 1.549 | 0.100 | 6.940 |
| | | ADHD | 92 | 6.653 | 14.010 | 0.100 | 89.210 |
| | | Diff (1-2) | _ | -3.952 | 11.293 | | |
| | Cashew | Control | 51 | 8.620 | 13.756 | 0.100 | 81.890 |
| | | ADHD | 92 | 9.483 | 19.123 | 0.610 | 111.06 |
| | | Diff (1-2) | _ | -0.863 | 17.410 | | |
| | Cauliflower | Control | 51 | 4.203 | 2.424 | 0.430 | 11.770 |
| | | ADHD | 92 | 7.444 | 11.577 | 1.010 | 94.290 |
| | | Diff (1-2) | _ | -3.241 | 9.412 | | |
| | Celery | Control | 51 | 7.815 | 5.560 | 2.060 | 32.830 |
| | | ADHD | 92 | 11.498 | 13.759 | 2.010 | 86.600 |
| | | Diff (1-2) | _ | -3.683 | 11.539 | | |
| | Cheddar_Ch_ | Control | 51 | 25.261 | 59.384 | 1.530 | 400.00 |
| | | ADHD | 92 | 27.840 | 36.629 | 0.100 | 189.39 |
| | | Diff (1-2) | _ | -2.579 | 46.005 | | |
| | Chicken | Control | 51 | 14.077 | 8.350 | 2.690 | 50.000 |
| | | ADHD | 92 | 16.458 | 13.609 | 2.270 | 79.130 |
| | | Diff (1-2) | _ | -2.381 | 12.010 | | |
| | Chili_Pepper | Control | 51 | 7.281 | 6.348 | 0.570 | 32.360 |
| | | ADHD | 92 | 10.595 | 16.487 | 0.870 | 111.31 |
| | | Diff (1-2) | _ | -3.314 | 13.774 | | |
| | Chocolate | Control | 51 | 13.516 | 6.136 | 3.410 | 30.540 |
| | | ADHD | 92 | 18.291 | 15.883 | 3.950 | 114.88 |
| | | Diff (1-2) | _ | -4.775 | 13.272 | | |
| | Cinnamon | Control | 51 | 8.315 | 6.347 | 1.490 | 38.800 |
| | | ADHD | 92 | 12.609 | 15.262 | 0.490 | 111.50 |
| | | Diff (1-2) | _ | -4.294 | 12.830 | _ | _ |
| | Clam | Control | 51 | 36.890 | 57.603 | 7.450 | 400.00 |
| | | ADHD | 92 | 31.984 | 32.189 | 3.640 | 213.69 |
| | | Diff (1-2) | _ | 4.906 | 42.957 | _ | _ |
| | Codfish | Control | 51 | 27.484 | 34.270 | 6.170 | 203.91 |
| | | ADHD | 92 | 22.764 | 36.339 | 1.270 | 342.06 |
| | | Diff (1-2) | _ | 4.720 | 35.619 | _ | _ |
| | Coffee | Control | 51 | 34.003 | 55.076 | 6.730 | 400.00 |
| | | ADHD | 92 | 33.999 | 41.052 | 3.850 | 219.51 |
| | | Diff (1-2) | _ | 0.004 | 46.512 | _ | _ |
| | Cola_Nut | Control | 51 | 11.928 | 5.390 | 2.630 | 27.260 |
| | | ADHD | 92 | 13.835 | 7.148 | 1.830 | 33.990 |
| | | Diff (1-2) | _ | -1.907 | 6.579 | _ | _ |
| | Corn | Control | 51 | 7.351 | 5.171 | 2.080 | 27.010 |
| | | ADHD | 92 | 19.225 | 32.677 | 0.530 | 188.39 |
| | | Diff (1-2) | _ | -11.874 | 26.431 | _ | _ |
| | Cottage_Ch_ | Control | 51 | 83.139 | 107.442 | 2.120 | 400.00 |
| | | ADHD | 92 | 105.535 | 116.561 | 2.210 | 400.00 |
| | | Diff (1-2) | _ | -22.396 | 113.411 | _ | _ |
| | Cow_Milk | Control | 51 | 65.188 | 90.937 | 1.800 | 400.00 |
| | | ADHD | 92 | 83.361 | 88.266 | 1.100 | 400.00 |
| | | Diff (1-2) | _ | -18.173 | 89.222 | _ | _ |
| | Crab | Control | 51 | 30.367 | 21.673 | 3.770 | 114.37 |
| | | ADHD | 92 | 25.751 | 27.638 | 3.460 | 213.52 |
| | | Diff (1-2) | _ | 4.616 | 25.682 | _ | _ |
| | Cucumber | Control | 51 | 6.560 | 5.111 | 1.270 | 26.500 |
| | | ADHD | 92 | 21.432 | 56.304 | 1.600 | 353.35 |
| | | Diff (1-2) | _ | -14.873 | 45.334 | _ | _ |
| | Egg | Control | 51 | 70.569 | 97.119 | 5.110 | 400.00 |
| | | ADHD | 92 | 109.862 | 120.655 | 3.070 | 400.00 |
| | | Diff (1-2) | _ | -39.293 | 112.872 | _ | _ |
| | Eggplant | Control | 51 | 3.980 | 4.120 | 0.100 | 26.500 |
| | | ADHD | 92 | 9.399 | 23.449 | 0.210 | 155.52 |
| | | Diff (1-2) | _ | -5.419 | 18.997 | _ | _ |
| | Garlic | Control | 51 | 8.615 | 4.650 | 0.100 | 23.410 |
| | | ADHD | 92 | 13.875 | 12.722 | 2.830 | 65.170 |
| | | Diff (1-2) | _ | -5.259 | 10.589 | _ | _ |
| | Goat_Milk | Control | 51 | 12.632 | 28.034 | 0.100 | 149.14 |
| | | ADHD | 92 | 12.332 | 15.717 | 0.100 | 88.430 |
| | | Diff (1-2) | _ | 0.300 | 20.931 | _ | _ |
| | Grape | Control | 51 | 13.069 | 6.123 | 2.350 | 44.190 |
| | | ADHD | 92 | 14.913 | 9.885 | 5.840 | 63.770 |
| | | Diff (1-2) | _ | -1.844 | 8.738 | _ | _ |
| | Grapefruit | Control | 51 | 3.128 | 1.808 | 0.100 | 8.040 |
| | | ADHD | 92 | 5.639 | 8.356 | 0.630 | 54.840 |
| | | Diff (1-2) | _ | -2.511 | 6.799 | _ | _ |
| | Green_Pea | Control | 51 | 5.735 | 5.596 | 0.100 | 27.010 |
| | | ADHD | 92 | 9.805 | 13.113 | 0.100 | 65.560 |
| | | Diff (1-2) | _ | -4.070 | 11.049 | _ | _ |
| | Green_Pepper | Control | 51 | 4.001 | 2.220 | 1.150 | 11.460 |
| | | ADHD | 92 | 6.259 | 8.363 | 0.320 | 54.580 |
| | | Diff (1-2) | _ | -2.258 | 6.848 | _ | _ |
| | Halibut | Control | 51 | 11.307 | 6.425 | 0.850 | 35.370 |
| | | ADHD | 92 | 12.371 | 15.568 | 2.650 | 98.220 |
| | | Diff (1-2) | _ | -1.064 | 13.079 | _ | _ |
| | Honey | Control | 51 | 8.233 | 3.766 | 0.530 | 22.800 |
| | | ADHD | 92 | 10.606 | 7.379 | 2.570 | 48.440 |
| | | Diff (1-2) | _ | -2.373 | 6.338 | _ | _ |
| | Lemon | Control | 51 | 2.559 | 1.273 | 0.100 | 6.270 |
| | | ADHD | 92 | 3.103 | 2.731 | 0.110 | 17.390 |
| | | Diff (1-2) | _ | -0.543 | 2.321 | _ | _ |
| | Lettuce | Control | 51 | 9.676 | 5.181 | 3.730 | 27.090 |
| | | ADHD | 92 | 17.674 | 23.129 | 2.720 | 121.69 |
| | | Diff (1-2) | _ | -7.998 | 18.836 | _ | _ |
| | Lima_Bean | Control | 51 | 4.955 | 4.159 | 0.100 | 25.010 |
| | | ADHD | 92 | 6.487 | 5.863 | 0.880 | 32.820 |
| | | Diff (1-2) | _ | -1.531 | 5.321 | _ | _ |
| | Lobster | Control | 51 | 7.009 | 4.347 | 1.180 | 23.980 |
| | | ADHD | 92 | 9.874 | 16.089 | 1.160 | 118.16 |
| | | Diff (1-2) | _ | -2.865 | 13.182 | _ | _ |
| | Malt | Control | 51 | 13.807 | 7.087 | 1.920 | 31.200 |
| | | ADHD | 92 | 17.057 | 12.192 | 2.810 | 98.820 |
| | | Diff (1-2) | _ | -3.250 | 10.665 | _ | _ |
| | Millet | Control | 51 | 3.882 | 6.158 | 0.100 | 45.890 |
| | | ADHD | 92 | 3.253 | 1.777 | 0.210 | 8.500 |
| | | Diff (1-2) | _ | 0.629 | 3.935 | _ | _ |
| | Mushroom | Control | 51 | 22.699 | 22.937 | 1.490 | 106.22 |
| | | ADHD | 92 | 29.321 | 27.815 | 2.700 | 131.64 |
| | | Diff (1-2) | _ | -6.622 | 26.189 | _ | _ |
| | Mustard | Control | 51 | 5.399 | 2.689 | 0.910 | 11.660 |
| | | ADHD | 92 | 7.816 | 6.990 | 0.120 | 46.630 |
| | | Diff (1-2) | _ | -2.417 | 5.839 | _ | _ |
| | Oat | Control | 51 | 11.120 | 9.562 | 0.100 | 49.540 |
| | | ADHD | 92 | 24.648 | 35.440 | 1.210 | 228.72 |
| | | Diff (1-2) | _ | -13.529 | 29.035 | _ | _ |
| | Olive | Control | 51 | 15.480 | 19.299 | 3.050 | 111.23 |
| | | ADHD | 92 | 30.122 | 61.991 | 3.280 | 400.00 |
| | | Diff (1-2) | _ | -14.642 | 51.110 | _ | _ |
| | Onion | Control | 51 | 9.739 | 10.258 | 1.170 | 70.460 |
| | | ADHD | 92 | 27.033 | 65.323 | 1.800 | 400.00 |
| | | Diff (1-2) | _ | -17.293 | 52.833 | _ | _ |
| | Orange | Control | 51 | 23.728 | 28.880 | 4.170 | 149.43 |
| | | ADHD | 92 | 47.326 | 75.864 | 3.210 | 400.00 |
| | | Diff (1-2) | _ | -23.598 | 63.326 | _ | _ |
| | Oyster | Control | 51 | 44.126 | 34.721 | 9.620 | 168.93 |
| | | ADHD | 92 | 51.409 | 66.452 | 5.780 | 400.00 |
| | | Diff (1-2) | _ | -7.283 | 57.249 | _ | _ |
| | Parsley | Control | 51 | 21.958 | 46.256 | 5.990 | 342.33 |
| | | ADHD | 92 | 19.005 | 15.536 | 5.350 | 76.780 |
| | | Diff (1-2) | _ | 2.953 | 30.241 | _ | _ |
| | Peach | Control | 51 | 6.506 | 7.491 | 0.100 | 34.650 |
| | | ADHD | 92 | 23.107 | 66.936 | 0.210 | 400.00 |
| | | Diff (1-2) | _ | -16.601 | 53.958 | _ | _ |
| | Peanut | Control | 51 | 5.445 | 4.274 | 0.100 | 24.230 |
| | | ADHD | 92 | 6.205 | 10.593 | 0.210 | 85.730 |
| | | Diff (1-2) | _ | -0.759 | 8.882 | _ | _ |
| | Pineapple | Control | 51 | 8.460 | 18.977 | 0.100 | 122.86 |
| | | ADHD | 92 | 17.163 | 45.432 | 0.640 | 400.00 |
| | | Diff (1-2) | _ | -8.703 | 38.208 | _ | _ |
| | Pinto_Bean | Control | 51 | 9.830 | 9.653 | 0.210 | 47.920 |
| | | ADHD | 92 | 17.675 | 26.357 | 1.480 | 139.23 |
| | | Diff (1-2) | _ | -7.845 | 21.940 | _ | _ |
| | Pork | Control | 51 | 15.096 | 8.745 | 4.800 | 44.260 |
| | | ADHD | 92 | 14.611 | 15.147 | 2.000 | 111.43 |
| | | Diff (1-2) | _ | 0.485 | 13.236 | _ | _ |
| | Potato | Control | 51 | 8.664 | 2.240 | 4.900 | 14.010 |
| | | ADHD | 92 | 13.359 | 17.362 | 3.120 | 118.58 |
| | | Diff (1-2) | _ | -4.696 | 14.012 | _ | _ |
| | Rice | Control | 51 | 18.985 | 14.969 | 4.900 | 73.100 |
| | | ADHD | 92 | 26.191 | 27.478 | 3.730 | 189.04 |
| | | Diff (1-2) | _ | -7.206 | 23.807 | _ | _ |
| | Rye | Control | 51 | 4.185 | 2.647 | 0.230 | 17.990 |
| | | ADHD | 92 | 6.593 | 7.838 | 0.100 | 54.320 |
| | | Diff (1-2) | _ | -2.409 | 6.491 | _ | _ |
| | Safflower | Control | 51 | 6.557 | 5.364 | 1.620 | 36.650 |
| | | ADHD | 92 | 10.126 | 19.454 | 1.180 | 178.13 |
| | | Diff (1-2) | _ | -3.569 | 15.951 | _ | _ |
| | Salmon | Control | 51 | 13.155 | 11.632 | 3.480 | 68.370 |
| | | ADHD | 92 | 15.231 | 41.326 | 2.200 | 400.00 |
| | | Diff (1-2) | _ | -2.076 | 33.914 | _ | _ |
| | Sardine | Control | 51 | 29.733 | 14.098 | 12.950 | 76.730 |
| | | ADHD | 92 | 30.082 | 19.193 | 6.570 | 112.43 |
| | | Diff (1-2) | _ | -0.349 | 17.556 | _ | _ |
| | Scallop | Control | 51 | 53.504 | 22.302 | 15.620 | 107.71 |
| | | ADHD | 92 | 54.016 | 42.424 | 10.920 | 283.14 |
| | | Diff (1-2) | _ | -0.511 | 36.578 | _ | _ |
| | Sesame | Control | 51 | 91.740 | 91.167 | 6.640 | 400.00 |
| | | ADHD | 92 | 91.778 | 112.395 | 4.760 | 400.00 |
| | | Diff (1-2) | _ | -0.039 | 105.358 | _ | _ |
| | Shrimp | Control | 51 | 31.906 | 31.340 | 5.360 | 151.14 |
| | | ADHD | 92 | 17.770 | 18.162 | 2.540 | 103.29 |
| | | Diff (1-2) | _ | 14.136 | 23.689 | _ | _ |
| | Sole | Control | 51 | 5.010 | 3.858 | 0.230 | 29.090 |
| | | ADHD | 92 | 5.034 | 3.886 | 0.210 | 32.900 |
| | | Diff (1-2) | _ | -0.024 | 3.876 | _ | _ |
| | Soybean | Control | 51 | 14.277 | 10.254 | 4.150 | 51.570 |
| | | ADHD | 92 | 21.073 | 24.100 | 2.750 | 115.37 |
| | | Diff (1-2) | _ | -6.796 | 20.301 | _ | _ |
| | Spinach | Control | 51 | 20.913 | 15.581 | 3.290 | 66.870 |
| | | ADHD | 92 | 19.725 | 19.295 | 5.350 | 124.21 |
| | | Diff (1-2) | _ | 1.188 | 18.065 | _ | _ |
| | Squashes | Control | 51 | 5.696 | 2.997 | 2.050 | 13.840 |
| | | ADHD | 92 | 13.463 | 27.966 | 0.620 | 210.70 |
| | | Diff (1-2) | _ | -7.767 | 22.538 | _ | _ |
| | Strawberry | Control | 51 | 4.585 | 4.755 | 0.110 | 27.900 |
| | | ADHD | 92 | 15.284 | 50.795 | 0.100 | 331.49 |
| | | Diff (1-2) | _ | -10.699 | 40.905 | _ | _ |
| | String_Bean | Control | 51 | 34.495 | 21.114 | 12.540 | 94.210 |
| | | ADHD | 92 | 36.872 | 27.247 | 7.860 | 184.36 |
| | | Diff (1-2) | _ | -2.377 | 25.243 | _ | _ |
| | Sunflower_Sd | Control | 51 | 7.402 | 4.309 | 1.490 | 21.170 |
| | | ADHD | 92 | 8.995 | 6.411 | 1.790 | 33.590 |
| | | Diff (1-2) | _ | -1.593 | 5.754 | _ | _ |
| | Sweet_Pot_ | Control | 51 | 13.319 | 8.693 | 4.460 | 53.650 |
| | | ADHD | 92 | 15.735 | 14.552 | 2.520 | 94.140 |
| | | Diff (1-2) | _ | -2.416 | 12.785 | _ | _ |
| | Swiss_Ch_ | Control | 51 | 37.893 | 78.801 | 1.490 | 400.00 |
| | | ADHD | 92 | 38.949 | 59.178 | 1.470 | 386.11 |
| | | Diff (1-2) | _ | -1.056 | 66.799 | _ | _ |
| | Tea | Control | 51 | 19.459 | 7.609 | 8.930 | 38.010 |
| | | ADHD | 92 | 26.526 | 16.518 | 9.070 | 118.42 |
| | | Diff (1-2) | _ | -7.066 | 14.022 | _ | _ |
| | Tobacco | Control | 51 | 28.550 | 13.486 | 7.880 | 65.660 |
| | | ADHD | 92 | 38.636 | 32.339 | 9.270 | 196.29 |
| | | Diff (1-2) | _ | -10.085 | 27.193 | _ | _ |
| | Tomato | Control | 51 | 7.412 | 5.926 | 1.920 | 30.760 |
| | | ADHD | 92 | 26.083 | 69.738 | 0.990 | 400.00 |
| | | Diff (1-2) | _ | -18.670 | 56.136 | _ | _ |
| | Trout | Control | 51 | 15.255 | 16.016 | 3.000 | 93.130 |
| | | ADHD | 92 | 15.496 | 41.328 | 2.570 | 400.00 |
| | | Diff (1-2) | _ | -0.241 | 34.544 | _ | _ |
| | Tuna | Control | 51 | 8.130 | 6.362 | 3.050 | 33.880 |
| | | ADHD | 92 | 5.577 | 5.696 | 0.850 | 46.670 |
| | | Diff (1-2) | _ | 2.553 | 5.941 | _ | _ |
| | Turkey | Control | 51 | 11.859 | 5.301 | 4.490 | 28.920 |
| | | ADHD | 92 | 13.482 | 12.293 | 2.670 | 87.480 |
| | | Diff (1-2) | _ | -1.623 | 10.368 | _ | _ |
| | Walnut_Blk | Control | 51 | 19.796 | 13.830 | 5.670 | 79.530 |
| | | ADHD | 92 | 24.730 | 20.952 | 4.530 | 99.510 |
| | | Diff (1-2) | _ | -4.934 | 18.739 | _ | _ |
| | Wheat | Control | 51 | 14.031 | 16.565 | 3.200 | 116.33 |
| | | ADHD | 92 | 26.488 | 47.247 | 2.110 | 400.00 |
| | | Diff (1-2) | _ | -12.457 | 39.217 | _ | _ |
| | Yeast_Baker | Control | 51 | 6.905 | 4.322 | 2.230 | 24.960 |
| | | ADHD | 92 | 14.363 | 32.026 | 1.360 | 230.36 |
| | | Diff (1-2) | _ | -7.458 | 25.857 | _ | _ |
| | Yeast_Brewer | Control | 51 | 9.946 | 8.060 | 1.490 | 37.540 |
| | | ADHD | 92 | 17.777 | 29.355 | 1.790 | 177.52 |
| | | Diff (1-2) | _ | -7.830 | 24.066 | _ | _ |
| | Yogurt | Control | 51 | 19.256 | 34.792 | 0.100 | 223.20 |
| | | ADHD | 92 | 16.536 | 16.856 | 1.380 | 101.94 |
| | | Diff (1-2) | _ | 2.720 | 24.751 | _ | _ |
| MALE | Almond | Control | 81 | 4.955 | 2.457 | 1.600 | 14.840 |
| | | ADHD | 45 | 7.873 | 9.885 | 0.750 | 50.700 |
| | | Diff (1-2) | _ | -2.917 | 6.210 | _ | _ |
| | Amer_Cheese | Control | 81 | 33.623 | 47.729 | 1.710 | 234.20 |
| | | ADHD | 45 | 41.001 | 45.930 | 1.440 | 238.18 |
| | | Diff (1-2) | _ | -7.378 | 47.099 | _ | _ |
| | Apple | Control | 81 | 4.768 | 4.226 | 0.990 | 30.110 |
| | | ADHD | 45 | 14.584 | 59.891 | 0.100 | 400.00 |
| | | Diff (1-2) | _ | -9.816 | 35.837 | _ | _ |
| | Avocado | Control | 81 | 2.950 | 2.086 | 0.200 | 15.510 |
| | | ADHD | 45 | 2.762 | 2.450 | 0.360 | 14.930 |
| | | Diff (1-2) | _ | 0.188 | 2.222 | _ | _ |
| | Banana | Control | 81 | 4.016 | 5.531 | 0.800 | 48.430 |
| | | ADHD | 45 | 5.730 | 10.042 | 0.620 | 57.670 |
| | | Diff (1-2) | _ | -1.714 | 7.451 | _ | _ |
| | Barley | Control | 81 | 9.009 | 35.683 | 1.080 | 324.19 |
| | | ADHD | 45 | 9.982 | 21.334 | 0.240 | 142.39 |
| | | Diff (1-2) | _ | -0.974 | 31.352 | _ | _ |
| | Beef | Control | 81 | 10.820 | 19.739 | 2.370 | 162.33 |
| | | ADHD | 45 | 8.968 | 10.461 | 1.180 | 64.970 |
| | | Diff (1-2) | _ | 1.853 | 17.035 | _ | _ |
| | Blueberry | Control | 81 | 3.790 | 2.258 | 0.880 | 12.560 |
| | | ADHD | 45 | 3.964 | 4.805 | 0.100 | 33.490 |
| | | Diff (1-2) | _ | -0.174 | 3.388 | _ | _ |
| | Broccoli | Control | 81 | 7.175 | 5.133 | 2.100 | 30.730 |
| | | ADHD | 45 | 10.317 | 13.071 | 2.140 | 84.710 |
| | | Diff (1-2) | _ | -3.142 | 8.810 | _ | _ |
| | Buck_Wheat | Control | 81 | 5.548 | 3.014 | 1.670 | 23.700 |
| | | ADHD | 45 | 6.486 | 4.736 | 0.610 | 25.060 |
| | | Diff (1-2) | _ | -0.938 | 3.718 | _ | _ |
| | Butter | Control | 81 | 16.652 | 19.178 | 1.550 | 93.140 |
| | | ADHD | 45 | 33.273 | 31.469 | 0.120 | 117.94 |
| | | Diff (1-2) | _ | -16.621 | 24.263 | _ | _ |
| | Cabbage | Control | 81 | 5.952 | 10.811 | 0.980 | 94.740 |
| | | ADHD | 45 | 13.046 | 34.923 | 1.780 | 230.48 |
| | | Diff (1-2) | _ | -7.094 | 22.543 | | |
| | Cane_Sugar | Control | 81 | 23.047 | 28.025 | 3.900 | 170.78 |
| | | ADHD | 45 | 30.327 | 54.839 | 3.250 | 360.99 |
| | | Diff (1-2) | _ | -7.280 | 39.672 | _ | |
| | Cantaloupe | Control | 81 | 5.879 | 4.368 | 1.960 | 29.570 |
| | | ADHD | 45 | 20.911 | 61.974 | 0.360 | 400.00 |
| | | Diff (1-2) | _ | -15.032 | 37.083 | | |
| | Carrot | Control | 81 | 4.016 | 3.787 | 1.180 | 27.680 |
| | | ADHD | 45 | 14.156 | 41.503 | 0.480 | 224.76 |
| | | Diff (1-2) | _ | -10.140 | 24.909 | | |
| | Cashew | Control | 81 | 9.724 | 11.603 | 1.020 | 59.200 |
| | | ADHD | 45 | 9.675 | 13.714 | 0.100 | 78.260 |
| | | Diff (1-2) | _ | 0.050 | 12.393 | | |
| | Cauliflower | Control | 81 | 4.865 | 3.698 | 1.510 | 24.160 |
| | | ADHD | 45 | 11.647 | 30.698 | 1.800 | 205.18 |
| | | Diff (1-2) | _ | -6.781 | 18.526 | | |
| | Celery | Control | 81 | 8.967 | 5.476 | 2.950 | 34.790 |
| | | ADHD | 45 | 15.251 | 26.609 | 2.990 | 155.83 |
| | | Diff (1-2) | _ | -6.283 | 16.450 | | |
| | Cheddar_Ch_ | Control | 81 | 26.696 | 45.931 | 1.690 | 283.73 |
| | | ADHD | 45 | 43.425 | 70.376 | 0.600 | 355.84 |
| | | Diff (1-2) | _ | -16.729 | 55.843 | | |
| | Chicken | Control | 81 | 16.053 | 12.550 | 2.940 | 76.880 |
| | | ADHD | 45 | 14.770 | 10.368 | 4.290 | 45.240 |
| | | Diff (1-2) | _ | 1.283 | 11.822 | | |
| | Chili_Pepper | Control | 81 | 7.835 | 5.613 | 1.570 | 38.040 |
| | | ADHD | 45 | 10.254 | 11.413 | 1.800 | 54.050 |
| | | Diff (1-2) | _ | -2.419 | 8.157 | | |
| | Chocolate | Control | 81 | 16.623 | 11.019 | 3.010 | 59.470 |
| | | ADHD | 45 | 16.970 | 12.146 | 4.580 | 49.160 |
| | | Diff (1-2) | _ | -0.347 | 11.432 | | |
| | Cinnamon | Control | 81 | 9.850 | 7.037 | 1.640 | 40.480 |
| | | ADHD | 45 | 9.821 | 6.585 | 1.810 | 32.670 |
| | | Diff (1-2) | _ | 0.029 | 6.880 | _ | _ |
| | Clam | Control | 81 | 33.566 | 20.277 | 3.190 | 98.480 |
| | | ADHD | 45 | 26.397 | 25.701 | 2.780 | 132.61 |
| | | Diff (1-2) | _ | 7.169 | 22.353 | _ | _ |
| | Codfish | Control | 81 | 25.075 | 33.649 | 6.490 | 277.17 |
| | | ADHD | 45 | 29.288 | 58.990 | 3.900 | 400.00 |
| | | Diff (1-2) | _ | -4.212 | 44.331 | _ | _ |
| | Coffee | Control | 81 | 30.318 | 43.408 | 4.320 | 356.95 |
| | | ADHD | 45 | 33.387 | 40.314 | 3.970 | 237.41 |
| | | Diff (1-2) | _ | -3.069 | 42.336 | _ | _ |
| | Cola_Nut | Control | 81 | 15.243 | 8.049 | 4.080 | 38.820 |
| | | ADHD | 45 | 14.139 | 8.193 | 4.790 | 36.670 |
| | | Diff (1-2) | _ | 1.105 | 8.101 | _ | _ |
| | Corn | Control | 81 | 9.923 | 12.544 | 2.360 | 95.510 |
| | | ADHD | 45 | 11.735 | 10.231 | 2.160 | 53.180 |
| | | Diff (1-2) | _ | -1.812 | 11.775 | _ | _ |
| | Cottage_Ch_ | Control | 81 | 76.631 | 102.972 | 1.210 | 400.00 |
| | | ADHD | 45 | 123.448 | 125.633 | 2.290 | 400.00 |
| | | Diff (1-2) | _ | -46.817 | 111.542 | _ | _ |
| | Cow_Milk | Control | 81 | 60.822 | 83.166 | 1.770 | 400.00 |
| | | ADHD | 45 | 93.019 | 93.827 | 2.040 | 400.00 |
| | | Diff (1-2) | _ | -32.197 | 87.098 | _ | _ |
| | Crab | Control | 81 | 32.448 | 37.288 | 4.770 | 299.11 |
| | | ADHD | 45 | 25.297 | 20.928 | 5.170 | 126.85 |
| | | Diff (1-2) | _ | 7.151 | 32.442 | _ | _ |
| | Cucumber | Control | 81 | 8.752 | 8.584 | 1.880 | 61.860 |
| | | ADHD | 45 | 27.909 | 68.764 | 2.530 | 400.00 |
| | | Diff (1-2) | _ | -19.157 | 41.538 | _ | _ |
| | Egg | Control | 81 | 62.505 | 92.408 | 3.780 | 400.00 |
| | | ADHD | 45 | 120.210 | 128.233 | 2.650 | 400.00 |
| | | Diff (1-2) | _ | -57.705 | 106.508 | _ | _ |
| | Eggplant | Control | 81 | 5.045 | 5.910 | 1.370 | 48.790 |
| | | ADHD | 45 | 8.238 | 16.933 | 1.180 | 90.580 |
| | | Diff (1-2) | _ | | -3.193 11.148 | _ | _ |
| | Garlic | Control | 81 | 11.918 | 9.606 | 3.040 | 52.160 |
| | | ADHD | 45 | 15.901 | 20.797 | 0.230 | 116.36 |
| | | Diff (1-2) | _ | -3.983 | 14.595 | _ | _ |
| | Goat_Milk | Control | 81 | 11.177 | 16.325 | 0.500 | 96.690 |
| | | ADHD | 45 | 14.943 | 17.272 | 0.600 | 90.320 |
| | | Diff (1-2) | _ | -3.767 | 16.667 | _ | _ |
| | Grape | Control | 81 | 15.645 | 5.750 | 8.060 | 47.250 |
| | | ADHD | 45 | 14.363 | 11.715 | 5.650 | 65.050 |
| | | Diff (1-2) | _ | 1.282 | 8.368 | _ | _ |
| | Grapefruit | Control | 81 | 4.255 | 3.961 | 0.810 | 32.910 |
| | | ADHD | 45 | 11.103 | 44.847 | 0.120 | 304.26 |
| | | Diff (1-2) | _ | -6.848 | 26.903 | _ | _ |
| | Green_Pea | Control | 81 | 7.020 | 6.334 | 1.020 | 35.200 |
| | | ADHD | 45 | 12.287 | 18.687 | 1.320 | 87.570 |
| | | Diff (1-2) | _ | -5.267 | 12.239 | _ | _ |
| | Green_Pepper | Control | 81 | 4.715 | 3.714 | 1.660 | 32.330 |
| | | ADHD | 45 | 7.840 | 13.389 | 1.390 | 76.860 |
| | | Diff (1-2) | _ | -3.124 | 8.515 | _ | _ |
| | Halibut | Control | 81 | 14.289 | 15.877 | 4.410 | 135.74 |
| | | ADHD | 45 | 9.302 | 6.073 | 1.440 | 30.940 |
| | | Diff (1-2) | _ | 4.987 | 13.256 | _ | _ |
| | Honey | Control | 81 | 10.351 | 5.111 | 2.730 | 29.820 |
| | | ADHD | 45 | 11.212 | 10.312 | 4.380 | 64.910 |
| | | Diff (1-2) | _ | -0.861 | 7.388 | _ | _ |
| | Lemon | Control | 81 | 3.051 | 2.461 | 0.200 | 20.660 |
| | | ADHD | 45 | 3.049 | 4.172 | 0.100 | 28.260 |
| | | Diff (1-2) | _ | 0.002 | 3.175 | _ | _ |
| | Lettuce | Control | 81 | 12.815 | 7.663 | 3.730 | 39.970 |
| | | ADHD | 45 | 22.311 | 39.582 | 4.060 | 245.35 |
| | | Diff (1-2) | _ | -9.497 | 24.369 | _ | _ |
| | Lima_Bean | Control | 81 | 6.294 | 5.249 | 1.550 | 35.110 |
| | | ADHD | 45 | 8.261 | 10.797 | 1.440 | 64.200 |
| | | Diff (1-2) | _ | -1.967 | 7.690 | _ | _ |
| | Lobster | Control | 81 | 9.454 | 6.640 | 1.310 | 41.980 |
| | | ADHD | 45 | 8.113 | 5.548 | 1.920 | 27.520 |
| | | Diff (1-2) | _ | 1.341 | 6.274 | _ | _ |
| | Malt | Control | 81 | 15.173 | 8.267 | 2.550 | 51.290 |
| | | ADHD | 45 | 16.614 | 8.000 | 4.680 | 42.770 |
| | | Diff (1-2) | _ | -1.441 | 8.173 | _ | _ |
| | Millet | Control | 81 | 4.065 | 4.304 | 1.440 | 40.360 |
| | | ADHD | 45 | 3.069 | 1.661 | 1.090 | 10.770 |
| | | Diff (1-2) | _ | 0.996 | 3.596 | _ | _ |
| | Mushroom | Control | 81 | 27.234 | 27.375 | 2.820 | 118.76 |
| | | ADHD | 45 | 31.311 | 37.013 | 2.170 | 165.04 |
| | | Diff (1-2) | _ | -4.076 | 31.139 | _ | _ |
| | Mustard | Control | 81 | 6.992 | 4.300 | 1.950 | 30.770 |
| | | ADHD | 45 | 9.070 | 6.716 | 2.350 | 27.670 |
| | | Diff (1-2) | _ | -2.078 | 5.285 | _ | _ |
| | Oat | Control | 81 | 18.201 | 20.144 | 1.180 | 88.430 |
| | | ADHD | 45 | 24.258 | 44.906 | 0.240 | 289.44 |
| | | Diff (1-2) | _ | -6.057 | 31.263 | _ | _ |
| | Olive | Control | 81 | 17.589 | 31.696 | 3.550 | 281.30 |
| | | ADHD | 45 | 32.810 | 71.753 | 3.760 | 400.00 |
| | | Diff (1-2) | _ | -15.221 | 49.750 | _ | _ |
| | Onion | Control | 81 | 13.450 | 23.822 | 2.270 | 210.93 |
| | | ADHD | 45 | 21.552 | 43.799 | 2.260 | 288.97 |
| | | Diff (1-2) | _ | -8.101 | 32.355 | _ | _ |
| | Orange | Control | 81 | 26.423 | 37.325 | 2.820 | 314.77 |
| | | ADHD | 45 | 52.303 | 86.202 | 6.980 | 400.00 |
| | | Diff (1-2) | _ | -25.880 | 59.460 | _ | _ |
| | Oyster | Control | 81 | 49.593 | 42.026 | 7.660 | 250.39 |
| | | ADHD | 45 | 51.189 | 40.645 | 2.990 | 145.00 |
| | | Diff (1-2) | _ | -1.596 | 41.542 | _ | _ |
| | Parsley | Control | 81 | 17.745 | 7.651 | 5.300 | 59.620 |
| | | ADHD | 45 | 18.454 | 19.955 | 7.580 | 120.17 |
| | | Diff (1-2) | _ | -0.710 | 13.381 | _ | _ |
| | Peach | Control | 81 | 10.413 | 10.155 | 1.910 | 53.130 |
| | | ADHD | 45 | 29.666 | 69.796 | 1.710 | 390.11 |
| | | Diff (1-2) | _ | -19.253 | 42.369 | _ | _ |
| | Peanut | Control | 81 | 5.730 | 9.912 | 1.220 | 89.270 |
| | | ADHD | 45 | 7.088 | 9.960 | 0.210 | 61.860 |
| | | Diff (1-2) | _ | -1.358 | 9.929 | _ | _ |
| | Pineapple | Control | 81 | 12.433 | 44.326 | 1.660 | 400.00 |
| | | ADHD | 45 | 24.370 | 71.590 | 1.680 | 400.00 |
| | | Diff (1-2) | _ | -11.937 | 55.554 | _ | _ |
| | Pinto_Bean | Control | 81 | 9.371 | 6.088 | 2.000 | 33.950 |
| | | ADHD | 45 | 18.432 | 47.574 | 0.840 | 326.50 |
| | | Diff (1-2) | _ | -9.061 | 28.758 | _ | _ |
| | Pork | Control | 81 | 16.675 | 14.641 | 4.200 | 89.420 |
| | | ADHD | 45 | 13.623 | 14.044 | 3.990 | 95.400 |
| | | Diff (1-2) | _ | 3.052 | 14.432 | _ | _ |
| | Potato | Control | 81 | 12.243 | 8.339 | 4.920 | 75.770 |
| | | ADHD | 45 | 19.614 | 42.471 | 3.490 | 238.41 |
| | | Diff (1-2) | _ | -7.372 | 26.171 | _ | _ |
| | Rice | Control | 81 | 24.230 | 16.518 | 4.810 | 79.620 |
| | | ADHD | 45 | 26.083 | 23.639 | 4.400 | 104.65 |
| | | Diff (1-2) | _ | -1.852 | 19.347 | _ | _ |
| | Rye | Control | 81 | 5.122 | 3.376 | 1.570 | 23.490 |
| | | ADHD | 45 | 8.482 | 9.991 | 0.100 | 42.730 |
| | | Diff (1-2) | _ | -3.360 | 6.540 | _ | _ |
| | Safflower | Control | 81 | 7.553 | 4.019 | 2.450 | 27.490 |
| | | ADHD | 45 | 11.774 | 16.638 | 2.070 | 99.350 |
| | | Diff (1-2) | _ | -4.221 | 10.423 | _ | _ |
| | Salmon | Control | 81 | 16.308 | 15.973 | 0.100 | 136.52 |
| | | ADHD | 45 | 11.480 | 12.387 | 0.370 | 66.780 |
| | | Diff (1-2) | _ | 4.827 | 14.800 | _ | _ |
| | Sardine | Control | 81 | 33.100 | 14.613 | 7.840 | 87.490 |
| | | ADHD | 45 | 34.280 | 34.250 | 3.530 | 223.07 |
| | | Diff (1-2) | _ | -1.181 | 23.537 | _ | _ |
| | Scallop | Control | 81 | 50.308 | 23.098 | 11.060 | 116.33 |
| | | ADHD | 45 | 55.161 | 42.723 | 6.980 | 162.20 |
| | | Diff (1-2) | _ | -4.854 | 31.494 | _ | _ |
| | Sesame | Control | 81 | 73.448 | 87.621 | 3.430 | 400.00 |
| | | ADHD | 45 | 98.254 | 111.756 | 2.880 | 400.00 |
| | | Diff (1-2) | _ | -24.806 | 96.876 | _ | _ |
| | Shrimp | Control | 81 | 34.185 | 40.052 | 2.930 | 272.28 |
| | | ADHD | 45 | 19.372 | 14.056 | 4.160 | 57.840 |
| | | Diff (1-2) | _ | 14.812 | 33.242 | _ | _ |
| | Sole | Control | 81 | 5.290 | 2.521 | 2.240 | 20.370 |
| | | ADHD | 45 | 4.138 | 1.985 | 0.370 | 10.540 |
| | | Diff (1-2) | _ | 1.152 | 2.344 | _ | _ |
| | Soybean | Control | 81 | 16.814 | 12.312 | 3.480 | 81.380 |
| | | ADHD | 45 | 19.752 | 19.790 | 4.900 | 110.99 |
| | | Diff (1-2) | _ | -2.938 | 15.387 | _ | _ |
| | Spinach | Control | 81 | 14.620 | 6.503 | 5.380 | 40.130 |
| | | ADHD | 45 | 22.821 | 33.693 | 4.590 | 232.80 |
| | | Diff (1-2) | _ | -8.201 | 20.739 | _ | _ |
| | Squashes | Control | 81 | 7.200 | 4.790 | 2.260 | 24.680 |
| | | ADHD | 45 | 19.699 | 52.168 | 2.460 | 332.85 |
| | | Diff (1-2) | _ | -12.499 | 31.313 | _ | _ |
| | Strawberry | Control | 81 | 5.072 | 4.417 | 1.000 | 29.160 |
| | | ADHD | 45 | 15.664 | 59.370 | 0.730 | 400.00 |
| | | Diff (1-2) | _ | -10.591 | 35.543 | _ | _ |
| | String_Bean | Control | 81 | 37.257 | 22.322 | 7.890 | 146.17 |
| | | ADHD | 45 | 45.077 | 59.336 | 7.050 | 400.00 |
| | | Diff (1-2) | _ | -7.820 | 39.633 | _ | _ |
| | Sunflower_Sd | Control | 81 | 8.566 | 5.303 | 2.450 | 31.260 |
| | | ADHD | 45 | 11.785 | 17.504 | 3.250 | 119.64 |
| | | Diff (1-2) | _ | -3.220 | 11.264 | _ | _ |
| | Sweet_Pot_ | Control | 81 | 17.535 | 13.698 | 4.100 | 74.660 |
| | | ADHD | 45 | 16.360 | 17.323 | 3.100 | 91.230 |
| | | Diff (1-2) | _ | 1.175 | 15.084 | _ | _ |
| | Swiss_Ch_ | Control | 81 | 35.608 | 58.963 | 2.010 | 299.50 |
| | | ADHD | 45 | 54.448 | 81.068 | 2.030 | 357.47 |
| | | Diff (1-2) | _ | -18.840 | 67.638 | _ | _ |
| | Tea | Control | 81 | 23.966 | 9.868 | 7.620 | 46.400 |
| | | ADHD | 45 | 28.329 | 23.408 | 8.760 | 159.87 |
| | | Diff (1-2) | _ | -4.363 | 16.039 | _ | _ |
| | Tobacco | Control | 81 | 36.231 | 21.642 | 8.830 | 125.93 |
| | | ADHD | 45 | 42.184 | 34.190 | 8.580 | 211.54 |
| | | Diff (1-2) | _ | -5.953 | 26.776 | _ | _ |
| | Tomato | Control | 81 | 9.199 | 6.995 | 2.320 | 40.930 |
| | | ADHD | 45 | 30.259 | 70.799 | 2.530 | 387.96 |
| | | Diff (1-2) | _ | -21.059 | 42.546 | _ | _ |
| | Trout | Control | 81 | 14.686 | 9.991 | 3.220 | 83.960 |
| | | ADHD | 45 | 13.454 | 14.761 | 3.330 | 85.230 |
| | | Diff (1-2) | _ | 1.233 | 11.904 | _ | _ |
| | Tuna | Control | 81 | 8.305 | 6.512 | 2.110 | 39.020 |
| | | ADHD | 45 | 12.132 | 25.126 | 2.120 | 122.92 |
| | | Diff (1-2) | _ | -3.827 | 15.855 | _ | _ |
| | Turkey | Control | 81 | 14.012 | 11.117 | 4.080 | 65.180 |
| | | ADHD | 45 | 13.009 | 8.626 | 4.040 | 36.080 |
| | | Diff (1-2) | _ | 1.003 | 10.302 | _ | _ |
| | Walnut_Blk | Control | 81 | 20.821 | 10.403 | 5.680 | 58.470 |
| | | ADHD | 45 | 28.055 | 32.043 | 7.160 | 149.93 |
| | | Diff (1-2) | _ | -7.234 | 20.836 | _ | _ |
| | Wheat | Control | 81 | 13.359 | 10.034 | 3.240 | 71.930 |
| | | ADHD | 45 | 43.230 | 74.407 | 4.300 | 400.00 |
| | | Diff (1-2) | _ | -29.871 | 45.050 | _ | _ |
| | Yeast_Baker | Control | 81 | 12.471 | 20.370 | 2.070 | 123.35 |
| | | ADHD | 45 | 12.101 | 16.201 | 1.390 | 77.180 |
| | | Diff (1-2) | _ | 0.371 | 18.995 | _ | _ |
| | Yeast_Brewer | Control | 81 | 15.903 | 21.144 | 2.640 | 130.89 |
| | | ADHD | 45 | 18.229 | 26.775 | 2.030 | 121.54 |
| | | Diff (1-2) | _ | -2.326 | 23.298 | _ | _ |
| | Yogurt | Control | 81 | 15.650 | 16.294 | 3.000 | 73.200 |
| | | ADHD | 45 | 18.662 | 17.105 | 1.280 | 76.030 |
| | | Diff (1-2) | _ | -3.012 | 16.587 | _ | _ |

### Upper Quantiles of ELISA Signal Scores among Control Subjects as Candidates for Test Cutpoints in Determining "Positive" or "Negative"

Top 26 Foods Ranked by Descending order of Discriminatory Ability using Permutation Test ADD/ADHD Subjects vs. Controls

**Table 4**

| *Food Ranking* | *Food* | *Sex* | *Cutpoint* | |
|---|---|---|---|---|
| | | | *90th percentile* | *95th percentile* |
| 1 | Cantaloupe | FEMALE | 7.774 | 9.885 |
| | | MALE | 10.247 | 14.980 |
| 2 | Wheat | FEMALE | 22.676 | 40.002 |
| | | MALE | 23.891 | 30.922 |
| 3 | Tomato | FEMALE | 12.757 | 20.137 |
| | | MALE | 16.321 | 23.825 |
| 4 | Cucumber | FEMALE | 12.269 | 17.960 |
| | | MALE | 16.150 | 22.827 |
| 5 | Squashes | FEMALE | 10.267 | 11.963 |
| | | MALE | 13.574 | 18.485 |
| 6 | Almond | FEMALE | 6.392 | 7.269 |
| | | MALE | 8.212 | 10.337 |
| 7 | Egg | FEMALE | 188.62 | 321.65 |
| | | MALE | 145.49 | 298.13 |
| 8 | Cauliflower | FEMALE | 7.594 | 8.954 |
| | | MALE | 7.799 | 12.288 |
| 9 | Pinto_Bean | FEMALE | 20.059 | 32.885 |
| | | MALE | 17.813 | 21.226 |
| 10 | Broccoli | FEMALE | 9.069 | 12.037 |
| | | MALE | 13.200 | 18.126 |
| 11 | Orange | FEMALE | 59.347 | 90.581 |
| | | MALE | 47.525 | 68.415 |
| 12 | Butter | FEMALE | 40.002 | 67.588 |
| | | MALE | 43.433 | 60.984 |
| 13 | Corn | FEMALE | 13.161 | 19.357 |
| | | MALE | 17.173 | 28.120 |
| 14 | Lettuce | FEMALE | 16.940 | 21.280 |
| | | MALE | 23.239 | 30.306 |
| 15 | Rye | FEMALE | 6.198 | 8.944 |
| | | MALE | 8.399 | 11.978 |
| 16 | Peach | FEMALE | 16.434 | 25.262 |
| | | MALE | 22.310 | 32.987 |
| 17 | Green_Pea | FEMALE | 13.221 | 18.595 |
| | | MALE | 14.660 | 21.095 |
| 18 | Carrot | FEMALE | 5.055 | 6.010 |
| | | MALE | 6.600 | 9.684 |
| 19 | Tea | FEMALE | 29.837 | 33.245 |
| | | MALE | 37.540 | 42.112 |
| 20 | Mustard | FEMALE | 9.607 | 10.799 |
| | | MALE | 11.762 | 14.581 |
| 21 | Strawberry | FEMALE | 7.900 | 14.127 |
| | | MALE | 9.448 | 14.125 |
| 22 | Celery | FEMALE | 14.210 | 19.974 |
| | | MALE | 15.168 | 19.728 |
| 23 | Green_Pepper | FEMALE | 6.912 | 8.275 |
| | | MALE | 7.092 | 9.348 |
| 24 | Garlic | FEMALE | 15.016 | 17.352 |
| | | MALE | 24.205 | 34.240 |
| 25 | Oat | FEMALE | 22.743 | 27.596 |
| | | MALE | 51.597 | 63.749 |
| 26 | Onion | FEMALE | 18.635 | 26.190 |
| | | MALE | 26.971 | 32.220 |
| 27 | Banana | FEMALE | 5.276 | 8.990 |
| | | MALE | 6.632 | 10.083 |
| 28 | Eggplant | FEMALE | 7.193 | 11.328 |
| | | MALE | 7.631 | 12.926 |
| 29 | Cabbage | FEMALE | 8.484 | 15.011 |
| | | MALE | 9.596 | 16.710 |
| 30 | Safflower | FEMALE | 10.328 | 16.067 |
| | | MALE | 11.820 | 14.861 |
| 31 | Olive | FEMALE | 27.849 | 55.153 |
| | | MALE | 28.220 | 44.995 |
| 32 | Cottage_Ch_ | FEMALE | 244.09 | 354.86 |
| | | MALE | 225.78 | 341.05 |
| 33 | Grapefruit | FEMALE | 5.731 | 6.772 |
| | | MALE | 7.127 | 10.165 |
| 34 | Walnut_Blk | FEMALE | 38.596 | 49.729 |
| | | MALE | 35.760 | 41.973 |
| 35 | Cow_Milk | FEMALE | 155.08 | 281.94 |
| | | MALE | 149.48 | 244.73 |
| 36 | Soybean | FEMALE | 27.522 | 38.061 |
| | | MALE | 31.249 | 41.905 |
| 37 | Chili_Pepper | FEMALE | 15.566 | 21.521 |
| | | MALE | 14.808 | 18.731 |

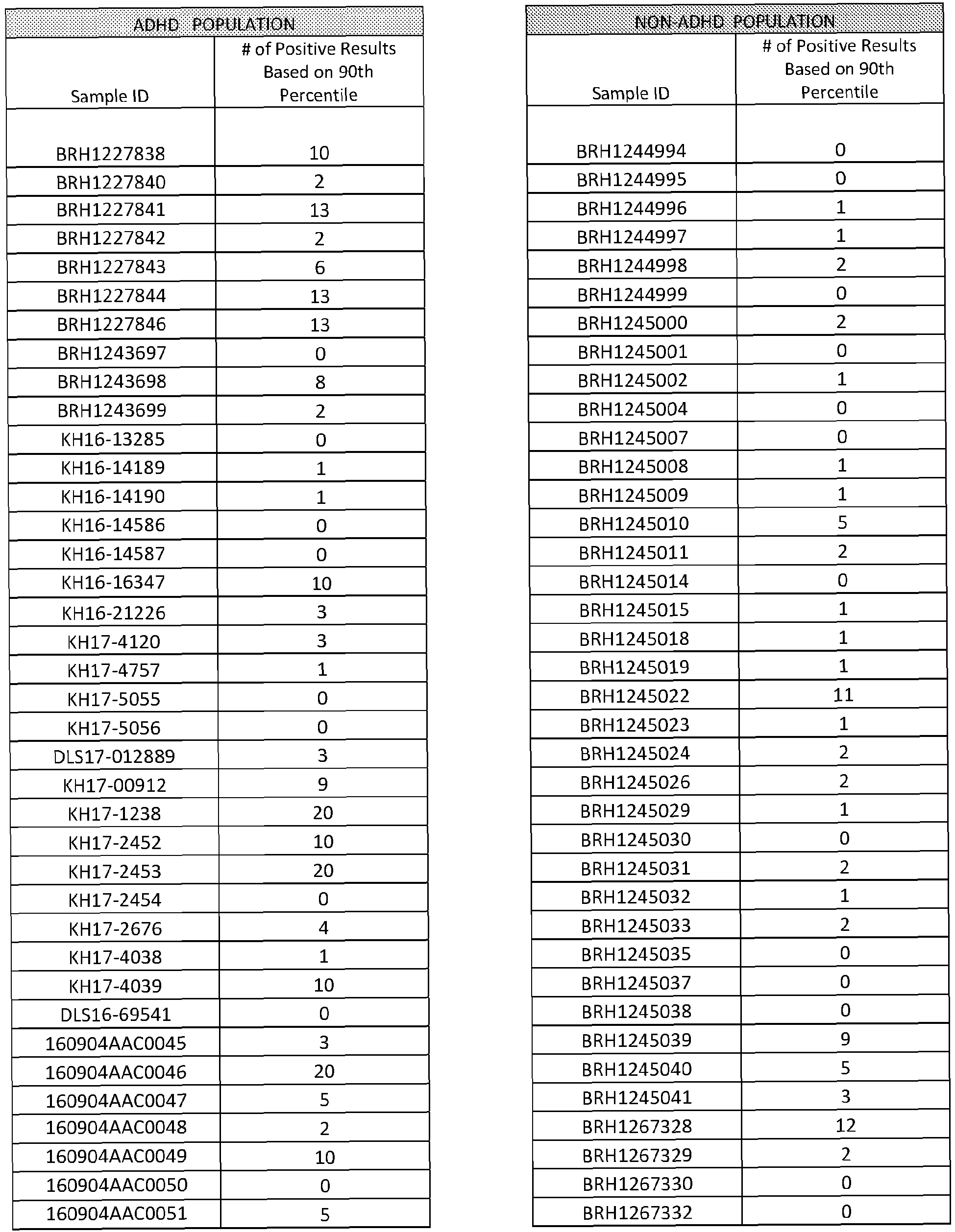

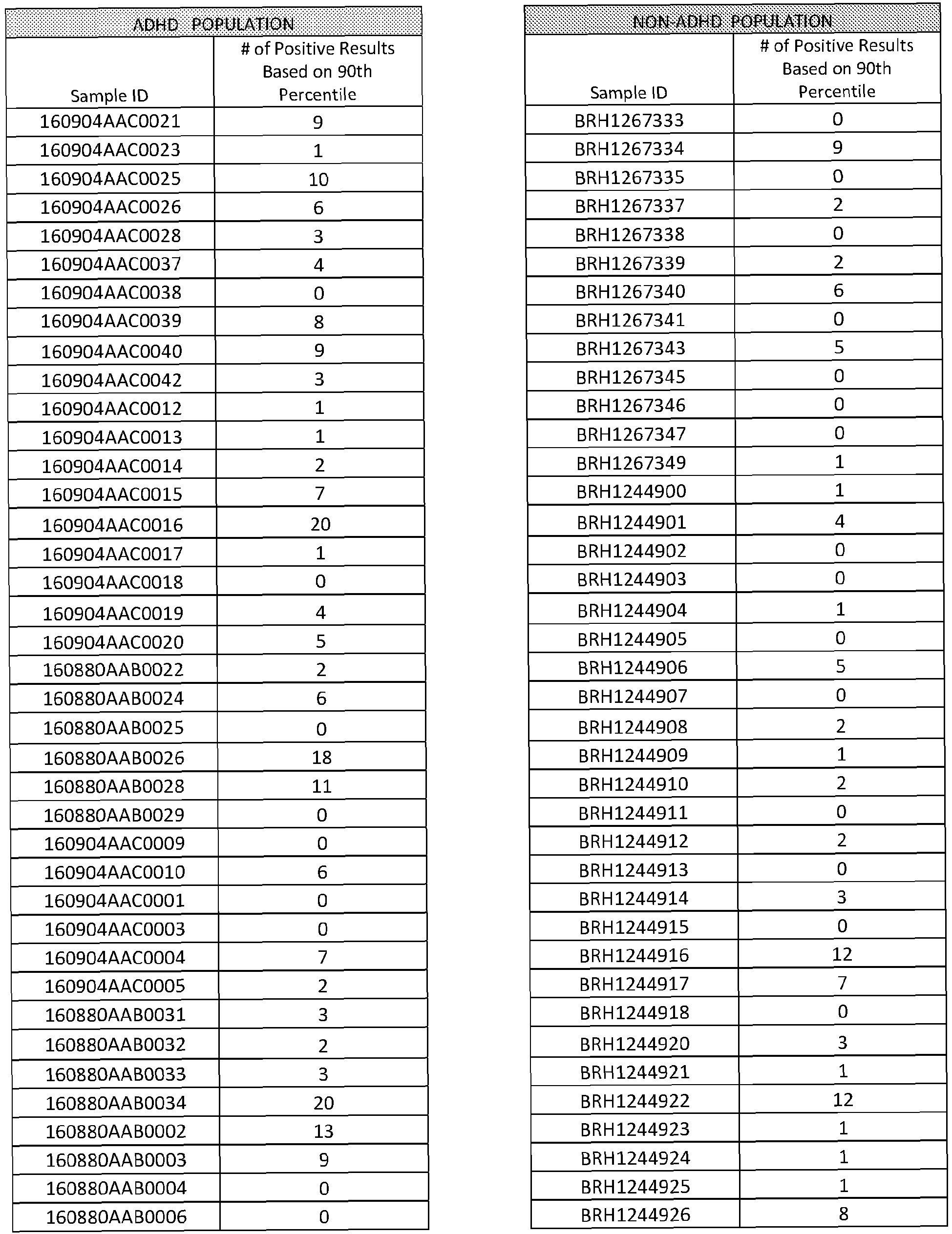

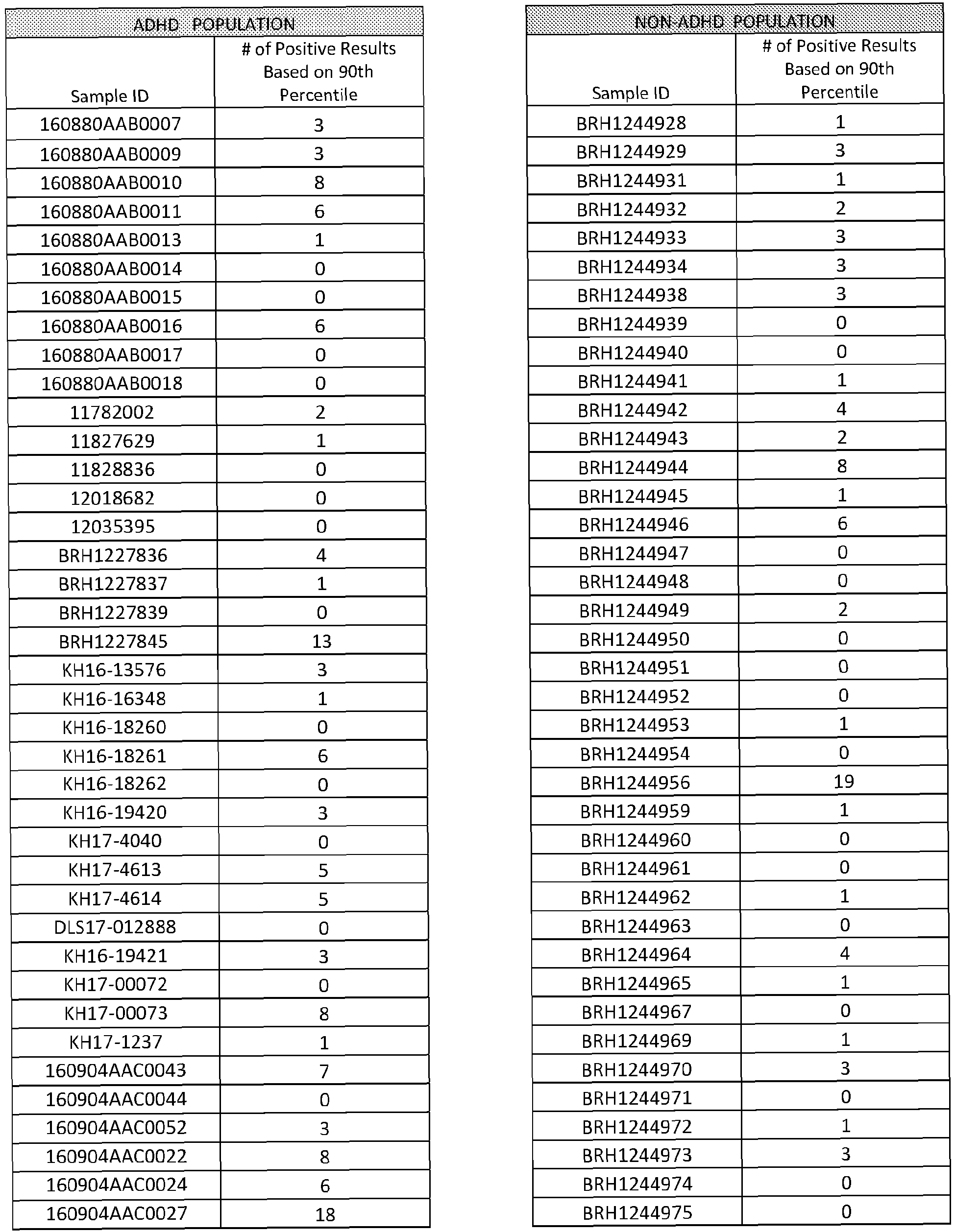

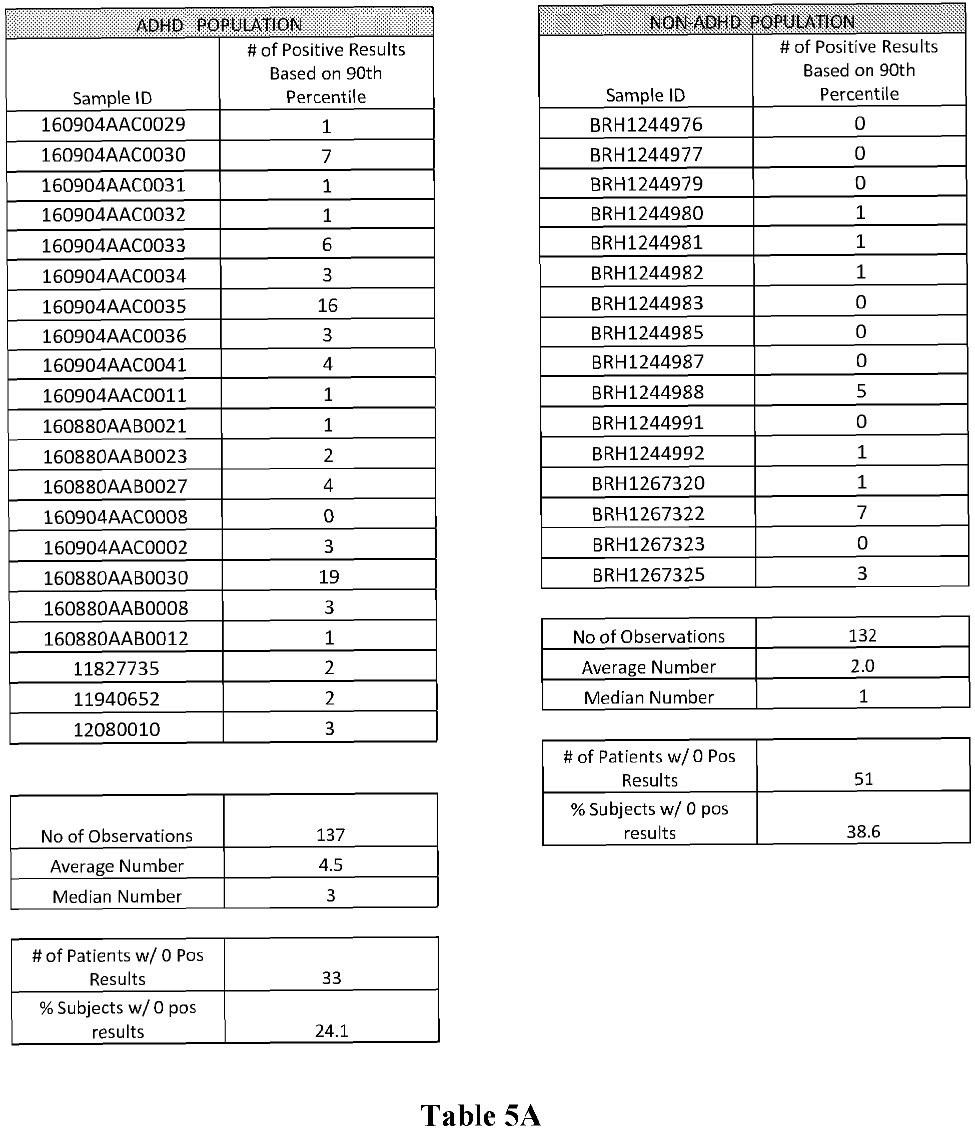

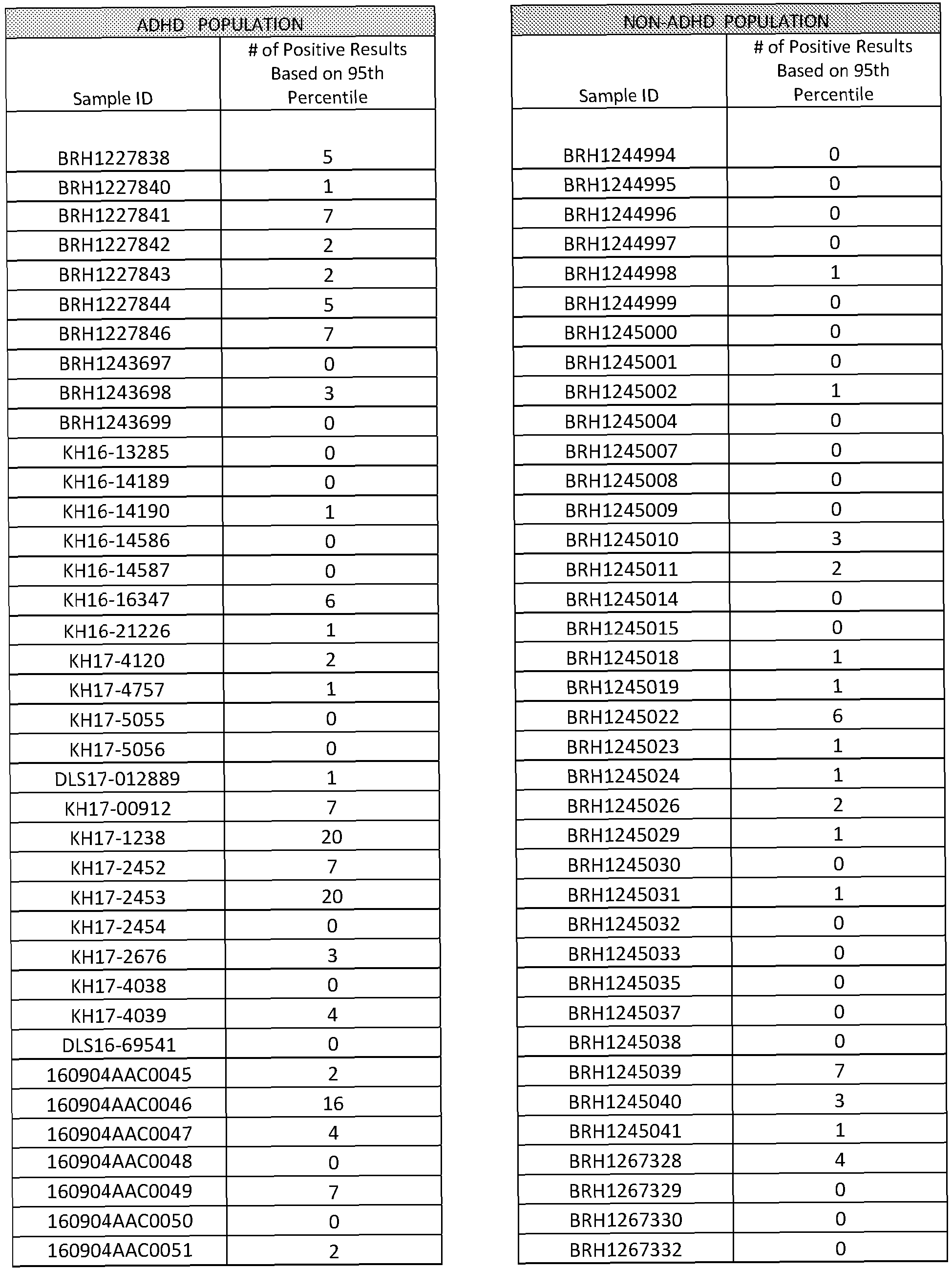

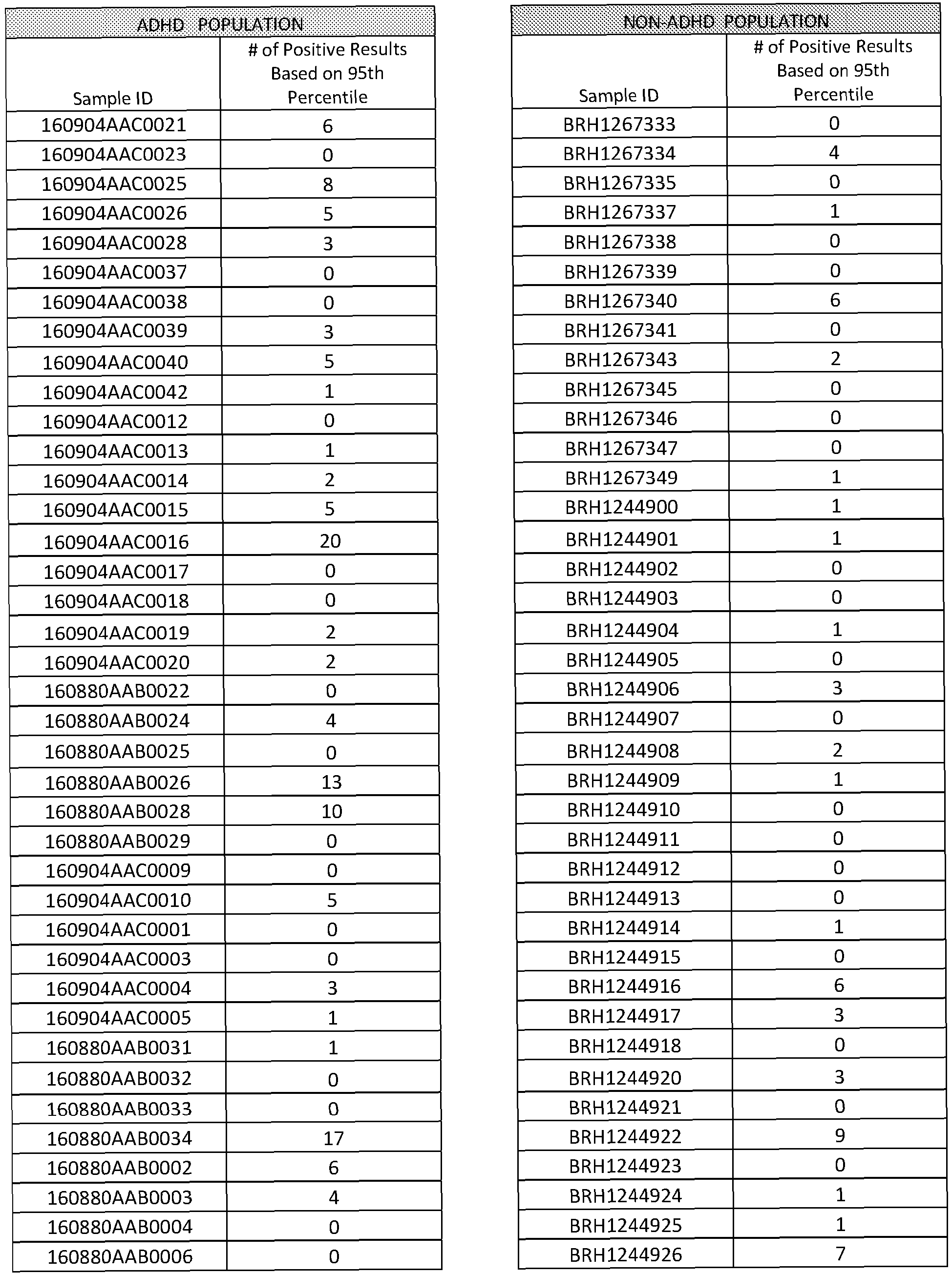

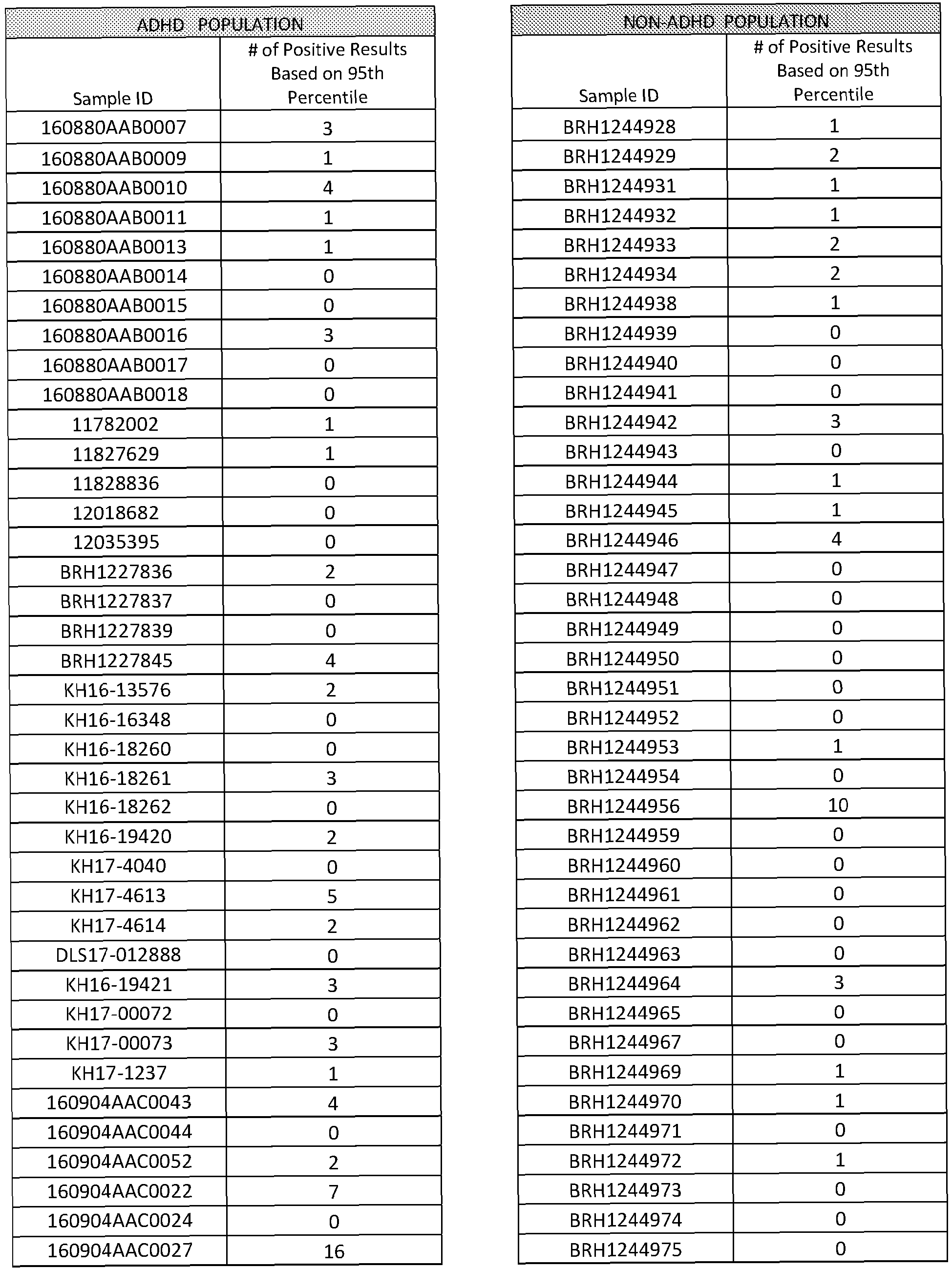

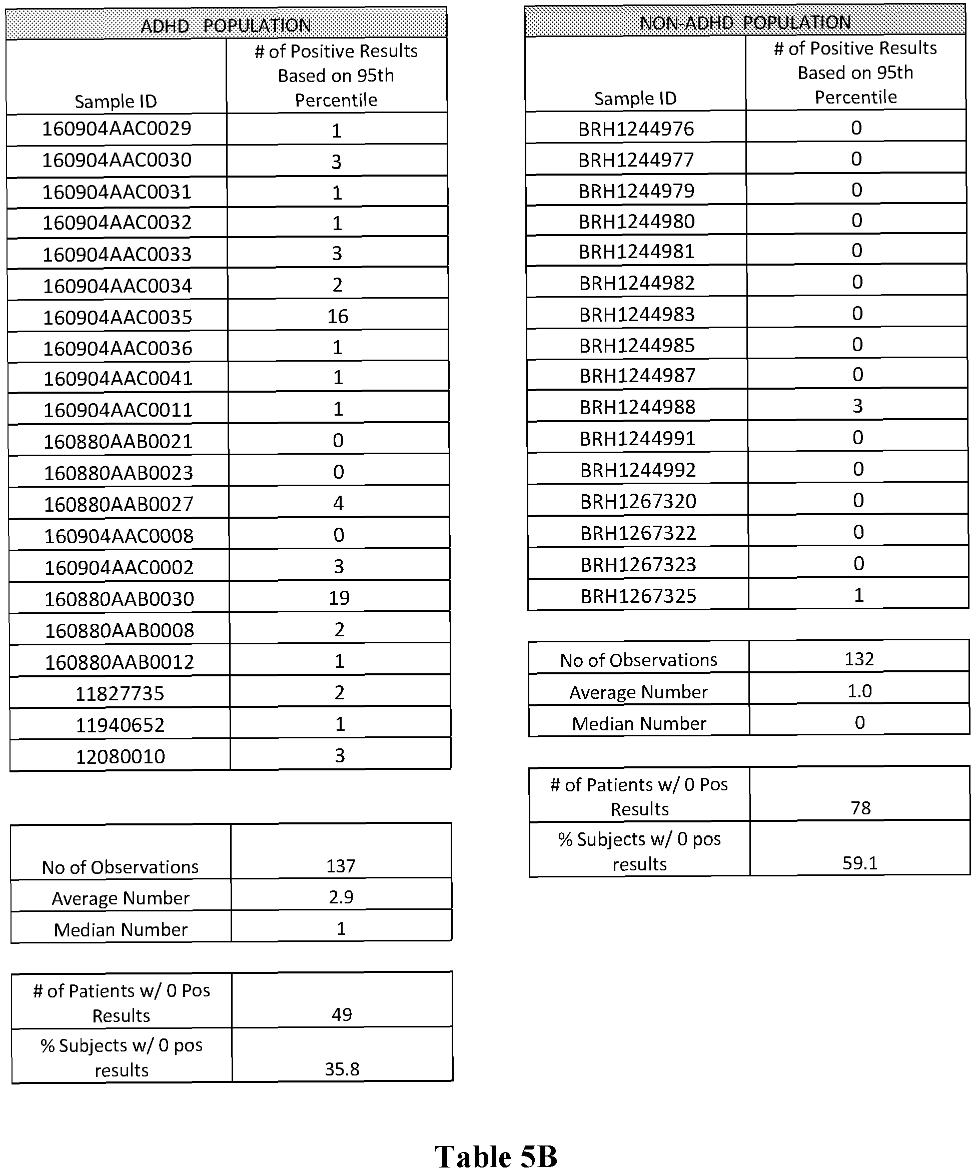

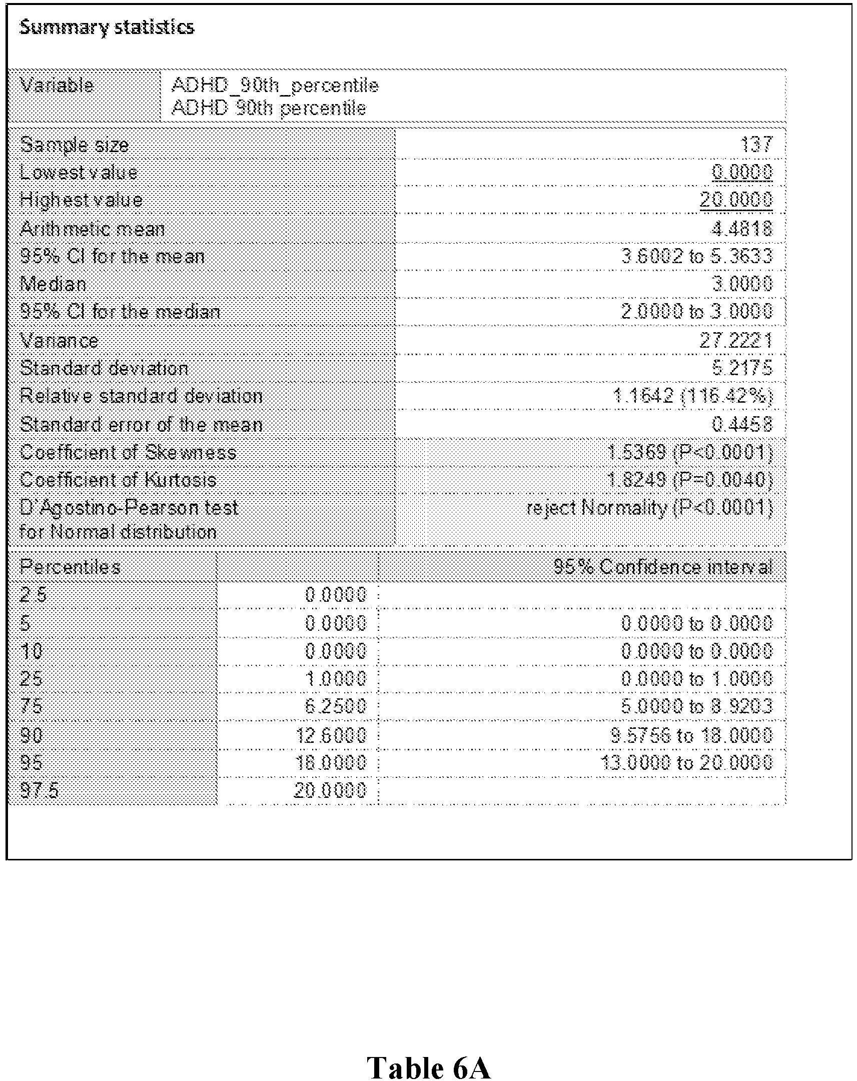

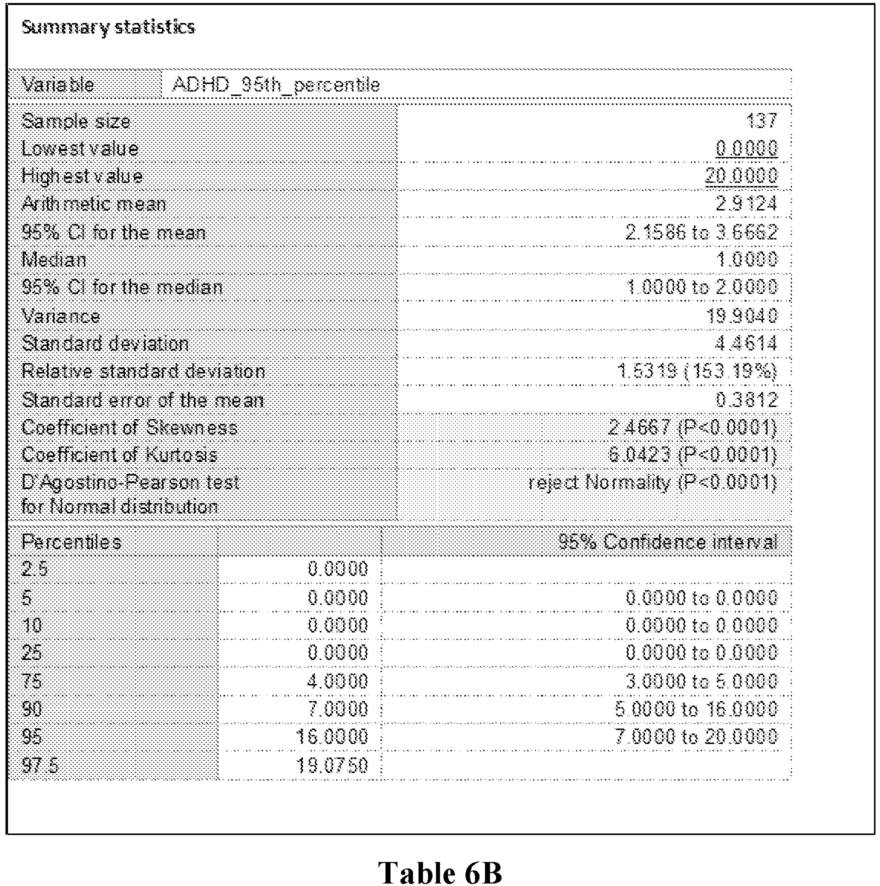

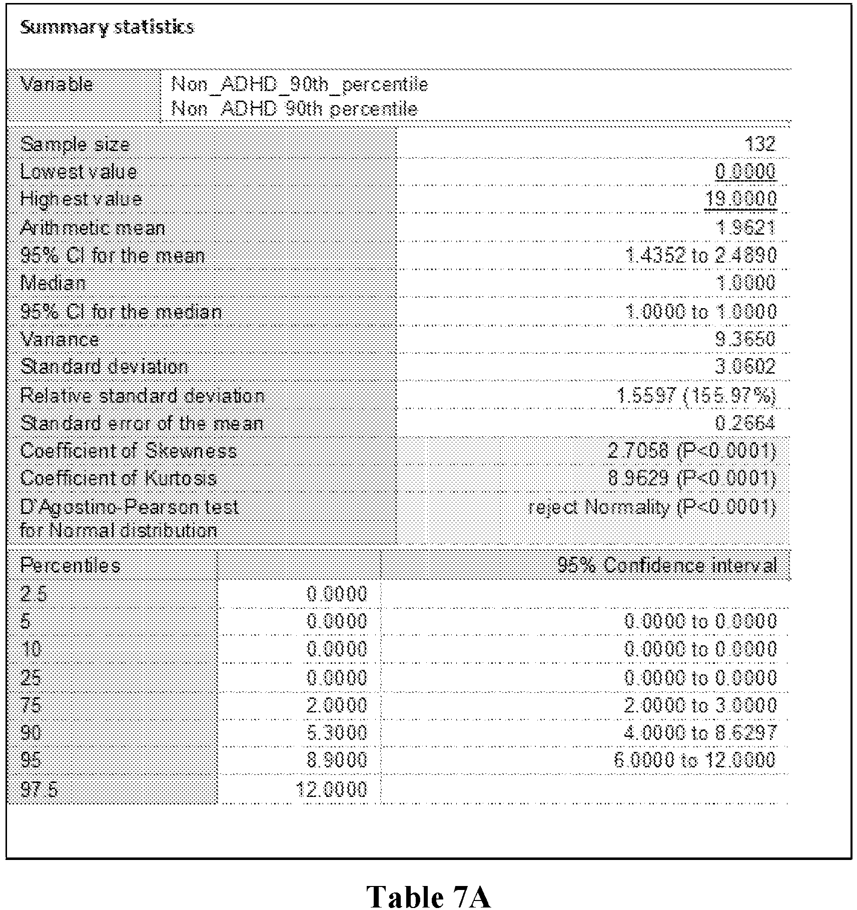

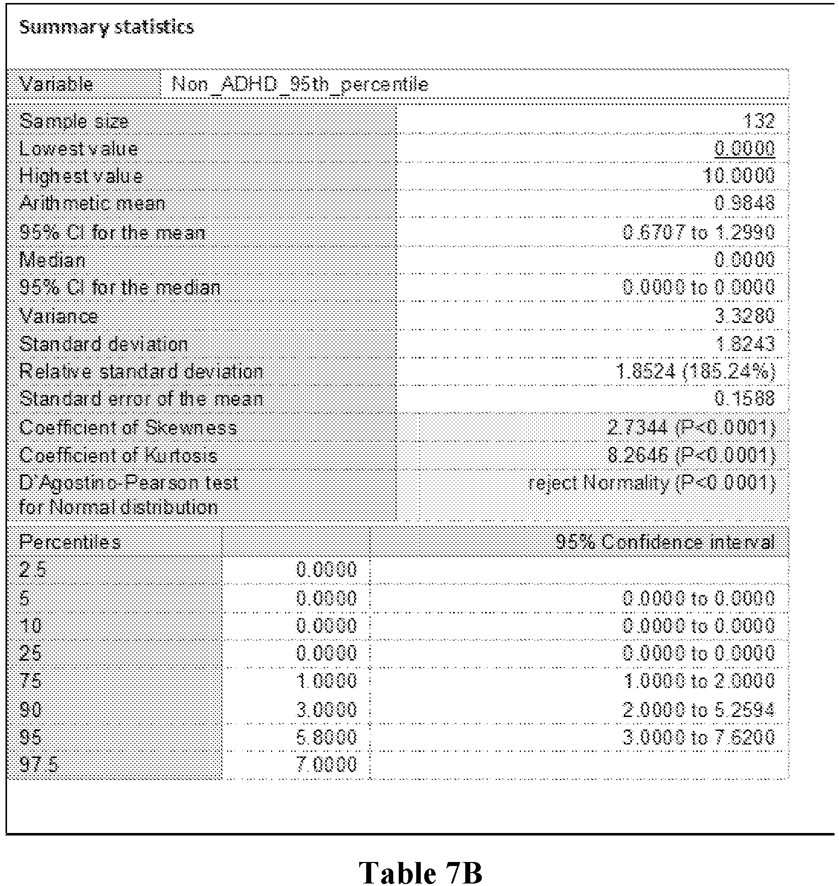

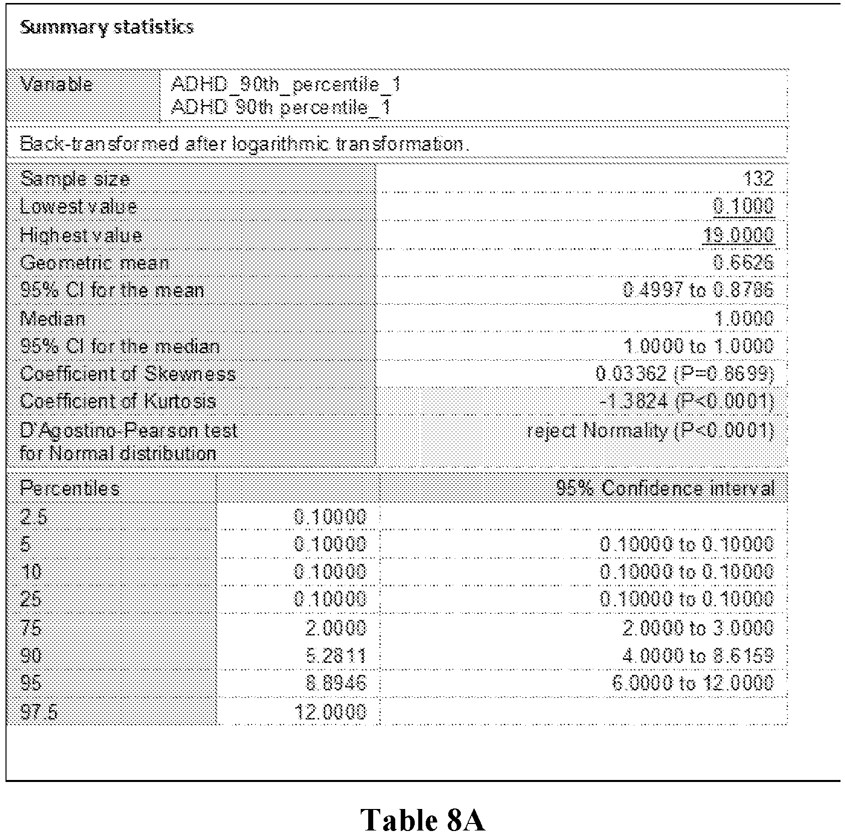

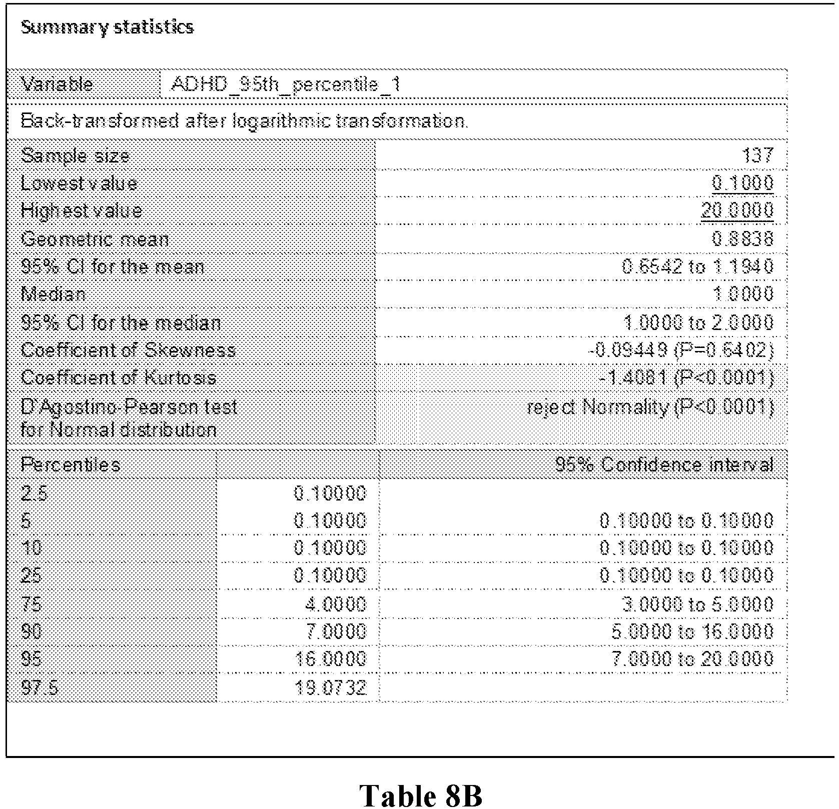

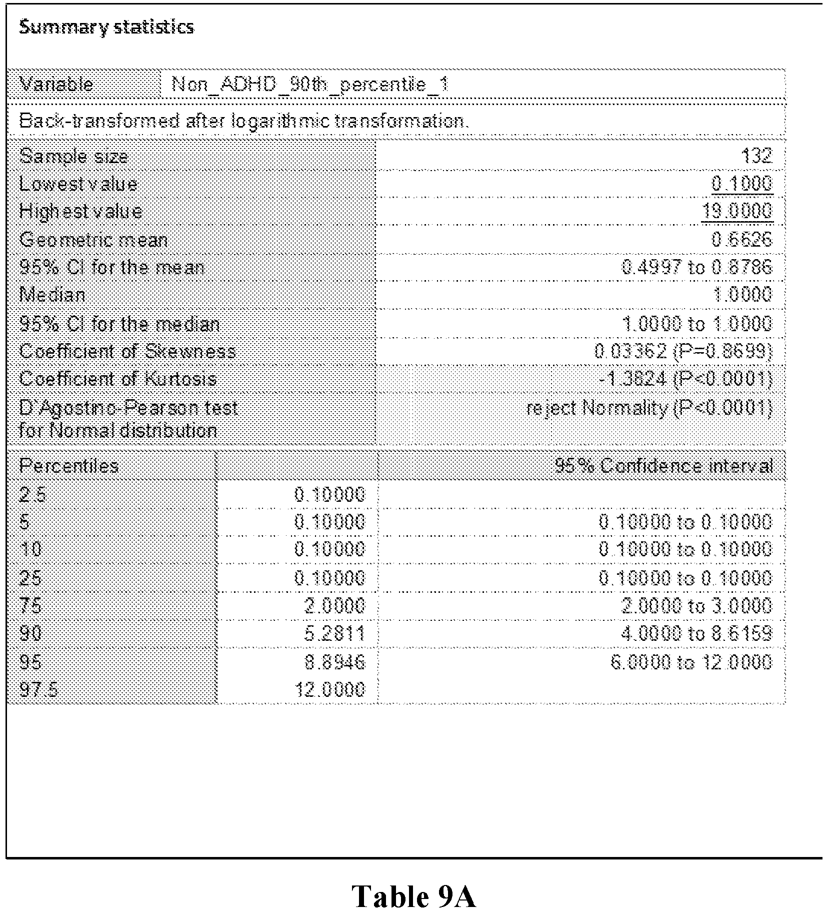

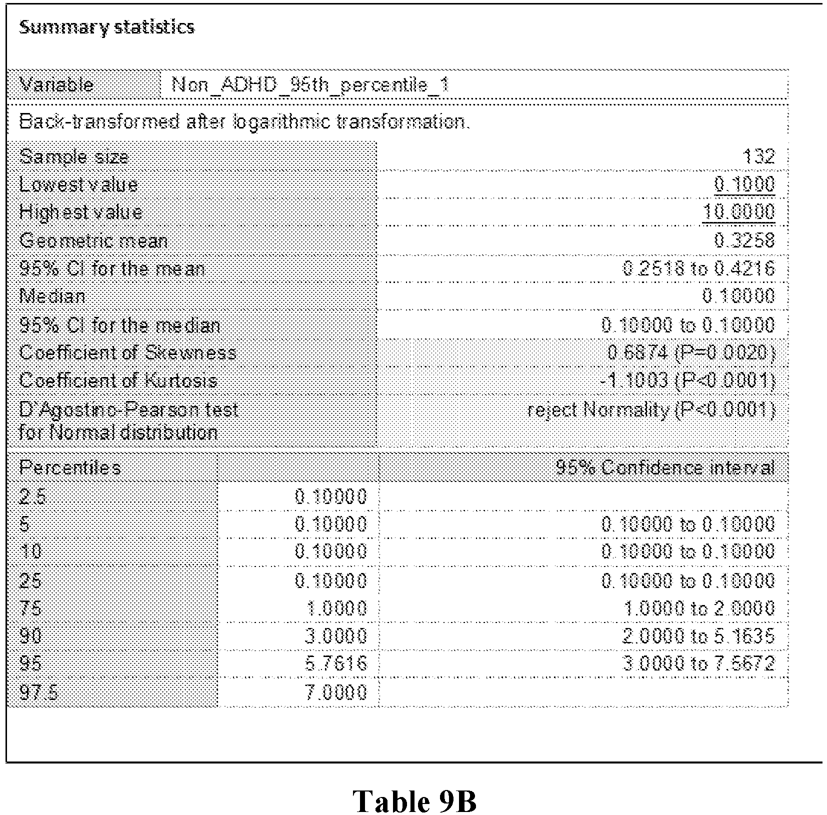

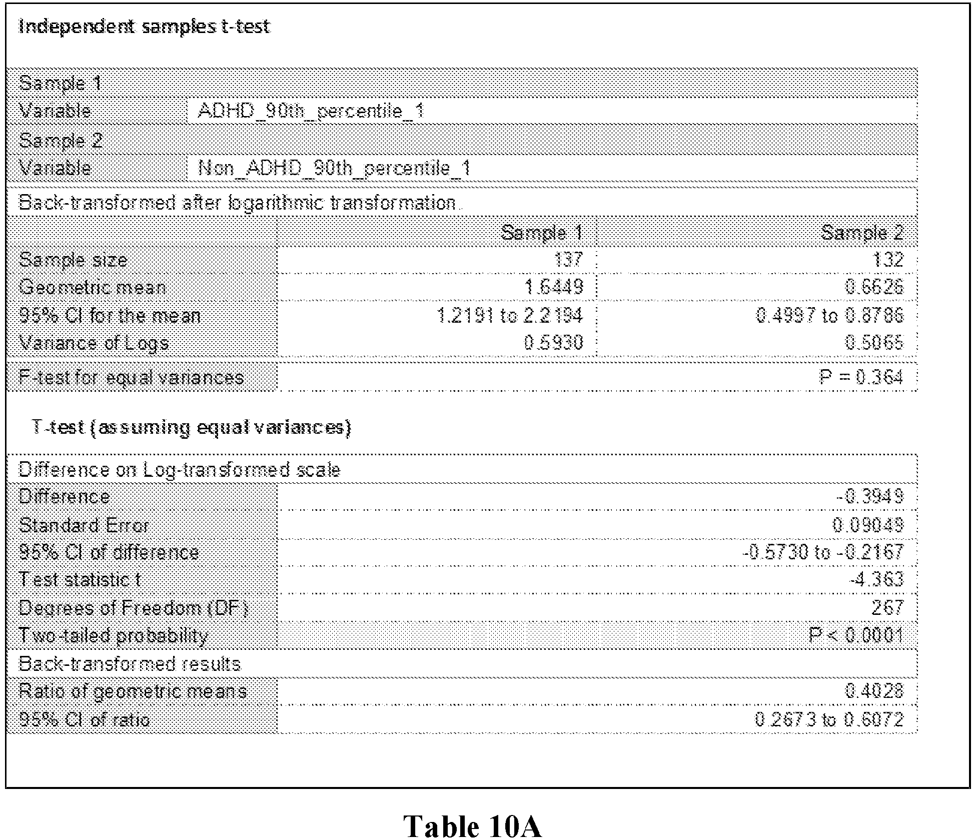

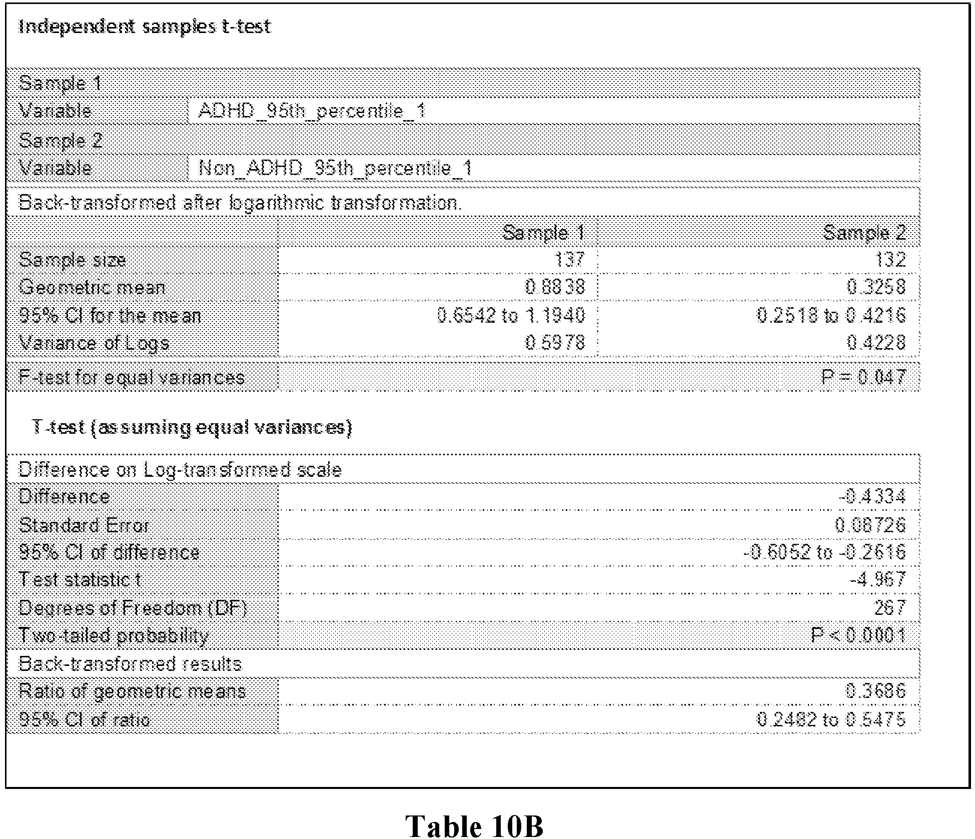

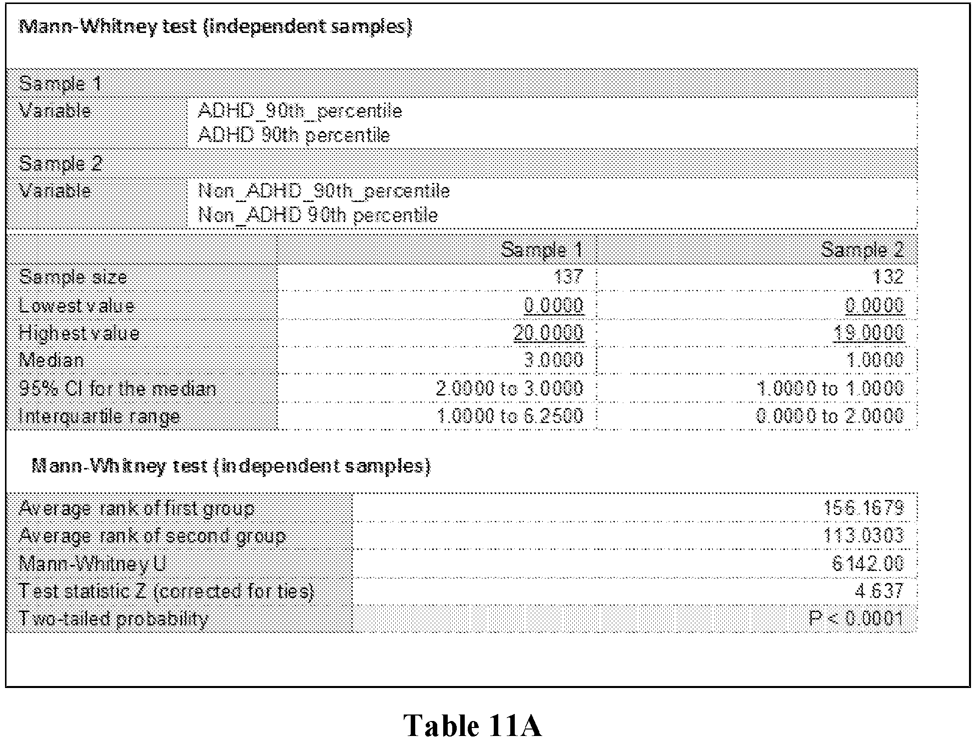

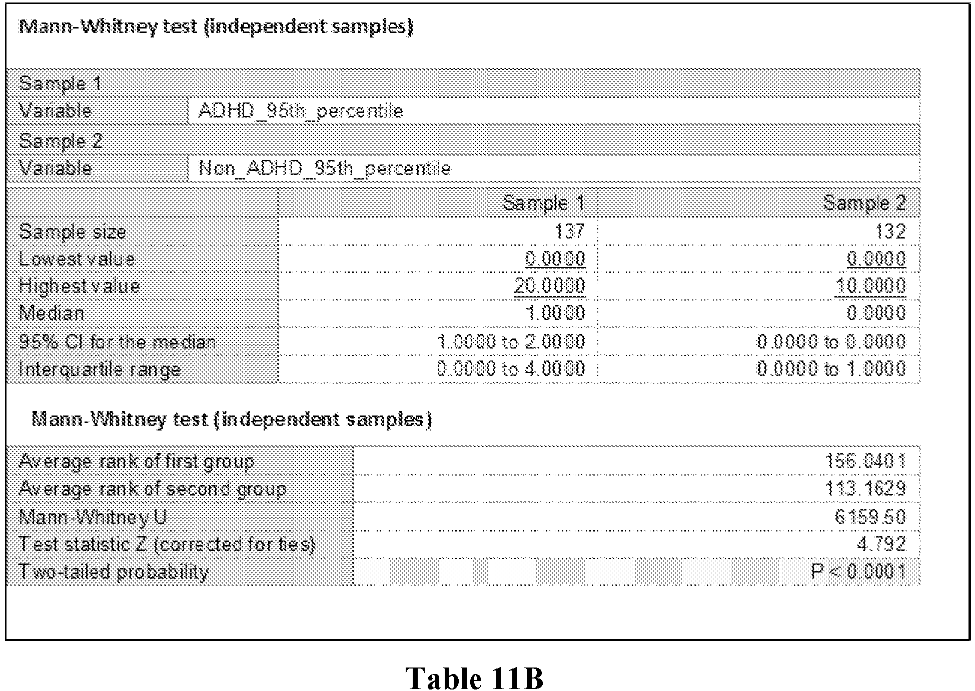

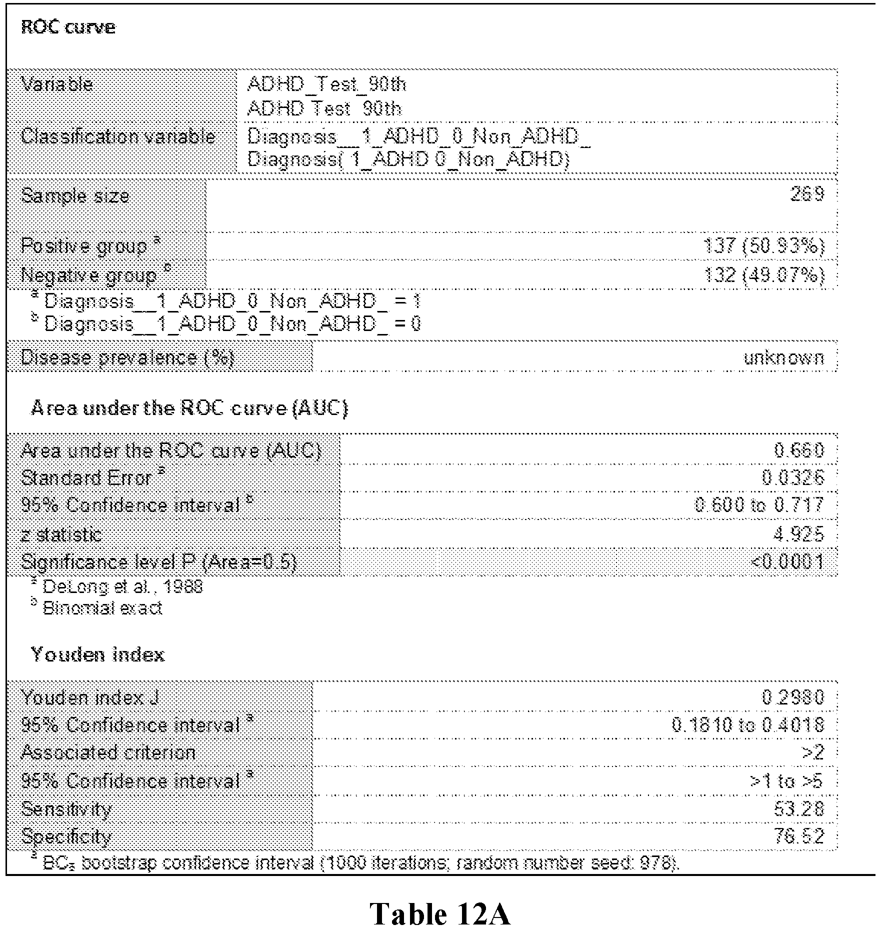

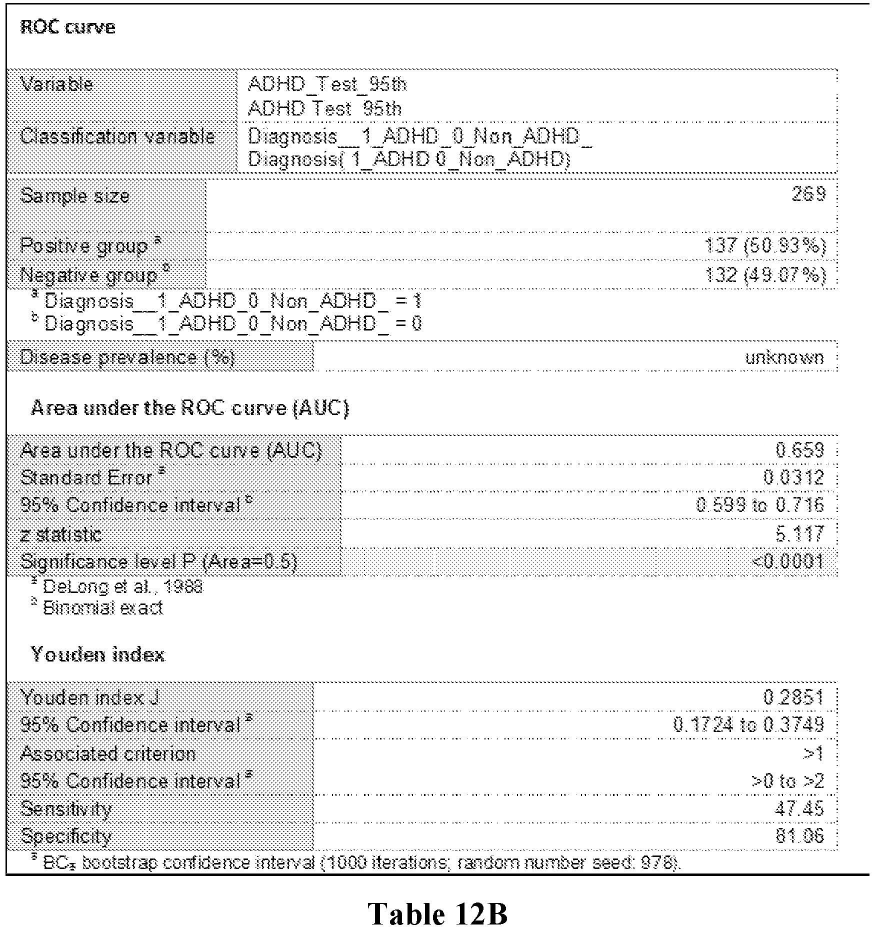

### Performance Metrics in Predicting ADD/ADHD Status from Number of Positive Foods Using 90th Percentile of ELISA Signal to determine Positive

**Table 13A**

| | | | | | | |
|---|---|---|---|---|---|---|
| *Sex* | *No. of Positive Foods as Cutoff* | *Sensitivity* | *Specificity* | *Positive Predictive Value* | *Negative Predictive Value* | *Overall Percent Agreement* |
| FEMALE | 1 | 0.85 | 0.26 | 0.67 | 0.48 | 0.64 |
| | 2 | 0.75 | 0.40 | 0.70 | 0.48 | 0.63 |
| | 3 | 0.67 | 0.55 | 0.73 | 0.48 | 0.63 |
| | 4 | 0.60 | 0.66 | 0.76 | 0.48 | 0.63 |
| | 5 | 0.56 | 0.72 | 0.78 | 0.47 | 0.62 |
| | 6 | 0.52 | 0.76 | 0.79 | 0.46 | 0.60 |
| | 7 | 0.48 | 0.77 | 0.79 | 0.45 | 0.59 |
| | 8 | 0.45 | 0.80 | 0.80 | 0.44 | 0.57 |
| | 9 | 0.42 | 0.82 | 0.81 | 0.44 | 0.56 |
| | 10 | 0.38 | 0.85 | 0.82 | 0.43 | 0.55 |
| | 11 | 0.35 | 0.88 | 0.83 | 0.43 | 0.53 |
| | 12 | 0.33 | 0.89 | 0.83 | 0.42 | 0.53 |
| | 13 | 0.30 | 0.90 | 0.84 | 0.42 | 0.52 |
| | 14 | 0.28 | 0.91 | 0.84 | 0.41 | 0.50 |
| | 15 | 0.25 | 0.91 | 0.83 | 0.40 | 0.49 |
| | 16 | 0.23 | 0.92 | 0.83 | 0.40 | 0.48 |
| | 17 | 0.21 | 0.93 | 0.83 | 0.39 | 0.47 |
| | 18 | 0.20 | 0.94 | 0.85 | 0.39 | 0.46 |
| | 19 | 0.18 | 0.94 | 0.86 | 0.39 | 0.45 |
| | 20 | 0.16 | 0.96 | 0.88 | 0.39 | 0.45 |
| | 21 | 0.15 | 0.97 | 0.89 | 0.39 | 0.44 |
| | 22 | 0.13 | 0.97 | 0.90 | 0.38 | 0.43 |
| | 23 | 0.12 | 0.98 | 0.93 | 0.38 | 0.43 |
| | 24 | 0.10 | 1.00 | 1.00 | 0.38 | 0.42 |
| | 25 | 0.09 | 1.00 | 1.00 | 0.38 | 0.41 |
| | 26 | 0.08 | 1.00 | 1.00 | 0.38 | 0.41 |
| | 27 | 0.07 | 1.00 | 1.00 | 0.38 | 0.40 |
| | 28 | 0.07 | 1.00 | 1.00 | 0.37 | 0.40 |
| | 29 | 0.07 | 1.00 | 1.00 | 0.37 | 0.40 |
| | 30 | 0.07 | 1.00 | 1.00 | 0.37 | 0.40 |
| | 31 | 0.06 | 1.00 | 1.00 | 0.37 | 0.40 |
| | 32 | 0.05 | 1.00 | 1.00 | 0.37 | 0.39 |
| | 33 | 0.05 | 1.00 | 1.00 | 0.37 | 0.39 |
| | 34 | 0.04 | 1.00 | 1.00 | 0.37 | 0.38 |
| | 35 | 0.04 | 1.00 | 1.00 | 0.36 | 0.38 |
| | 36 | 0.02 | 1.00 | 1.00 | 0.36 | 0.37 |
| | 37 | 0.02 | 1.00 | 1.00 | 0.36 | 0.37 |

### Performance Metrics in Predicting ADD/ADHD Status from Number of Positive Foods Using 90th Percentile of ELISA Signal to determine Positive

**Table 13B**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| MALE | 1 | 0.89 | 0.25 | 0.40 | 0.80 | 0.48 |
| | 2 | 0.77 | 0.44 | 0.43 | 0.78 | 0.56 |
| | 3 | 0.65 | 0.57 | 0.45 | 0.74 | 0.60 |
| | 4 | 0.57 | 0.66 | 0.48 | 0.73 | 0.63 |
| | 5 | 0.50 | 0.73 | 0.52 | 0.73 | 0.65 |
| | 6 | 0.44 | 0.79 | 0.54 | 0.72 | 0.67 |
| | 7 | 0.37 | 0.83 | 0.55 | 0.70 | 0.67 |
| | 8 | 0.33 | 0.85 | 0.56 | 0.70 | 0.66 |
| | 9 | 0.29 | 0.87 | 0.55 | 0.69 | 0.66 |
| | 10 | 0.27 | 0.88 | 0.56 | 0.69 | 0.66 |
| | 11 | 0.26 | 0.90 | 0.58 | 0.68 | 0.67 |
| | 12 | 0.24 | 0.91 | 0.60 | 0.68 | 0.67 |
| | 13 | 0.23 | 0.92 | 0.60 | 0.68 | 0.67 |
| | 14 | 0.21 | 0.92 | 0.58 | 0.68 | 0.67 |
| | 15 | 0.18 | 0.92 | 0.57 | 0.67 | 0.66 |
| | 16 | 0.15 | 0.93 | 0.56 | 0.67 | 0.65 |
| | 17 | 0.12 | 0.94 | 0.50 | 0.66 | 0.65 |
| | 18 | 0.11 | 0.94 | 0.50 | 0.66 | 0.64 |
| | 19 | 0.10 | 0.94 | 0.50 | 0.65 | 0.64 |
| | 20 | 0.09 | 0.95 | 0.50 | 0.65 | 0.64 |
| | 21 | 0.08 | 0.96 | 0.50 | 0.65 | 0.64 |
| | 22 | 0.08 | 0.96 | 0.50 | 0.65 | 0.64 |
| | 23 | 0.07 | 0.96 | 0.50 | 0.65 | 0.65 |
| | 24 | 0.07 | 0.97 | 0.60 | 0.65 | 0.65 |
| | 25 | 0.07 | 0.98 | 0.67 | 0.65 | 0.65 |
| | 26 | 0.07 | 0.98 | 0.67 | 0.65 | 0.65 |
| | 27 | 0.07 | 0.98 | 0.67 | 0.65 | 0.65 |
| | 28 | 0.07 | 0.98 | 0.67 | 0.65 | 0.65 |
| | 29 | 0.04 | 0.98 | 0.67 | 0.65 | 0.65 |
| | 30 | 0.04 | 0.98 | 0.67 | 0.65 | 0.65 |
| | 31 | 0.04 | 0.98 | 0.67 | 0.65 | 0.65 |
| | 32 | 0.04 | 1.00 | 0.67 | 0.65 | 0.65 |
| | 33 | 0.03 | 1.00 | 1.00 | 0.65 | 0.65 |
| | 34 | 0.00 | 1.00 | 1.00 | 0.65 | 0.65 |
| | 35 | 0.00 | 1.00 | 1.00 | 0.64 | 0.64 |
| | 36 | 0.00 | 1.00 | 0.00 | 0.64 | 0.64 |
| | 37 | 0.00 | 1.00 | 0.00 | 0.64 | 0.64 |

### Performance Metrics in Predicting ADD/ADHD Status from Number of Positive Foods Using 95th Percentile of ELISA Signal to determine Positive

**Table 14A**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| FEMALE | 1 | 0.73 | 0.41 | 0.69 | 0.46 | 0.62 |
| | 2 | 0.61 | 0.58 | 0.72 | 0.45 | 0.60 |
| | 3 | 0.53 | 0.72 | 0.77 | 0.46 | 0.60 |
| | 4 | 0.47 | 0.79 | 0.81 | 0.46 | 0.59 |
| | 5 | 0.43 | 0.82 | 0.81 | 0.45 | 0.57 |
| | 6 | 0.39 | 0.84 | 0.81 | 0.43 | 0.55 |
| | 7 | 0.35 | 0.85 | 0.81 | 0.42 | 0.53 |
| | 8 | 0.31 | 0.88 | 0.82 | 0.41 | 0.51 |
| | 9 | 0.27 | 0.91 | 0.84 | 0.41 | 0.49 |
| | 10 | 0.23 | 0.93 | 0.85 | 0.40 | 0.48 |
| | 11 | 0.20 | 0.94 | 0.86 | 0.39 | 0.47 |
| | 12 | 0.18 | 0.94 | 0.86 | 0.39 | 0.45 |
| | 13 | 0.16 | 0.97 | 0.88 | 0.39 | 0.44 |
| | 14 | 0.14 | 0.97 | 0.89 | 0.38 | 0.44 |
| | 15 | 0.12 | 0.97 | 0.91 | 0.38 | 0.43 |
| | 16 | 0.11 | 1.00 | 1.00 | 0.38 | 0.42 |
| | 17 | 0.10 | 1.00 | 1.00 | 0.38 | 0.42 |
| | 18 | 0.09 | 1.00 | 1.00 | 0.38 | 0.41 |
| | 19 | 0.08 | 1.00 | 1.00 | 0.38 | 0.41 |
| | 20 | 0.07 | 1.00 | 1.00 | 0.37 | 0.40 |
| | 21 | 0.07 | 1.00 | 1.00 | 0.37 | 0.40 |
| | 22 | 0.07 | 1.00 | 1.00 | 0.37 | 0.40 |
| | 23 | 0.07 | 1.00 | 1.00 | 0.37 | 0.40 |
| | 24 | 0.07 | 1.00 | 1.00 | 0.37 | 0.40 |
| | 25 | 0.06 | 1.00 | 1.00 | 0.37 | 0.40 |
| | 26 | 0.06 | 1.00 | 1.00 | 0.37 | 0.39 |
| | 27 | 0.05 | 1.00 | 1.00 | 0.37 | 0.39 |
| | 28 | 0.05 | 1.00 | 1.00 | 0.37 | 0.39 |
| | 29 | 0.05 | 1.00 | 1.00 | 0.37 | 0.38 |
| | 30 | 0.04 | 1.00 | 1.00 | 0.37 | 0.38 |
| | 31 | 0.04 | 1.00 | 1.00 | 0.36 | 0.38 |
| | 32 | 0.03 | 1.00 | 1.00 | 0.36 | 0.38 |
| | 33 | 0.03 | 1.00 | 1.00 | 0.36 | 0.38 |
| | 34 | 0.02 | 1.00 | 1.00 | 0.36 | 0.37 |
| | 35 | 0.02 | 1.00 | 1.00 | 0.36 | 0.37 |
| | 36 | 0.02 | 1.00 | 1.00 | 0.36 | 0.36 |
| | 37 | 0.02 | 1.00 | 1.00 | 0.36 | 0.36 |

### Performance Metrics in Predicting ADD/ADHD Status from Number of Positive Foods Using 95th Percentile of ELISA Signal to determine Positive

**Table 14B**

| *Sex* | *No. of Positive Foods as Cutoff* | *Sensitivity* | *Specificity* | *Positive Predictive Value* | *Negative Predictive Value* | *Overall Percent Agreement* |
|---|---|---|---|---|---|---|
| MALE | 1 | 0.80 | 0.43 | 0.44 | 0.80 | 0.57 |
| | 2 | 0.65 | 0.63 | 0.49 | 0.76 | 0.64 |
| | 3 | 0.50 | 0.73 | 0.50 | 0.72 | 0.65 |
| | 4 | 0.41 | 0.82 | 0.56 | 0.71 | 0.67 |
| | 5 | 0.33 | 0.87 | 0.59 | 0.70 | 0.68 |
| | 6 | 0.28 | 0.90 | 0.60 | 0.69 | 0.68 |
| | 7 | 0.24 | 0.91 | 0.60 | 0.69 | 0.67 |
| | 8 | 0.21 | 0.92 | 0.62 | 0.68 | 0.67 |
| | 9 | 0.18 | 0.94 | 0.60 | 0.67 | 0.67 |
| | 10 | 0.15 | 0.94 | 0.57 | 0.67 | 0.66 |
| | 11 | 0.12 | 0.94 | 0.57 | 0.66 | 0.65 |
| | 12 | 0.10 | 0.95 | 0.50 | 0.66 | 0.65 |
| | 13 | 0.08 | 0.96 | 0.50 | 0.65 | 0.64 |
| | 14 | 0.07 | 0.96 | 0.50 | 0.65 | 0.64 |
| | 15 | 0.07 | 0.96 | 0.50 | 0.65 | 0.65 |
| | 16 | 0.07 | 0.97 | 0.50 | 0.65 | 0.65 |
| | 17 | 0.07 | 0.98 | 0.60 | 0.65 | 0.65 |
| | 18 | 0.07 | 0.98 | 0.67 | 0.65 | 0.65 |
| | 19 | 0.07 | 0.98 | 0.67 | 0.65 | 0.65 |
| | 20 | 0.07 | 0.98 | 0.67 | 0.65 | 0.65 |
| | 21 | 0.07 | 0.98 | 0.67 | 0.65 | 0.66 |
| | 22 | 0.07 | 0.98 | 0.75 | 0.65 | 0.66 |
| | 23 | 0.07 | 1.00 | 1.00 | 0.65 | 0.66 |
| | 24 | 0.07 | 1.00 | 1.00 | 0.66 | 0.66 |
| | 25 | 0.07 | 1.00 | 1.00 | 0.66 | 0.66 |
| | 26 | 0.06 | 1.00 | 1.00 | 0.65 | 0.66 |
| | 27 | 0.04 | 1.00 | 1.00 | 0.65 | 0.66 |
| | 28 | 0.04 | 1.00 | 1.00 | 0.65 | 0.66 |
| | 29 | 0.04 | 1.00 | 1.00 | 0.65 | 0.66 |
| | 30 | 0.04 | 1.00 | 1.00 | 0.65 | 0.66 |
| | 31 | 0.03 | 1.00 | 1.00 | 0.65 | 0.65 |
| | 32 | 0.00 | 1.00 | 1.00 | 0.65 | 0.65 |
| | 33 | 0.00 | 1.00 | 1.00 | 0.64 | 0.64 |
| | 34 | 0.00 | 1.00 | 1.00 | 0.64 | 0.64 |
| | 35 | 0.00 | 1.00 | 1.00 | 0.64 | 0.64 |
| | 36 | 0.00 | 1.00 | . | 0.64 | 0.64 |
| | 37 | 0.00 | 1.00 | . | 0.64 | 0.64 |

## Claims

1. A test panel for testing food sensitivity in a patient diagnosed with or suspected of having attention deficit disorder / attention deficit hyperactivity disorder (ADD/ADHD), comprising:
a plurality of distinct food preparations, wherein each food preparation is independently coupled to an individually addressable solid carrier;
wherein the plurality of distinct food preparations consists of cantaloupe, wheat, tomato, cucumber, squashes, almond, egg, cauliflower, pinto bean, broccoli, orange, butter, corn, lettuce, rye, peach, green pea, carrot, tea, mustard, strawberry, celery, green pepper, garlic, oat, onion, banana, eggplant, cabbage, safflower, olive, cottage cheese, grapefruit, black walnut, cow's milk, soybean, chili pepper, tobacco, malt, cinnamon, cane sugar, and potato.

2. The test panel of claim 1, wherein the distinct food preparations are crude filtered aqueous extracts or processed aqueous extracts.

3. The test panel of claim 1 or claim 2, wherein the solid carrier is a well of a multiwell plate, a bead, an electrical sensor, a chemical sensor, a microchip, or an adsorptive film.

4. An *in vitro* method of testing food sensitivity in a patient diagnosed with or suspected of having attention deficit disorder / attention deficit hyperactivity disorder (ADD/ADHD), comprising:
contacting the test panel of claim 1 with a bodily fluid of a patient that is diagnosed with or suspected of having ADD/ADHD, wherein the step of contacting is performed under conditions that allow IgG from the bodily fluid to bind to at least one component of each food preparation;
measuring IgG bound to the at least one component of each food preparation to obtain a signal; and
updating or generating a report using the signal.

5. The method of claim 4, wherein the bodily fluid of the patient is selected from the group consisting of whole blood, plasma, serum, saliva, urine and a fecal suspension.

6. The method of claim 4 or claim 5, further comprising comparing the signal to a gender-stratified reference value for each food preparation using gender identification to obtain a result, wherein the gender-stratified reference value for each food preparation is the 90^{th} percentile rank, or higher, of signals obtained by contacting bodily fluid from a control group of subjects that is not diagnosed with or suspected of having ADD/ADHD with the food preparation.

## Patentansprüche

1. Testpanel zum Prüfen von Nahrungsmittelempfindlichkeit bei einem Patienten, bei dem eine Aufmerksamkeitsdefizitstörung / Aufmerksamkeitsdefizit-Hyperaktivitätsstörung (ADS/ADHS) diagnostiziert wurde oder bei dem der Verdacht auf diese besteht, umfassend:
eine Vielzahl unterschiedlicher Nahrungsmittelzubereitungen, wobei jede Nahrungsmittelzubereitung unabhängig an einen einzeln adressierbaren festen Träger gekoppelt ist;
wobei die Vielzahl unterschiedlicher Nahrungsmittelzubereitungen aus Kantalupmelone, Weizen, Tomate, Gurke, Kürbis, Mandel, Ei, Blumenkohl, Pintobohnen, Brokkoli, Orange, Butter, Mais, Kopfsalat, Roggen, Pfirsich, grünen Erbsen, Karotten, Tee, Senf, Erdbeere, Sellerie, grünem Pfeffer, Knoblauch, Hafer, Zwiebel, Banane, Aubergine, Kohl, Saflor, Olive, Hüttenkäse, Grapefruit, schwarzer Walnuss, Kuhmilch, Sojabohnen, Chili, Tabak, Malz, Zimt, Rohrzucker und Kartoffeln besteht.

2. Testpanel nach Anspruch 1, wobei die unterschiedlichen Nahrungsmittelzubereitungen grob gefilterte wässrige Extrakte oder verarbeitete wässrige Extrakte sind.

3. Testpanel nach Anspruch 1 oder Anspruch 2, wobei der feste Träger eine Vertiefung einer Mehrfachvertiefungsplatte, eine Perle, ein elektrischer Sensor, ein chemischer Sensor, ein Mikrochip oder ein Adsorptionsfilm ist.

4. In-vitro-Verfahren zum Testen der Nahrungsmittelempfindlichkeit bei einem Patienten, bei dem eine Aufmerksamkeitsdefizitstörung / Aufmerksamkeitsdefizit-Hyperaktivitätsstörung (ADS/ADHS) diagnostiziert wurde oder bei dem der Verdacht auf diese besteht, umfassend:
Inkontaktbringen des Testpanels nach Anspruch 1 mit einer Körperflüssigkeit eines Patienten, bei dem ADS/ADHS diagnostiziert wurde oder vermutet wird, wobei der Schritt des Inkontaktbringens unter Bedingungen erfolgt, die eine Bindung von IgG aus der Körperflüssigkeit an mindestens einen Bestandteil jeder Nahrungsmittelzubereitung ermöglichen;
Messen von an mindestens einem Bestandteil jeder Nahrungsmittelzubereitung gebundenem IgG, um ein Signal zu erhalten; und
Aktualisieren oder Erstellen eines Berichts mithilfe des Signals.

5. Verfahren nach Anspruch 4, wobei die Körperflüssigkeit des Patienten ausgewählt ist aus der Gruppe bestehend aus Vollblut, Plasma, Serum, Speichel, Urin und einer Stuhlsuspension.

6. Verfahren nach Anspruch 4 oder Anspruch 5, das ferner das Vergleichen des Signals mit einem nach Geschlecht geschichteten Referenzwert für jede Nahrungsmittelzubereitung unter Verwenden einer Geschlechtsidentifikation zum Erhalten eines Ergebnisses umfasst, wobei der nach Geschlecht geschichtete Referenzwert für jede Nahrungsmittelzubereitung dem 90. Perzentilrang oder höher von Signalen entspricht, die durch Inkontaktbringen von Körperflüssigkeiten einer Kontrollgruppe von Personen, bei denen ADS/ADHS weder diagnostiziert wurde noch der Verdacht auf ADS/ADHS besteht, mit der Nahrungsmittelzubereitung erhalten werden.

## Revendications

1. Panel de test pour tester la sensibilité alimentaire chez un patient diagnostiqué ou suspecté de souffrir d'un trouble déficitaire de l'attention avec ou sans hyperactivité (TDA/TDAH), comprenant :
une pluralité de préparations alimentaires distinctes, dans lequel chaque préparation alimentaire est couplée indépendamment à un véhicule solide adressable individuellement ;
dans lequel la pluralité de préparations alimentaires distinctes est constituée de cantaloup, blé, tomate, concombre, courge, amande, oeuf, chou-fleur, haricot pinto, brocoli, orange, beurre, maïs, laitue, seigle, pêche, pois vert, carotte, thé, moutarde, fraise, céleri, poivron vert, ail, avoine, oignon, banane, aubergine, chou, carthame, olive, fromage cottage, pamplemousse, noix noire, lait de vache, soja, piment, tabac, malt, cannelle, sucre de canne et pomme de terre.

2. Panel de test selon la revendication 1, dans lequel les préparations alimentaires distinctes sont des extraits aqueux bruts filtrés ou des extraits aqueux traités.

3. Panel de test selon la revendication 1 ou la revendication 2, dans lequel le véhicule solide est un puits d'une plaque multipuits, une bille, un capteur électrique, un capteur chimique, une micropuce ou un film adsorbant.

4. Méthode *in vitro* de test de sensibilité alimentaire chez un patient diagnostiqué ou suspecté de souffrir d'un trouble déficitaire de l'attention avec ou sans hyperactivité (TDA/TDAH), comprenant :
la mise en contact du panel de test selon la revendication 1 avec un fluide corporel d'un patient qui est diagnostiqué ou suspecté de souffrir d'un TDA/TDAH, dans lequel l'étape de mise en contact est effectuée dans des conditions qui permettent aux IgG du fluide corporel de se lier à au moins un composant de chaque préparation alimentaire ;
la mesure des IgG liés à l'au moins un composant de chaque préparation alimentaire pour obtenir un signal ; et
la mise à jour ou la génération d'un rapport à l'aide du signal.

5. Méthode selon la revendication 4, dans lequel le fluide corporel du patient est choisi dans le groupe constitué du sang total, plasma, sérum, salive, urine et une suspension fécale.

6. Méthode selon la revendication 4 ou la revendication 5, comprenant en outre la comparaison du signal à une valeur de référence stratifiée par sexe pour chaque préparation alimentaire en utilisant l'identification par sexe pour obtenir un résultat, dans lequel la valeur de référence stratifiée par sexe pour chaque préparation alimentaire est le rang du 90ème centile, ou plus, de signaux obtenus par la mise en contact d'un fluide corporel provenant d'un groupe témoin de sujets qui n'ont pas été diagnostiqués ou suspectés de souffrir de TDA/TDAH avec la préparation alimentaire.
